# EUROPEAN PATENT APPLICATION

(11) **EP 2 540 321 A1**
(43) Date of publication of application: **02.01.2013**
(21) Application number: 11172066.0
(22) Date of filing: 30.06.2011
(51) Int. Cl.: A61K 49/04, C07F 11/00

(54) **Bis Tridentate W3O2 Clusters for X-Ray imaging**

(71) Applicant: Bayer Intellectual Property GmbH, 40789 Monheim (DE)
(72) Inventor: Frenzel, Thomas, 65719 Hofheim (Taunus) (DE); Pietsch, Hubertus, 14532 Kleinmachnow (DE); Haßfeld, Jorma, 10439 Berlin (DE); Reinhardt, Michael, 14129 Berlin (DE); Jost, Gregor, 10318 Berlin (DE); Behrendt, Werner, 10709 Berlin (DE); Suelzle, Detlev, 13465 Berlin (DE); Schmitt-Willich, Heribert, 10551 Berlin (DE); Berger, Markus, 12167 Berlin (DE)
(74) Representative: BIP Patents

(57) **Abstract**

The present invention describes a new class of highly stable W₃O₂ Clusters, a method for their preparation, their use as X-ray contrast agents and the intermediates tri- or tetracarboxylic acids as desired tridentate ligand resources.

## Description

The present invention describes a new class of trinuclear W₃O₂ tungsten clusters comprising tridentate tri- or tetracarboxylic acid ligands, a method for their preparation, their use as X-ray contrast agents and the intermediates, tri- or tetracarboxylic acids, as desired tridentate ligand resource.

### Background of the invention

Synthesis and characterization of bi-oxo capped trinuclear tungsten clusters have been described by *de novo* synthesis starting from Na₂WO₄ (Powell, G.; Richens, D.T. Inorg.Chem. 1993, 32, 4021-4029 or W(CO)₆ (Bino, A.; Cotton, F. A.; Dori, Z.; Koch, S.; Kueppers, H.; Millar, M.; Sekutowski, J. C. Inorg. Chem. 1978, 17, 3245-3253, Bino, A.; Hesse; K. F.; Kueppers, H. Acta Crystallogr. 1980, B36, 723-725, Cotton, F.A.; Dori, Z.; Marler, D.O.; Schwotzer, W. Inorg.Chem 1984, 23, 4033-4038 and Cotton, F.A.; Dori, Z.; Marler, D.O.; Schwotzer, W. Inorg.Chem 1984, 23, 4738-4742) or by ligand exchange synthesis (WO 97/03993 and WO 97/03994) starting from preformed clusters. These tungsten clusters are part of the larger class of trinuclear bicapped clusters. The existence of this class is known for more than 3 decades and continuous research on their synthetic accessibility or behaviour and their general properties has been performed since then. Their general geometry was described by Powell, G.; Richens, D.T. Inorg.Chem. 1993, 32, 4021-4029.

The water solubility and ability to absorb X-rays of these tungsten clusters and their potential use in diagnostic X-ray imaging has been described in WO 91/14460, WO 92/00698, WO 97/03993 and WO 97/03994.

Further investigations of these known tungsten clusters disclosed their chemical and physiological instability. Tungsten clusters with insufficient stability in aqueous solution or in the physiological environment of a patient would lead to a release of the ligand in the form of its free acid and the bare tungstate core. This would cause inacceptable toxic side effects in a patient, especially with respect to the expected high dose which will be necessary for a single diagnostic procedure. It is known from the well established triiodinated X-ray contrast agents that the dose for a single diagnostic procedure is in the range of 100-1500 mg iodine / kg body weight, equivalent to about 15-225 g of contrast agent for a 75 kg patient. Contrast media solutions are injected intravenously or intraarterially as a rapid bolus (usually in computed tomography (CT) via power injector) with the injection rate depending on the clinical indication or the examination protocol and area of interest.

Tungsten is characterized by a higher absorption coefficient for X-rays than iodine, especially in the range of tube voltages normally used in modern CT. A modern CT X-ray-tube, however, requires a minimum voltage of about 70 kV and reaches maximum voltage of 160 kV. As future technical developments in CT will not substantially change these parameters, iodine generally does not provide ideal attenuation features for this technology. In comparison to iodine the attenuation optimum (k-edge) of tungsten corresponds better to the ranges of voltages used in CT. Therefore the new tungsten clusters require a similar or lower contrast media dosage than conventional triiodinated contrast agents.

The use of tungsten based contrast agents will allow more flexibility for CT scanning protocols and lead to scan protocols that provide equivalent diagnostic value at lower radiation doses. Especially this feature is of high importance for CT. As technical development goals in terms of spatial and temporal resolution have approached the limit of clinical significance, reduction of the radiation burden of CT scanning has today become a central aspect of the development of new CT scanners and x-ray machines. Following the widely accepted ALARA-rule (radiation exposure has to be reduced to levels: As Low As Reasonably Achievable), the new tungsten based contrast agents will contribute to high-quality diagnostic imaging at reduced radiation exposure.

The aim of the present invention was to provide sufficiently stable, water soluble and well tolerated tungsten clusters for use as X-ray contrast agent in diagnostic imaging, especially in modern computed tomography.

This aim was achieved by the provision of the compounds of the present invention. Surprisingly, it was observed that monodentate ligands claimed in WO 97/03993 and WO 97/03994 can be replaced by tridentate ligands and that the clusters described in this patent application show a highly increased stability under heat sterilization conditions of the aqueous solution, and have an excellent tolerability in experimental animals as well as a high *in vivo* stability.

After intravenous injection the compounds of the present invention are excreted fast and quantitatively via the kidneys, comparable to the well established triiodinated X-ray contrast agents.

The invention of suitable new tridentate ligands for the W₃O₂ core and the synthesis of new stable bis tridentate W₃O₂ clusters enabled for the first time the practical use of this compound class as X-ray contrast agents in diagnostic imaging. By enabling tungsten based contrast agents the option to reduce radiation dose is a clear advantage of W₃O₂ clusters of the present invention over existing iodine based contrast agents due to the higher absorbtion coefficient of tungsten for X-rays compared to iodine.

### Summary of the invention

The present invention describes a new class of highly stable W₃O₂ clusters, a method for their preparation, their use as X-ray contrast agents and the intermediates tri- or tetracarboxylic acids as desired tridentate ligand resources.

### Detailed Description of the invention

In a first aspect, the present invention is directed to trinuclear tungsten clusters comprising tridentate carboxylic acid ligands, especially comprising at least three 3-propionic acid structur elements in each ligand.

In a preferred embodiment the trinuclear tungsten cluster comprises a W₃O₂ core.

In another preferred embodiment the trinuclear tungsten cluster comprises two tri- or tetracarboxylic acid ligands.

In a second aspect, the present invention is directed to compounds of the general formula I, 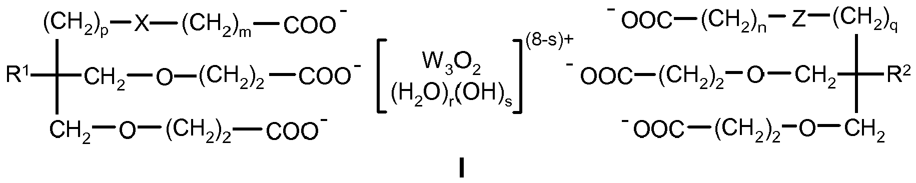 wherein
R¹ is H, CH₃, CH₂OH, CH₂OCH₂, CH₂O(CH₂)₂COOH, NH₂, NH(CH₂)₂OH, NHCH₂CH(OH)CH₂OH, NHCH(CH₂OH)₂, NHCH₂(CH(OH))₂CH₂OH, NHCH₂(CH(OH))₃CH₂OH, NH(CO)CH₂OCH₃ or OH;
R² is H, CH₃, CH₂OH, CH₂OCH₃, CH₂O(CH₂)₂COOH, NH₂, NH(CH₂)₂OH, NHCH₂CH(OH)CH₂OH, NHCH(CH₂OH)₂, NHCH₂(CH(OH))₂CH₂OH, NHCH₂(CH(OH))₃CH₂OH, NH(CO)CH₂OCH₃ or OH;
X is O or NR³;
   wherein R³ is H, (CH₂)₂OH, CH₂CH(OH)CH₂OH, CH(CH₂OH)₂, CH₂(CH(OH))₂CH₂OH or CH₂(CH(OH))₃CH₂OH;
Z is O or NR⁴;
   wherein R⁴ is H, (CH₂)₂OH, CH₂CH(OH)CH₂OH, CH(CH₂OH)₂, CH₂(CH(OH))₂CH₂OH or CH₂(CH(OH))₃CH₂OH;
m is 2, 3;
n is 2, 3;
p is 0, 1;
q is 0, 1;
r is 0, 1, 2, 3;
and
s is 0, 1, 2, 3;
   with the proviso that r + s is 3;

if necessary including any protonated species and any deprotonated species of said compounds, including all isomeric forms of said compounds, including but not limited to enantiomers, diastereomers, regioisomers and mixtures thereof, and any pharmaceutically acceptable salt of such compounds.

As known to the person skilled in the art, the central tris aqua W₃O₂ cluster ion acts as a polyprotic acid in aqueous solution. Dependent on the pH of the solution different protonation species [W₃O₂(H₂O)₃]⁸⁺, [W₃O₂(H₂O)₂MOH)⁷⁺, [W₃O₂(H₂O)(OH)₂]⁶⁺ and [W₃O₂(OH)₃]⁵⁺ are present (Bino, A.; Gibson, D. Inorganica Chimica Acta, Volume 1985, 104, 155-160). Additional counter ions may be present.

In a third aspect, the invention is directed to compounds of the general formula II, 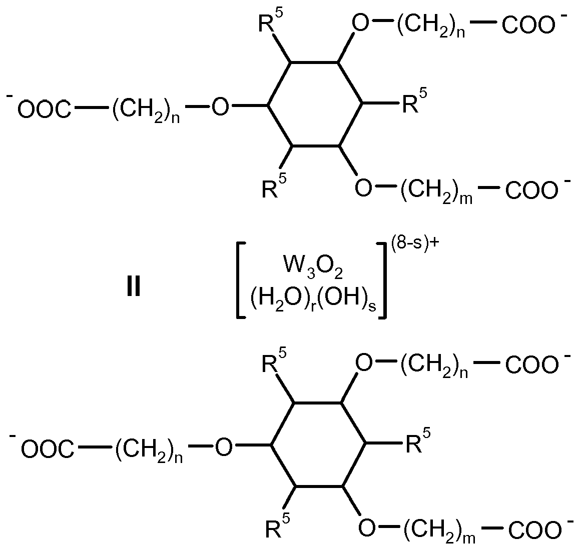 wherein
the substituents HOOC-(CH₂)ₙ-O exhibit all-cis configuration;
R⁵ is H or OH;
m is 1, 2;
n is 1, 2;
r is 0, 1, 2, 3;
and
s is 0, 1, 2, 3;
with the proviso that r + s is 3;
including any protonated species and any deprotonated species of said compounds, including all isomeric forms of said compounds, including but not limited to enantiomers, diastereomers, regioisomers and mixtures thereof, and any pharmaceutically acceptable salt of such compounds.

Trinuclear tungsten clusters of the general formulae I or II, which are charged at physiological pH, can be neutralized by addition of suitable, physiologically biocompatible counter ions, e.g. sodium ions or suitable cations of organic bases including, among others, those of primary, secondary or tertiary amines, for example *N*-methylglucamine. Lysine, arginine or ornithine are suitable cations of amino acids, as generally are those of other basic naturally occurring amino acids.

Suitable anions are the anions of acids, inorganic acids, such as, for example, hydrochloric acid, phosphoric acid and sulfuric acid, as well as the anions of organic acids such as, for example, acetic acid, citric acid, aspartic acid, glutamic acid, among others can be used.

A preferred compound of the general formula I is:
Monoaqua-κ*O*-bis{µ₃-3,3',3"-[methylidynetris(methyleneoxy)]tripropanoato-1κ²*O*¹,*O*²:2κ²*O*^{1'},*O*³:3κ²*O*^{2'},*O*^{3'}}dihydroxido-κ²*O*-di-µ₃-oxido-1:2:3κ⁶*O*-*triangulo*-tritungsten(3*W-W*)(IV) **1e**

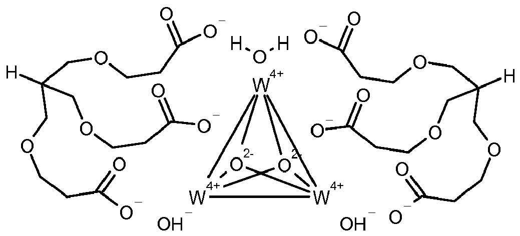

Another preferred compound of the general formula I is:
Monoaqua-κ*O*-bis{µ₃-3,3',3"-[ethyl-1,1,1-idynetris(methyleneoxy)]tripropanoato-1κ²*O*¹,*O*²:2κ²*O*^{1'},*O*³:3κ²*O*^{2'},*O*^{3'}}dihydroxido-κ²*O*-di-µ₃-oxido-1:2:3K⁶*O*-*triangulo-*tritungsten(3 *W-W*)(IV) **2c**

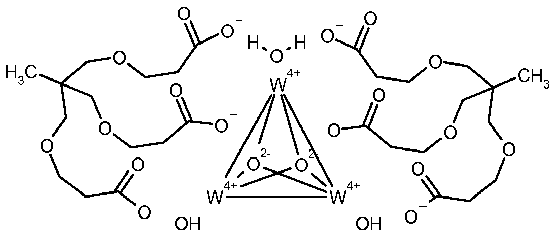

Another preferred compound of the general formula I is:
Monoaqua-κ*O*-bis{µ₃-3,3',3"-[aminomethylidynetris(methyleneoxy)]tripropanoato-1κ²*O*¹,*O*²:2κ²*O*^{1'},*O*³:3κ²*O*^{2'},*O*^{3'}}dihydroxido-κ²*O*-di-µ₃-oxido-1:2:3κ⁶*O*-*triangulo*-tritungsten(3 *W-W*)(IV) **3c**

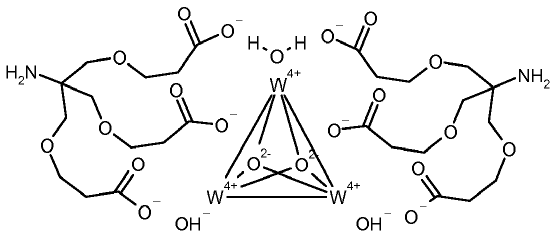

Another preferred compound of the general formula I is:
Monoaqua-κ*O*-bis{µ₃-3,3',3"-[2(2-carboxyethoxy)ethyl-1,1,1-idynetris(methylene-oxy)]tri-propanoato-1κ²*O*¹, *O*²:2κ²*O*¹,*O*³:3κ²*O*^{2'},*O*^{3'}}-dihydroxido-κ²*O*-di-µ₃-oxido-1:2:3κ⁶*O*-*triangulo-*tritungsten(3 *W*-*W*)(IV) **4b**

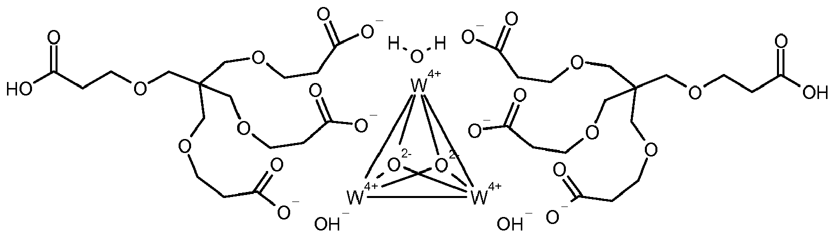

Another preferred compound of the general formula I is:
Monoaqua-κ*O*-bis(µ₃-3,3',3"-{[(2,3-dihydroxypropyl)amino]methylidynetris(methyleneoxy)}tri-propanoato-1κ²*O*¹, *O*²:2κ²*O*¹,*O*³:3κ²*O*^{2'}, *O*^{3'})-dihydroxido-κ²*O*-di-µ₃-oxido-1:2:3κ⁶*O*-*triangulo*-tri-tungsten(3 *W*-*W*)(IV) **5c**

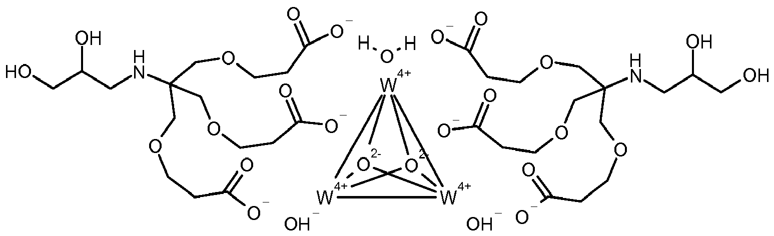

Another preferred compound of the general formula I is:
Monoaqua-κ*O*-bis(µ₃-3,3',3"-{[(1-deoxyxylit-1-yl)amino]methylidynetris(methyleneoxy)}-tripropanoato-1 κ²*O*¹,*O*²:2κ²*O*^{1'}, *O*³:3κ²*O*^{2'},*O*^{3'},*O*^{3'})-dihydroxido-κ²*O*-di-p3-oxido-1:2:3K⁶*O*-*triangulo*-tritungsten(3 *W*-*W*)(IV) **6c**

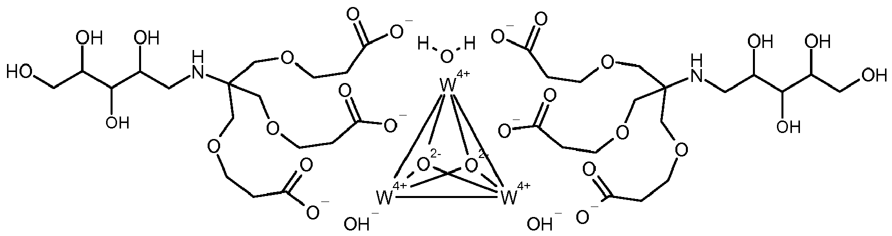

Another preferred compound of the general formula I is:
(µ₃-3,3',3"-{Aminomethylidynetris(methyleneoxy)}tripropanoato-1κ²*O*¹,*O*²:2κ²*O*^{1'}, *O*³: 3κ²*O*^{2'},*O*^{3'})monoaqua-κ*O*-dihydroxido-κ²*O*-di-µ₃-oxido-1:2:3κ⁶*O*-{µ₃-3,3',3"-[propane-1,2,3-triyltris(oxy)]tripropanoato-1κ²*O*^{1'},*O*²:2κ²*O*^{1'},*O*³:3κ²*O*^{2'},*O*^{3'}}*triangulo*-tritungsten(3 *W*-*W*)(IV) **7c**

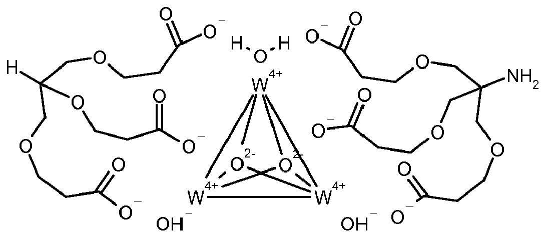

Another preferred compound of the general formula I is:
{µ₃-3,3',3"-[Aminomethylidynetris(methyleneoxy)]tripropanoato-1κ²*O*¹, *O*²:2κ²*O*^{1'}, *O*³: 3κ²*O*²,*O*^{3'}}monoaqua-κ*O*-dihydroxido-κ²*O*-{µ₃-3,3',3"-[methylidynetris(methylene-oxy)]tripropanoato-1κ²*O*¹, *O*²:2κ²*O*^{1'}, *O*³:3κ²*O*^{2'}, *O*^{3'}}di-µ₃-oxido-1:2:3κ⁶*O*-*triangulo-*tritungsten(3 *W*-*W*)(IV) **8**

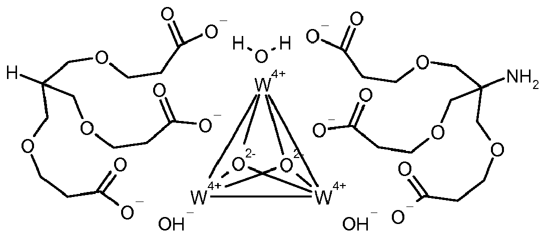

Another preferred compound of the general formula I is:
Monoaqua-κ*O*-dihydroxido-κ²*O*-(µ₃-3,3',3"-{[(2,3-dihydroxypropyl)amino]methylidynetris-(methyleneoxy)ltripropanoato-1κ²*O*¹,*O*²:2κ²*O*^{1'}, *O*³:3κ²*O*^{2'}, *O*^{3'})di-µ₃-oxido-1:2:3κ⁶*O*-{µ₃-3,3',3"-[propane-1,2,3-triyltris(oxy)]tripropanoato-1κ²*O*¹,*O*²:2κ²*O*¹,*O*³: 3κ²*O*^{2'},*O*^{3'}}*triangulo-*tritungsten(3 *W*-*W*)(IV) **9**

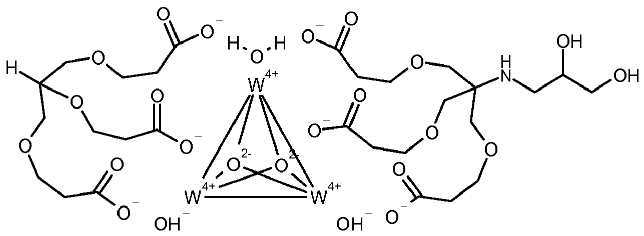

Another preferred compound of the general formula I is:
Monoaqua-κ*O*-bis{µ₃-3,3',3"-[2-hydroxyethyl-1,1,1-idynetris(methyleneoxy)-tripropanoato-1κ²*O*¹,*O*²:2κ²*O*^{1'},*O³:3*κ*²O^{2'},O^{3'}*}dihydroxido-κ²*O*-di-µ₃-oxido-1:2:3κ⁶*O*-*triangulo-*tritungsten(3 *W*-*W*)(IV) **11c**

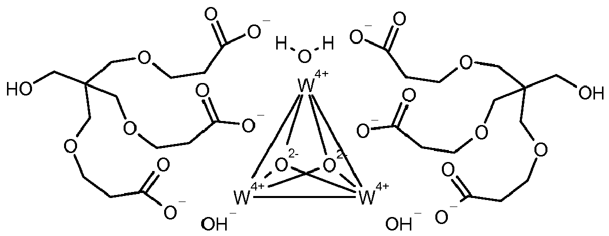

Another preferred compound of the general formula I is:
Monoaqua-κ*O*-bis(µ₃-3,3',3"-[2-methoxyethyl-1,1,1-idynetris(methyleneoxy)-tripropanoato-1κ²*O*¹,*O*²:2κ²*O*^{1'},*O*³,3κ²*O*^{2'},*O*^{3'}]dihydroxido-κ²*O*-di-µ₃-oxido-1:2:3κ⁶*O-triangulo-*tritungsten(3 *W*-*W*)(IV) **12c**

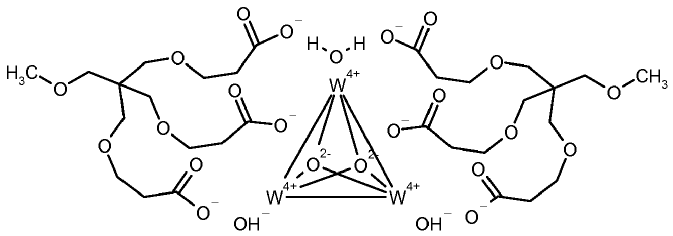

Another preferred compound of the general formula I is:
Monoaqua-κ*O*-dihydroxido-κ²*O*-bis(µ₃-3,3',3"-[hydroxymethylidyne tris(methyleneoxy)tri-propanoato-1κ²*O*¹,*O*²:2κ²*O*^{1'},*O*³3κ²*O*^{2'},*O*^{3'}]-di-µ₃-oxido-1:2:3κ⁶*O*-*triangulo*-tritungsten(3 *W-W*)(IV) **13e**

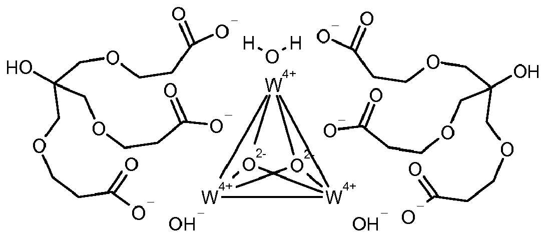

Another preferred compound of the general formula I is:
Monoaqua-κ*O*-dihydroxido-κ²*O*-(µ₃-3,3',3"-{[(2,3-dihydroxypropyl)amino]methylidynetris-(methyleneoxy)}tripropanoato-1κ²*O*¹,*O*²:2κ²*O*^{1'},*O*³:3κ²*O*^{2'}, *O*^{3'})(µ₃-3,3',3"-[hydroxy-methylidynetris(methyleneoxy)tripropanoato-1κ²*O*¹,*O*²:2κ²*O*^{1'},*O*³:3κ²*O*^{2'}, *O*^{3'}]di-µ₃-oxido-1:2:3κ⁶*O*-*triangulo*-tritungsten(3 *W*-*W*)(IV) **14**

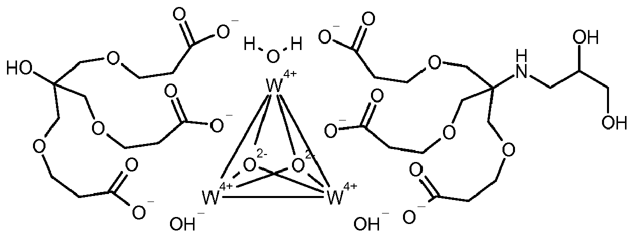

Another preferred compound of the general formula I is:
Monoaqua-κ*O*-dihydroxido-κ²*O*-bis(µ₃-3,3',3"-{[(2-hydroxyethyl)amino]methylidyne- tris-(methyleneoxy)}tripropanoato-1κ²*O*¹,*O*²:2κ²*O*^{1'},*O*³:3κ²*O*^{2'},*O*^{3'})-di-µ₃-oxido-1:2:3κ⁶*O*-*triangulo-*tritungsten(3 *W*-*W*)(IV) **15c**

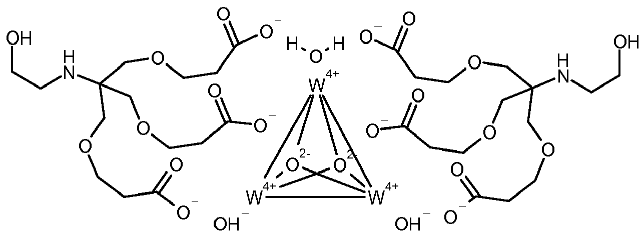

Another preferred compound of the general formula I is:
Monoaqua-κ*O*-dihydroxido-κ²*O*-(µ₃-3,3',3"-{[(2-hydroxyethy)amino]methylidynetris-(methyleneoxy)}tripropanoato-1κ²*O*¹, *O*²:2κ²*O*^{1'}, *O*³:3κ²*O*^{2'}, *O*^{3'})(µ₃-3,3',3"-[hydroxy-methylidynetris(methyleneoxy)tripropanoato-1κ²*O*¹,*O*²:2κ²*O*^{1'},*O*³:3κ²*O*^{2'}, *O*^{3'}]-di-µ₃-oxido-1:2:3κ⁶*O*-*triangulo*-tritungsten(3 *W*-*W*)(IV) **16**

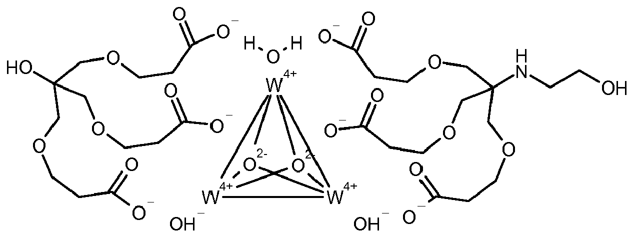

Another preferred compound of the general formula I is:
Monoaqua-κ*O*-bis{µ₃-3,3',3"-[(methoxyacetyl)aminomethylidynetris(methyleneoxy)] - tripropanoato-1κ²*O*¹,*O*²:2κ²*O*^{1'},*O*³:3κ²*O*^{2'}*O*^{3'}}dihydroxido-κ²*O*-di-µ₃-oxido-1:2:3κ⁶*O-triangulo*-tritungsten(3 *W*-*W*)(IV) **17c**

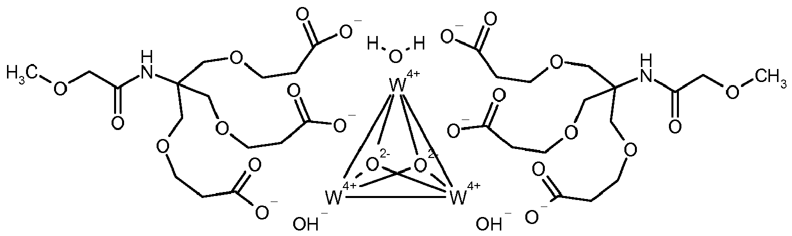

Another preferred compound of the general formula I is:
Monoaqua-κ*O*-dihydroxido-κ²*O*-(µ₃-3,3',3"-{[(2,3-dihydroxypropyl)amino]methylidynetris-(methyleneoxy)ltripropanoato-1κ²*O*¹,*O*²:2κ²*O*^{1'},*O*³:3κ²*O*^{2'},*O*^{3'}){µ₃-3,3',3"-[2-hydroxyethyl-1,1,1-idynetris(methyleneoxy)-tripropanoato-1κ²*O*¹,*O*²: 2κ²*O*^{1'},*O*³:3κ²*O*^{2'},*O*^{3'}}di-µ₃-oxido-1:2:3κ⁶*O*-*triangulo*-tritungsten(3 *W*-*W*)(IV) **18**

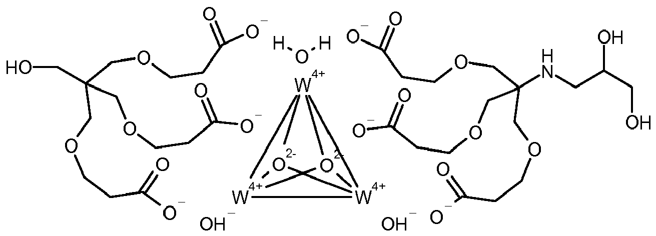

Another preferred compound of the general formula I is:
{µ₃-3,3',3"-[aminomethylidynetris(methyleneoxy)}]tripropanoato-1κ²*O*¹,*O*²:2κ²*O*^{1'},*O*³ :3κ²*O*^{2'},*O*^{3'})monoaqua-κ*O*-dihydroxido-κ²*O*-{µ₃-3,3',3"-[(methoxyacetyl)-aminomethylidynetris(methyleneoxy)]tripropanoato-1κ²*O*¹,*O*²:2κ²*O*¹,*O*³:3κ²*O*^{2'},*O*^{3'}}di-µ₃-oxido-1:2:3κ⁶*O*-*triangulo*-tritungsten(3 *W*-*W*)(IV) **19**

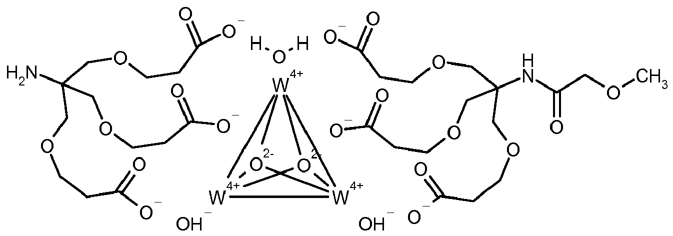

Another preferred compound of the general formula I is:
Monoaqua-κ*O*-dihydroxido-κ²*O*-(µ₃-3,3',3"-{[(1-deoxyerythritol-1-yl)amino]methylidynetris-(methyleneoxy)}tripropanoato-1κ²*O*¹,*O*²:2κ²*O*^{1'},*O*³:3κ²*O*^{2'},*O*^{3'})di-µ₃-oxido-{µ₃-3,3',3"-[propane-1,2,3-triyltris(oxy)]tripropanoato-1κ²*O*¹,*O*²:2κ²*O*^{1'},*O*³: 3κ²*O*^{2'},*O*^{3'}}-1:2:3κ⁶*O-triangulo*-tritungsten(3 *W*-*W*)(IV) **20c**

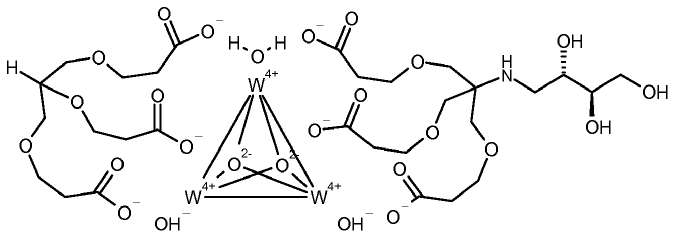

Another preferred compound of the general formula I is:
Monoaqua-κ*O*-dihydroxido-κ²*O*-(µ₃,3',3"-{[(2,3-dihydroxypropan-1-yl)amino]-methylidynetris(methyleneoxy)}tripropanoato-1κ²*O*¹,*O*²:2κ²*O*^{1'},*O*³:3κ²*O*^{2'},*O*^{3'}){µ₃-3,3',3"-[(methoxyacetyl)aminomethylidynetris(methyleneoxy)]tripropanoato-1κ²*O*¹,*O*²:2κ²*O*^{1'},*O*³:3κ^{2'},*O*^{3'}}-di-µ₃-oxido-1:2:3κ⁶*O*-*triangulo*-tritungsten(3 *W*-*W*)(IV) **21**

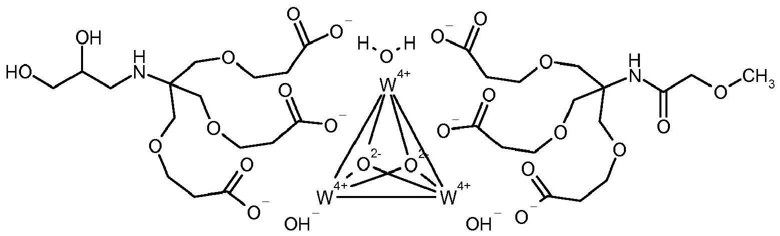

Another preferred compound of the general formula I is:
Monoaqua-κ*O*-dihydroxido-κ²*O*-(µ₃-3,3',3"-{[(1-deoxyerythritol-1-yl)amino]-methylidynetris(methyleneoxy)}tripropanoato-1κ²*O*^{1'}, *O*²:2κ²*O*^{1'},*O*³:3κ²*O*^{2'},*O*^{3'}){µ₃-3,3',3"-[(methoxyacetyl)aminomethylidynetris(methyleneoxy)]tripropanoato-1κ²O¹,O²:2κ²*O*^{1'},*O*³:3κ²*O*^{2'},*O*^{3'}}-di-µ₃-oxido-1:2:3κ⁶*O*-*triangulo*-tritungsten(3 *W*-*W*)(IV) **22**

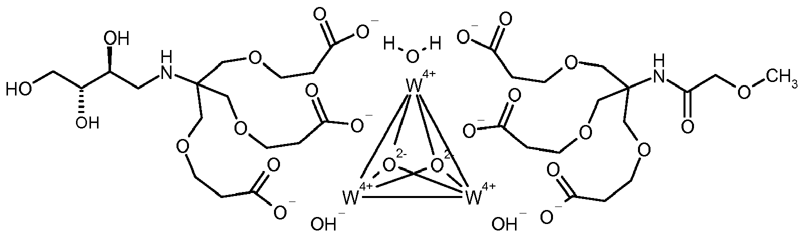

A preferred compound of the general formula II is:
Monoaqua-κ*O*-bis{µ₃-3,3',3"-[(1α,3α,5α)-cyclohexane-1,3,5-triyltris(oxy)]tripropanoato-1κ²*O*¹, *O*²:2κ²*O*^{1'}, *O*³:3κ²*O*^{2'},*O*^{3'})dihydroxido-κ²*O*-di-µ₃-oxido-1:2:3κ⁶*O*-*triangulo-*tritungsten(3 *W-W)*(IV) **10c**

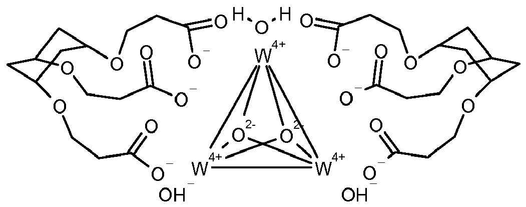

In a fourth aspect, the invention is directed to the use of compounds of the general formulae III or IV or mixtures as intermediates thereof for the preparation of trinuclear tungsten clusters comprising tridentate carboxylic acid ligands, 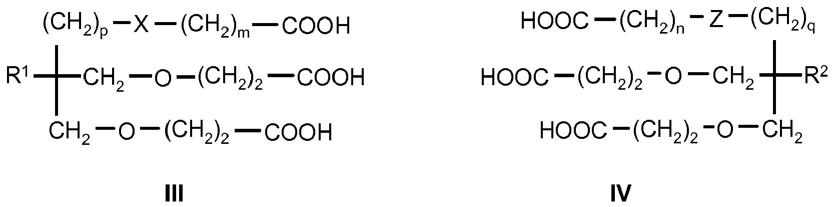 wherein
R¹ is H, CH₃, CH₂OH CH₂OCH₃ CH₂O(CH₂)₂COOH, NH₂, NH(CH₂)₂OH, NHCH₂CH(OH)CH₂OH, NHCH(CH₂OH)₂, NHCH₂(CH(OH))₂CH₂OH, NHCH₂(CH(OH))₃CH₂OH, NH(CO)CH₂OCH₃ or OH;
R² is H, CH₃, CH₂OH, CH₂OCH₃, CH₂O(CH₂)₂COOH, NH₂, NH(CH₂)₂OH, NHCH₂CH(OH)CH₂OH, NHCH(CH₂OH)₂, NHCH₂(CH(OH))₂CH₂OH, NHCH₂(CH(OH))₃CH₂OH, NH(CO)CH₂OCH₃ or OH;
X is O or NR³;
   wherein R³ is H, (CH₂)₂OH, CH₂CH(OH)CH₂OH, CH(CH₂OH)₂, CH₂(CH(OH))₂CH₂OH or CH₂(CH(OH))₃CH₂OH;
Z is O or NR⁴;
   wherein R⁴ is selected from the group comprising H, (CH₂)₂OH, CH₂CH(OH)CH₂OH, CH(CH₂OH)₂, CH₂(CH(OH))₂CH₂OH, CH₂(CH(OH))₃CH₂OH;
m is 2, 3;
n is 2, 3;
p is 0, 1;
q is 0, 1;

In a preferred embodiment the invention is directed to the use of compounds of the general formulae III or IV or mixtures thereof for the manufacture of the compounds of the general formula I.

The Process for the preparation of trinuclear tungsten clusters comprising two tridentate carboxylic acid ligands of general formula I contains the reaction of monoaqua-κ*O-*hexakis(µ-acetato-κ²*O*)-dihydroxido-κ²*O*-di-µ₃-oxido-1:2:3κ⁶*O*-*triangulo*-tritungsten(3 *W-W*)(IV) or a salt of this cluster or sodium hexakis(µ-acetato-κ²*O*)-tris(acetato-κ*O*)-di-µ₃-oxido-1:2:3κ⁶*O*-*triangulo*-tritungsten(3 *W*-*W*)(IV) in aqueous solution with an aliquot or slight excess of the desired tridentate ligand or a mixture of tridentate ligands (compounds of the general formulae III or IV), heating the components to temperatures in the range from 80° to 150°C in combination with the optional use of a pressure vessel, if necessary microwave irradiation, applying heating times from 10 minutes up to 3 days, isolation and purification of the resulting clusters by reversed phase and/or ion exchange chromatography.

In a fifth aspect, the invention is directed to the use of compounds of the general formula V as intermediates for the preparation of trinuclear tungsten clusters comprising tridentate carboxylic acid ligands, 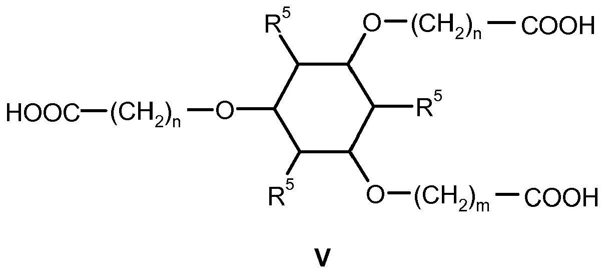 wherein
the substituents HOOC-(CH₂)₂-O exhibit all-cis configuration;
R⁵ is selected from the group comprising H or OH;
m is 1, 2
and
n is 1, 2.

including isomeric forms of said compound, such as enantiomers and diastereomers and mixtures thereof.

In a preferred embodiment the invention is directed to the use of compounds of the general formula V for the preparation/ manufacture of the compounds of the general formula II.

The Process for the preparation trinuclear tungsten clusters comprising two tridentate carboxylic acid ligands of general formula II, contains the reaction of monoaqua-κ*O-*hexakis(µ-acetato-κ²*O*)-dihydroxido-κ²*O*-di-µ₃-oxido-1:2:3κ⁶*O*-*triangulo*-tritungsten(3 *W-W*)(IV) or a salt of this cluster or sodium hexakis(µ-acetato-κ²*O*)-tris(acetato-κ*O*)-di-µ₃-oxido-1:2:3κ⁶*O*-*triangulo*-tritungsten(3 *W*-*W*)(IV) in aqueous solution in the presence of an aliquot or slight excess of the desired tridentate ligand, (compound of general formula V), heating the components to temperatures in the range from 80° to 150°C in combination with the optional use of a pressure vessel, if necessary microwave irradiation, applying heating times range from 10 minutes up to 3 days, isolation and purification of the resulting clusters by reversed phase and/or ion exchange chromatography.

In a sixth aspect, the invention is directed to the new trinuclear tungsten clusters comprising two tridentate carboxylic acid ligands of general formulae I or II, obtainable by ligand exchange reaction of monoaqua-κ*O*-hexakis(µ-acetato-κ²*O*)- dihydroxido-κ²*O*-di-µ₃-oxido-1:2:3κ⁶*O*-*triangulo*-tritungsten(3 *W*-*W*)(IV) or a salt of this cluster or sodium hexakis(µ-acetato-κ²*O*)-tris(acetato-κ*O*)-di-µ₃-oxido-1:2:3κ⁶*O*-*triangulo*-tritungsten(3 *W-W*)(IV) in aqueous solution in the presence of an aliquot or slight excess of the desired tridentate ligand or a mixture of tridentate ligands, (compounds of the general formulae III or IV or V), heating the components to temperatures in the range from 80° to 150°C in combination with the optional use of a pressure vessel, if necessary microwave irradiation, applying heating times from 10 minutes up to 3 days, isolation and purification of the resulting clusters by reversed phase and/or ion exchange chromatography.

A preferred compound of the general formula II and IV is:

3-{3-(2-Carboxyethoxy)-2-[(2-carboxyethoxy)methyl]propoxy}propanoic acid **1b**

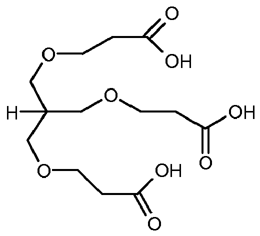

Another preferred compound of the general formula III and IV is:
3-{3-(2-Carboxyethoxy)-2-[(2-carboxyethoxy)methyl]-2-methylpropoxy}propanoic acid **2b**

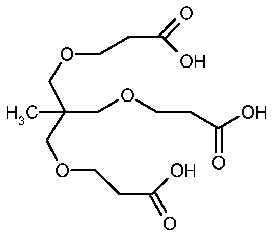

Another preferred compound of the general formula I and IV is:
3-{2-Amino-3-(2-carboxyethoxy)-2-[(2-carboxyethoxy)methyl]propoxy}propanoic acid **3b**

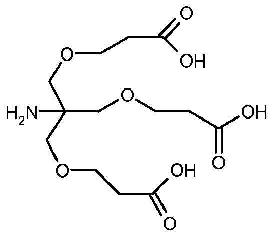

Another preferred compound of the general formula I and IV is:
3-{3-(2-Carboxyethoxy)-2,2-bis[(2-carboxyethoxy)methyl]propoxy}propanoic acid **4a**

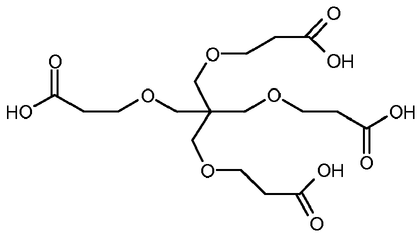

Another preferred compound of the general formula I and IV is:
3-{3-(2-Carboxyethoxy)-2-[(2-carboxyethoxy)methyl]-2-[(2,3-dihydroxypropyl)amino]-propoxy}propanoic acid **5b**

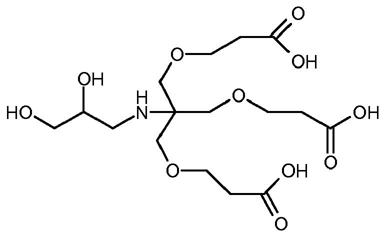

Another preferred compound of the general formula I and IV is:
1-({1,3-Bis(2-carboxyethoxy)-2-[(2-carboxyethoxy)methyl]propan-2-yl}amino)-1-deoxyxylitol **6b**

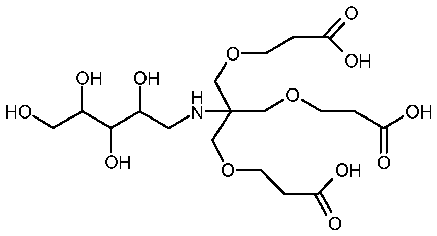

Another preferred compound of the general formula I and IV is:
3,3',3"-[Propane-1,2,3-triyltris(oxy)]tripropanoic acid **7b**

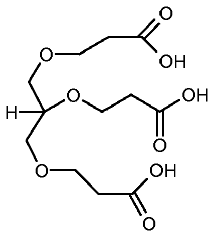

Another preferred compound of the general formula I and IV is:
3-[3-(2-Carboxyethoxy)-2-[(2-carboxyethoxy)methyl]-2-(hydroxymethyl)propoxy]propanoic acid **11b**

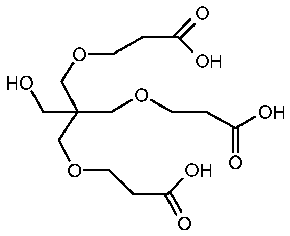

Another preferred compound of the general formula I and IV is:
3-[3-(2-Carboxyethoxy)-2-[(2-carboxyethoxy)methyl]-2-(methoxymethyl)propoxy]propanoic acid **12b**

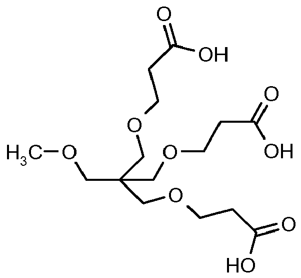

Another preferred compound of the general formula I and IV is:
3-{3-(2-Carboxyethoxy)-2-[(2-carboxyethoxy)methyl]-2-hydroxypropoxy}propanoic acid **13d**

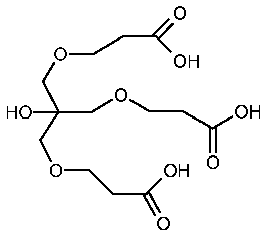

Another preferred compound of the general formula I and IV is:
3-{3-(2-Carboxyethoxy)-2-[(2-carboxyethoxy)methyl]-2-[(2-hydroxyethyl)amino]propoxy}-propanoic acid **15b**

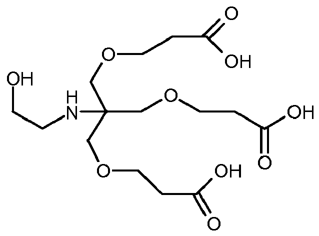

Another preferred compound of the general formula I and IV is:
3-{3-(2-Carboxyethoxy)-2-[(2-carboxyethoxy)methyl]-2-[(methoxyacetyl)amino]propoxy}-propanoic acid **17b**

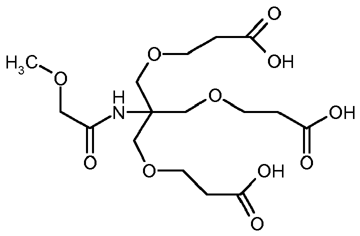

Another preferred compound of the general formula I and IV is:
1-({1,3-Bis(2-carboxyethoxy)-2-[(2-carboxyethoxy)methyl]propan-2-yl}amino)-1-deoxyerythrol **20b**

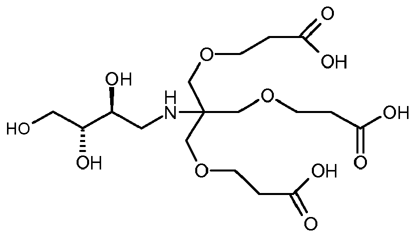

A preferred compound of the general formula V is:
3,3',3"-[(all-cis)-Cyclohexane-1,3,5-triyltris(oxy)]tripropanoic acid **10a**

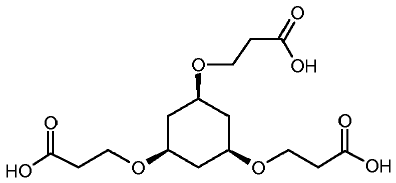

In an seventh aspect, the invention is directed to the process for the preparation of the compounds of the general formulae I and II.

In an eighth aspect, the invention is directed to compounds of general formulae I or II or mixtures thereof for the manufacture of diagnostic agents, especially of X-ray diagnostic agents for adminstration to humans or animals.

For the manufacture of diagnostic agents, for example the adminstration to human or animal subjects, the compounds of general formulae I or II or mixtures will conveniently be formulated together with pharmaceutical carriers or excipient. The contrast media of the invention may conveniently contain pharmaceutical formulation aids, for example stabilizers, antioxidants, pH adjusting agents, flavors, and the like. They may be formulated for parenteral or enteral administration or for direct administration into body cavities. For example, parenteral formulations contain a steril solution or suspension in a concentration range from 150 to 600 mg W/mL, especially 200 to 450 mgW/mL of the new bistridentate W₃O₂ clusters according to this invention. Thus the media of the invention may be in conventional pharmaceutical formulations such as solutions, suspensions, dispersions, syrups, etc. in physiologically acceptable carrier media, preferably in water for injections. When the contrast medium is formulated for parenteral administration, it will be preferably isotonic or hypertonic and close to pH 7.4.

Pharmaceutically acceptable salts of the compounds according to the invention also include salts of customary bases, such as, by way of example and by way of preference, alkali metal salts (for example sodium salts), alkaline earth metal salts (for example calcium salts) and ammonium salts, derived from ammonia or organic amines having 1 to 16 carbon atoms, such as, by way of example and by way of preference, *N*-methylglucamine.

For use as X-ray contrast agent, the media of the invention should generally have a sufficiently high percentage of tungsten, in particular a contrast medium with a high content of tungsten per molecule (minimum 35 % or higher).

### General synthesis of compounds of the invention

Tridentate carboxylic acids were synthesized (scheme 1) in analogy to published procedures (Eur. J. Inorg. Chem. 2001, 1789-1795). In the first step alkyl acrylates were added to trioles by base catalyzed Michael addition. Potential bases for this step are aqueous sodium hydroxide in DMSO, potassium *tert*-butylate or sodium hydroxide in the presence of quaternary alkyl ammonium salts. Saponification of alkyl ester was established by the use of strong acids like hydrochloric acid or trifluoromethane sulphonic acid, trifluoro acetic acid and formic acid. Basic saponification is also possible. Chemical transformations like reductive aminations, or amid formation at R1 are possible at the ester form , the free carboxylic acid form or the complexated form of the ligand. 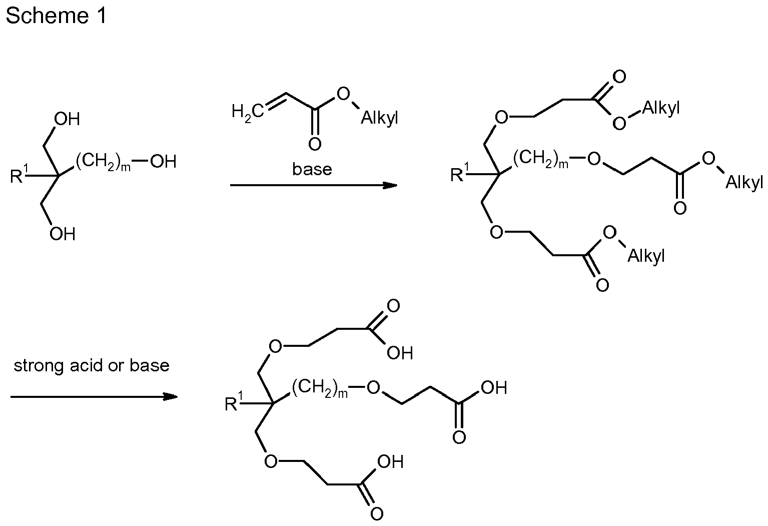 New W₃O₂ clusters were synthesized by ligand exchange reaction (WO 97/03994) of known tri-tungsten clusters like the hexa acetate or the nona acetate in aqueous solution in the presence of an aliquot or slight excess of the desired tridentate ligand or a mixture of tridentate ligands to obtain a new mixed W₃O₂ cluster. Heating temperatures generally range from 80° to 150°C in combination with the optional use of a pressure vessels. Microwave irradiation is a possible alternative to conventional heating sources. Heating times range from 10 minutes up to 3 days. Isolation and purification of the desired cluster is obtained by conventional chromatographic methods like preparative HPLC or ion exchange chromatography.

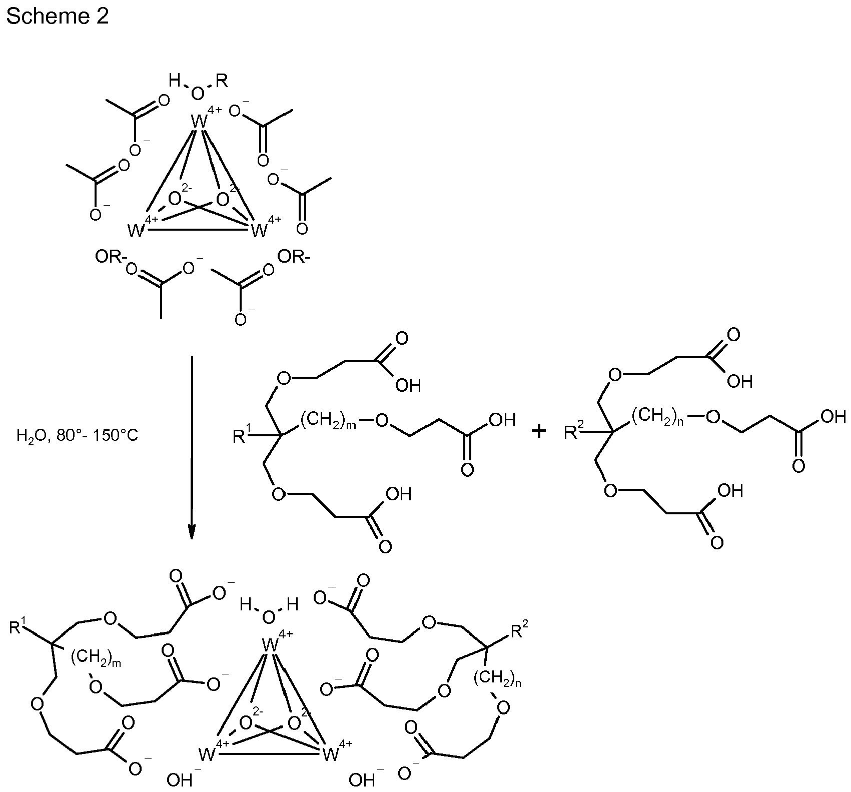

### Definitions

If chiral centres or other forms of isomeric centres are not otherwise defined in a compound according to the present invention, all forms of such stereoisomers, including enantiomers and diastereoisomers, are intended to be covered herein. Compounds containing chiral centres may be used as racemic mixture or as an enantiomerically enriched mixture or as a diastereomeric mixture or as a diastereomerically enriched mixture, or these isomeric mixtures may be separated using well-known techniques, and an individual stereoisomer maybe used alone.

Pharmaceutically acceptable salts of the compounds according to the invention include salts of mineral acids, carboxylic acids and sulfonic acids, for example salts of hydrochloric acid, sulfuric acid, phosphoric acid, methanesulfonic acid, ethanesulfonic acid, toluenesulfonic acid, benzenesulfonic acid, acetic acid, propionic acid, lactic acid, tartaric acid, malic acid, citric acid, fumaric acid, maleic acid and benzoic acid.

### Description of the Figures

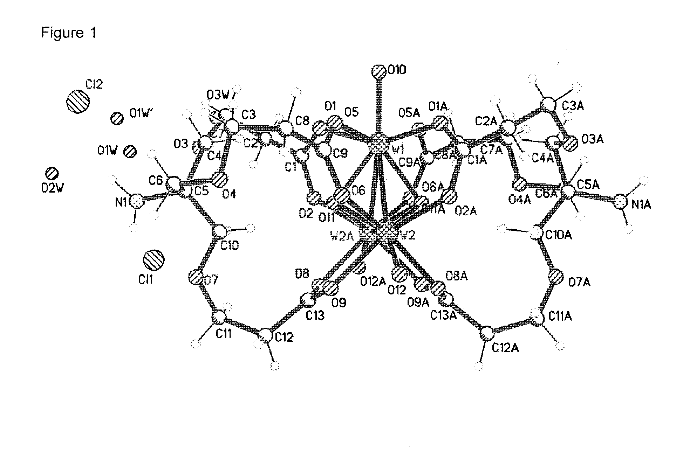

Figure 1: Crystal structure of **3d**.

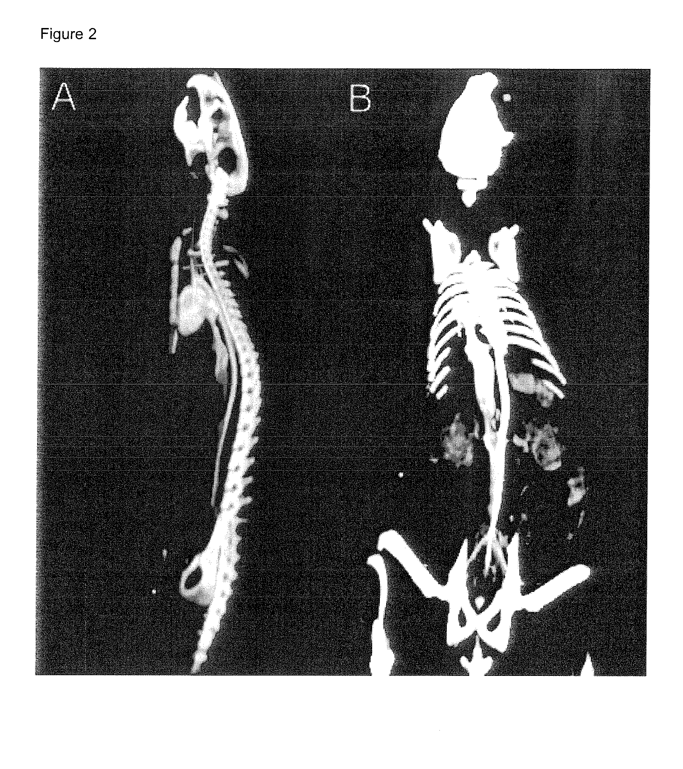

Figure 2: Preclinical CT-angiography in a rat: The CT-images shown in sagital (A) and coronary (B) view, visualized in maximum intensity projection. The heart, the main arterial and venous blood vessels and the kidneys show a high image contrast after administration of the tungsten based contrast agent.

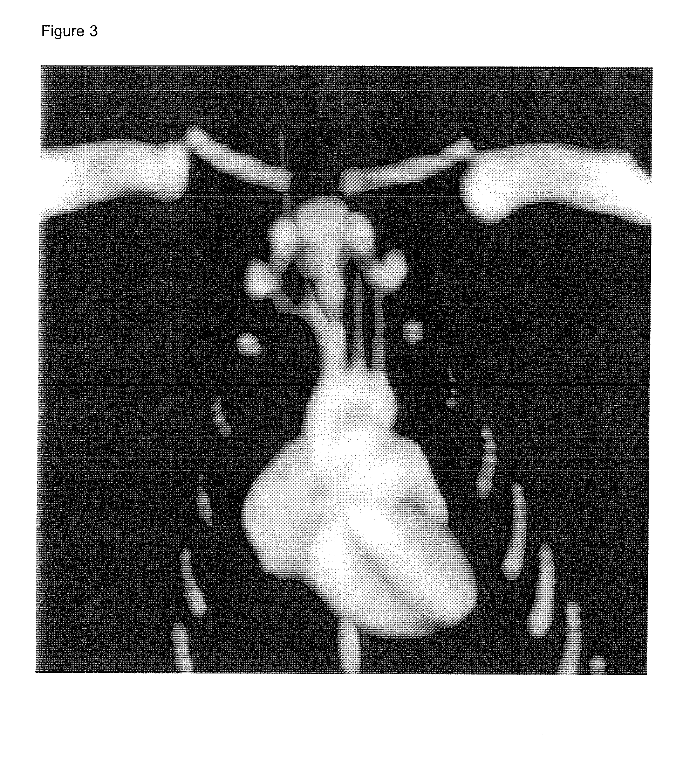

Figure 3: Preclinical CT-angiography in a rat: The image shows the thoracal region zoomed from the whole body image stack. The heart chambers and the aortic arch with the branches of the brachiocephalic, left common carotid and left subclavian arteries show a high image contrast after administration of the tungsten based contrast agent.

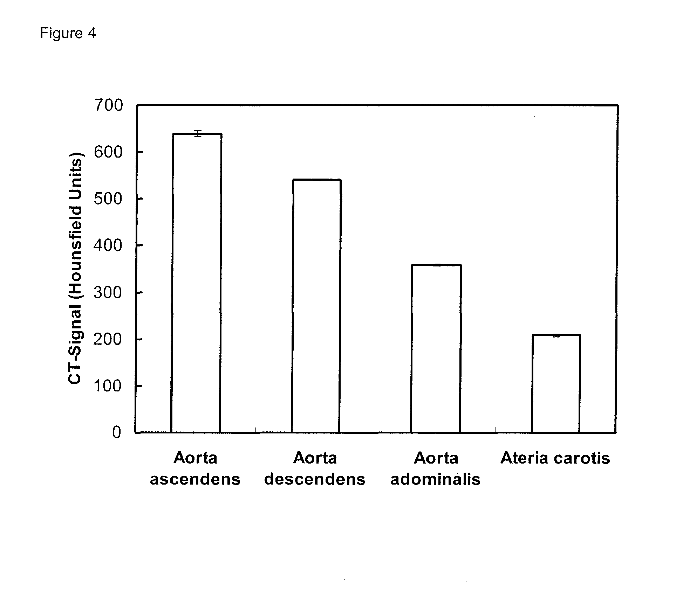

Figure 4: Preclinical x-ray CT-angiography in a rat: CT-signals after administration of a tungsten based contrast agent determined at representative anatomical positions: Aorta ascendens, Aorta descendens, Aorta abdominalis, Ateria carotis. The brackets represent the standard deviation from image analysis in three different image slices.

### Experimental Part

### Abbreviations

| | |
|---|---|
| Boc | *tert* butoxycarbonyl |
| Br | broad signal (in NMR data) |
| Cl | chemical ionisation |
| D | doublet |
| DAD | diode array detector |
| dd | doublet of doublet |
| ddd | doublet of doublet of doublet |
| dt | doublet of triplet |
| DMF | *N*,*N*-dimethylformamide |
| DMSO | dimethylsulfoxide |
| EI | electron ionisation |
| ELSD | evaporative light scattering detector |
| ESI | electrospray ionisation |
| EtOAc | ethyl acetate |
| Fmoc | fluorenylmethyloxycarbonyl |
| HPLC | high performance liquid chromatography |
| ICP-OES | Inductively coupled plasma - optical emission spectrometry |
| ICP-MS | Inductively coupled plasma - mass spectrometry |
| K₂CO₃ | potassium carbonate |
| MeCN | acetonitrile |
| MS | mass spectrometry |
| MTB | methyl *tert*-butyl ether |
| m | multiplet |
| mc | centred multiplet |
| NH₄Cl | ammonium chloride |
| NMR | nuclear magnetic resonance spectroscopy: chemical shifts (δ) are given in ppm. |
| q | quadruplett (quartet) |
| PMB | *para*-methoxybenzyl |
| rt | room temperature |
| S | singlet |
| t | triplet |
| TBAF | tetrabutylammonium fluoride |
| TBS | *tert*-butyldimethyl silyl |
| THF | tetrahydrofuran |
| THP | tetrahydropyran |
| UPLC | ultra performance liquid chromatography |

### Examples

### Example 1

### Monoaqua-κO-bis{µ₃-3,3',3"-[methylidynetris(methyleneoxy)]tripropanoato-1κ²O¹,O²:2κ²O^{1'},O³:3κ²O^{2'},O^{3'}}dihydroxido-κ²O-di-µ₃-oxido-1:2:3κ⁶O-triangulo-tritungsten(3 W-W)(IV)

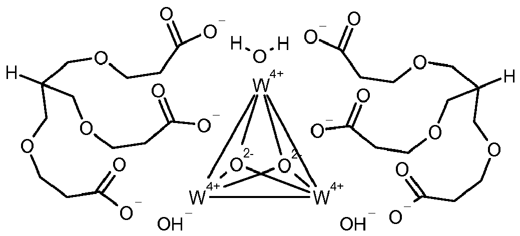

### Example 1a

### 3-{3-(3-Tert-butoxy-3-oxopropoxy)-2-[(3-tert-butoxy-3-oxopropoxy)methyl]propoxy}-propanoate

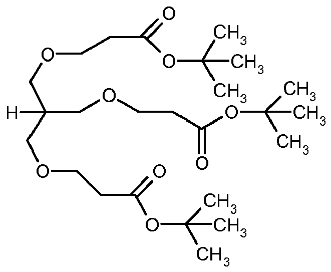

2-(Hydroxymethyl)propane-1,3-diol (2.0 g, 18.8 mmol) and *tert*-butyl prop-2-enoate are stirred in DMSO (3.3 mL) and 5 M aqueous sodium hydroxide solution (0.37 mL) at 20°C for 48 hours. The mixture was concentrated and the residue was purified by chromatography on silica gel (ethyl acetate in hexane, 20 to 100%) to yield 0.29 g of *tert-*butyl 3-{3-(3-*tert*-butoxy-3-oxopropoxy)-2-[(3-*tert*-butoxy-3-oxopropoxy)methyl]propoxy}-propanoate and 2.7 g of the di- or mono ether which were reacted under the same conditions to yield additional 4.0 g of the desired triether compound.
**¹H-NMR** (400 MHz, CDCl₃): δ = 1.47 (s, 27 H), 2.08 - 2.21 (m, 1 H), 2.46 (t, 6 H), 3.44 (d, 6 H), 3.63 (t, 6 H).

### Example 1b

### 3-{3-(2-Carboxyethoxy)-2-[(2-carboxyethoxy)methyl]propoxy}propanoic acid

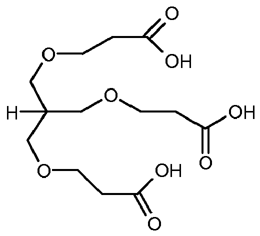

To a solution of *tert*-butyl 3-{3-(3-*tert*-butoxy-3-oxopropoxy)-2-[(3-*tert*-butoxy-3-oxopropoxy)methyl]propoxy)propanoate (4.0 g, mmol) in dioxane (20 mL) was added 6 M aqueous hydrochloric acid (6.5 mL, 39 mmol) and a 4 M solution of hydrochloric acid in dioxane (16.3 mL, 65.2 mmol). After stirring for 120 hours the solvent was removed under vacuum to yield 2.66 g 3-{3-(2-carboxyethoxy)-2-[(2-carboxyethoxy)methyl]propoxy}-propanoic acid as a yellow oil.
**¹H-NMR** (400 MHz, deuterium oxide) δ = 2.11 (m, 1 H), 2.58 (t, 6 H), 3.46 (d, 6 H), 3.69 (t, 6 H).
**LC/MS ES+** m/z 323.44 (M+1).

### Example 1c

### Sodium hexakis(µ-acetato-κ²O)-tris(acetato-κO)-di-µ₃-oxido-1:2:3κ⁶O-triangulo-tritungsten(3 W-W)(IV)

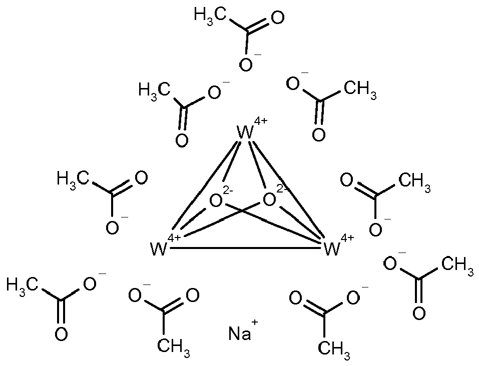

A suspension of sodium tungstate (26.4 g, 89.8 mmol) and tungsten hexacarbonyl (Aldrich, 97% purity, 130.4 g, 359.4 mmol) in acetic anhydride (2173 mL, 23.0 mol) was heated to 80°C. Air (approx. 5 L per minute) was bubbled through the mixture for 15 minutes, then the air stream was stopped and the bath temperature was raised to 145°C. After 18 hours, the mixture was cooled to rt. The yellow-brownish precipitate was filtered, washed with actic anhydride (100 mL), THF (200 mL) and *tert*-butylmethyl ether (200 mL) and dried (50°C, 50 mbar) to give sodium hexakis(µ-acetato-κ²*O*)-tris(acetato-κ*O*)-di-µ₃-oxido-1:2:3κ⁶*O*-*triangulo*-tritungsten(3 *W*-*W*)(IV) (127.8g, 112.3 mmol, 93,7% based on tungsten hexacarbonyl).
**¹H-NMR** (300 MHz, deuterium oxide) δ = 2.08 (s, 9 H), 2.28 (s, 18 H).
**LC/MS ES-** m/z 1115.03 (M-23).

### Example 1d

### Monoaqua-κO-hexakis(µ-acetato-κ²O)-dihydroxido-κ²O-di-µ₃-oxido-1:2:3κ⁶O-triangulo-tritungsten(3 W-W)(IV)

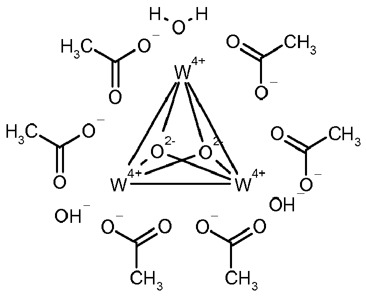

Sodium hexakis(µ-acetato-κ²*O*)-tris(acetato-κ*O*)-di-µ₃-oxido-1:2:3κ⁶*O*-*triangulo*-tritungsten(3 *W-W*)(IV) (20 g, 17.6 mmol) was refluxed in water (500mL) for 15 hours. The mixture was concentrated under vacuum and lyophilized to yield 18.3 g of raw monoaqua-κ*O*-hexakis(µ-acetato-κ²*O*)-dihydroxido-κ²*O*-di-µ₃-oxido-1:2:3κ⁶*O*-*triangulo*-tritungsten(3 *W*-*W*)(IV) together with sodium acetate and acetic acid.
**¹H-NMR** (300 MHz, deuterium oxide) δ = 2.25 (s, 18 H).
**LC/MS ES-** m/z 989.3 (M-1).

### Example 1e

### Monoaqua-κO-bis{µ₃-3,3',3"-[methylidynetris(methyleneoxy)]tripropanoato-1κ²O¹,O²:2κ²O^{1'},O³:3κ²O^{2'},O^{3'}}dihydroxido-κ²O-di-µ₃-oxido-1:2:3κ⁶O-triangulo-tritungsten(3 W-W)(IV)

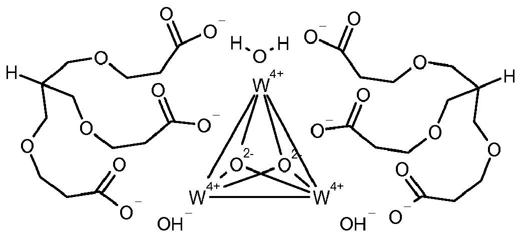

A suspension of 3-{3-(2-carboxyethoxy)-2-[(2-carboxyethoxy)methyl]propoxy]propanoic acid (2.6 g, 8.06 mmol) and monoaqua-κ*O*-hexakis(µ-acetato-κ²*O*)- dihydroxido-κ²*O*-di-µ₃-oxido-1:2:3κ⁶*O*-*triangulo*-tritungsten(3 *W*-*W*)(IV) (4.0 g, 4.03 mmol) in acetic acid (462 µL, 8.06 mmol) and water (300 mL) was separated into 10 pressure vessels which were irradiated in a micowave reactor for 15 minutes at 140°C. The reaction mixtures were filtrated and the combined fitrates were concentrated in vacuum. Separation on a preparative HPLC (acetonitrile water + acetic acid) yielded 0.56 g of monoaqua-κ*O*-bis{µ₃-3,3',3"-[methylidynetris(methyleneoxy)]tripropanoato-1κ²*O*¹, *O*²:2κ²*O*^{1'},*O*³:3κ²*O*^{2'},*O*^{3'})-dihydroxido-κ²*O*-di-µ₃-oxido-1:2:3κ⁶*O*-*triangulo*-tritungsten(3 *W*-*W*)(IV) after lyophylization.
**¹H-NMR** (400 MHz, deuterium oxide) δ = 1.71 - 1.82 (m, 2 H), 2.77 (br. t, 12 H), 3.43 (d, 12 H), 3.86 (br. t, 12 H).
**LC/MS ES-** m/z 1273.06 (M-1).

### Example 2

### Monoaqua-κO-bis{µ₃-3,3',3"-[ethyl-1,1,1-idynetris(methyleneoxy)]tripropanoato-1κ²O¹,O²:2κ²O^{1'},O³:3κ²O^{2'},O^{3'}}dihydroxido-κ²O-di-µ₃-oxido-1:2:3κ⁶O-triangulo-tritungsten(3 W-W)(IV)

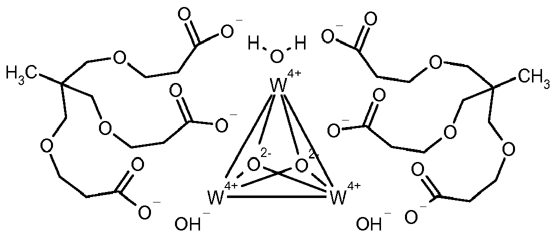

### Example 2a

### Di-tert-butyl 3,3'-{[2-(hydroxymethyl)-2-methylpropane-1,3-diyl]bis(oxy)}dipropanoate

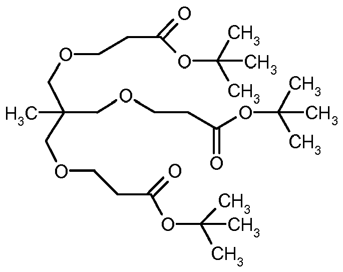

1,1,1-tris(hydroxymethyl)ethan (3.0 g, 24.9 mmol) and *tert*-butyl prop-2-enoate (14 g, 110 mmol) are stirred in DMSO (6 mL) and 5 molar aqueous sodium hydroxide solution (0.37 mL) at 20°C for 24 hours and additional 7 hours at 70°C. The mixture was concentrated and the residue was purified by chromatography on silica gel (ethyl acetate in hexane, 20 to 100%) to yield 0.89 g of di-*tert*-butyl 3,3'-{[2-(hydroxymethyl)-2-methylpropane-1,3-diyl]-bis(oxy)}dipropanoate.
**¹H-NMR** (400 MHz, CDCl₃): δ = 0.89 (s, 3 H), 1.46 (s, 27 H), 2.45 (t, 6 H), 3.26 (s, 6 H), 3.62 (t, 6 H).

### Example 2b

### 3-{3-(2-Carboxyethoxy)-2-[(2-carboxyethoxy)methyl]-2-methylpropoxy}propanoic acid

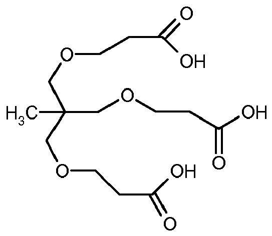

To a solution of *tert*-butyl 3-(3-(3-*tert*-butoxy-3-oxopropoxy)-2-[(3-*tert*-butoxy-3-oxo-propoxy)methyl]-2-hydroxypropoxy)propanoate (1.43 g, 2.83 mmol) in dioxane (15 mL) was added a 4 molar solution of hydrochloric acid in dioxane (5.7 mL, 22.8 mmol). After stirring for 96 hours the solvent was removed under vacuum to yield 1.09 g of 3-{3-(2-carboxyethoxy)-2-[(2-carboxyethoxy)methyl]-2-methylpropoxy)propanoic acid.
**¹H-NMR** (300 MHz, DMSO-*d*₆) δ = 0.86 (s, 3 H), 2.64 (t, 6 H), 3.34 (s, 6 H), 3.74 (t, 6 H).

### Example 2c

### Monoaqua-κO-bis{µ₃-3,3',3"-[ethyl-1,1,1-idynetris(methyleneoxy)]tripropanoato-1κ²O¹,O²:2κ²O^{1'},O³:3κ²O^{2'},O^{3'}}dihydroxido-κ²O-di-µ₃-oxido-1:2:3κ⁶O-triangulo-tritungsten(3 W-W)(IV)

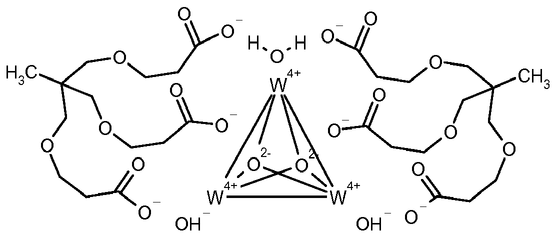

A suspension of 3-{3-(2-carboxyethoxy)-2-[(2-carboxyethoxy)methyl]-2-methylpropoxy}-propanoic acid (272 mg, 808 µmol) and monoaqua-κ*O*-hexakis(µ-acetato-κ²*O*)-dihydroxidO-κ²*O*-di-µ₃-oxido-1:2:3κ⁶*O*-*triangulo*-tritungsten**(3 *W*-*W*-**)(**IV**) (400 mg, 404 µmol) in acetic acid (46 µL, 0.8 mmol) and water (30 mL) was irradiated in a micowave reactor for 15 minutes at 140°C. The reaction mixtures were filtrated and the fitrates was concentrated in vacuum. Separation on a preparative HPLC (acetonitrile water + acetic acid) yielded 2.3 mg monoaqua-κ*O*-bis{µ3-3,3',3"-[ethyl-1,1,1-idynetris(methyleneoxy)]-tripropanoato-1κ²*O*¹,*O*²:2κ²*O*^{1'},*O*³:3κ²*O*^{2'},*O*^{3'}}dihydroxido-κ²*O*-di-µ₃-oxido-1:23κ⁶*O*-*triangulo*-tritungsten(3 *W-W*)(IV).
**¹H-NMR** (300 MHz, DMSO-d₆) δ = 0.68 (s, 6 H), 2.72 (br. t, 12 H), 3.11 (s, 12 H), 3.72 (br. t, 12 H).
**LC/MS ES-** m/z 1300.75 (M-1).

### Example 3

### Monoaqua-κO-bis{µ₃-3,3',3"-[aminomethylidynetris(methyleneoxy)]tripropanoato-1κ²O¹,O²:2κ²O^{1'},O³:3κ²O^{2'},O^{3'}}dihydroxido-κ²O-di-µ₃-oxido-1:2:3κ⁶O-triangulo-tritungsten(3 W-W)(IV)

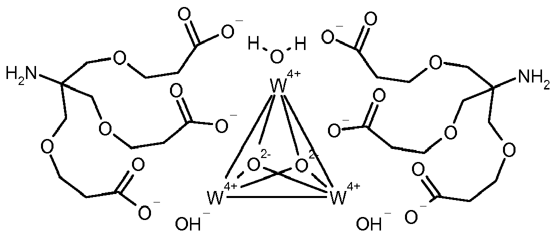

### Example 3a

### Tert-butyl 3-{2-amino-3-(3-tert-butoxy-3-oxopropoxy)-2-[(3-tert-butoxy-3-oxo-propoxy)methyl]propoxy}propanoate

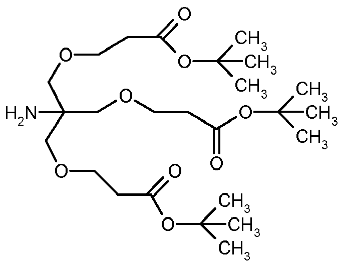

*Tert*-butyl 3-{2-amino-3-(3-*tert*-butoxy-3-oxopropoxy)-2-[(3-*tert*-butoxy-3-oxopropoxy)-methyl]propoxylpropanoate was synthesized following the procedure of Cardona, C.M.; Gawley, R.E. J. Org. Chem. 2002, 67, 1411 - 1413 yielding 16.2 g product starting from 15.7 g tris(hydroxymethyl)aminomethane after chromatography on silica gel.
**¹H-NMR** (400 MHz, CDCl₃) δ = 1.46 (s, 27 H), 2.46 (t, 6 H), 3.32 (s, 6 H), 3.65 (t, 6 H).

### Example 3b

### 3-{2-Amino-3-(2-carboxyethoxy)-2-[(2-carboxyethoxy)methyl]propoxy}propanoic acid

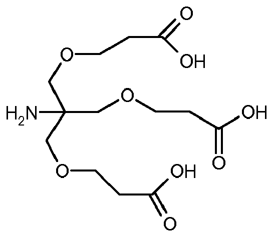

To a solution of *tert*-butyl 3-{2-amino-3-(3-*tert*-butoxy-3-oxopropoxy)-2-[(3-tert-butoxy-3-oxopropoxy)methyl]propoxy}propanoate (9.3 g, 18.9 mmol) in dioxane (16.4mL) was added 6 molar aqueous hydrochloric acid (2.6 mL, 15.5 mmol) and a 4 molar solution of hydrochloric acid in dioxane (6.5 mL, 25.9 mmol). After stirring for 96 hours the solvent was removed under vacuum to yield the hydro chloride salt, which was treated with ion exchange resin IRA 67 for three days. The ion exchange resin was washed with water followed by 20% aqueous acetic acid. The washing solutions were fractionated and fractions where no chloride was detected were concentrated under vacuum to yield 1.0 g 3-{2-amino-3-(2-carboxyethoxy)-2-[(2-carboxyethoxy)methyl]propoxy}propanoic acid as free base.
**¹H-NMR** (300 MHz, deuterium oxide) δ = 2.55 (t, 6 H), 3.59 (s, 6 H), 3.66 - 3.81 (m, 6 H).

### Example 3c

### Monoaqua-κO-bis{µ₃-3,3',3"-[aminomethylidynetris(methyleneoxy)]tripropanoato-1κ²O¹,O²:2κ²O^{1'},O³:3κ²O^{2'},O^{3'}}dihydroxido-κ²O-di-µ₃-oxido-1:2:3κ⁶O-triangulo-tritungsten(3 W-W)(IV)

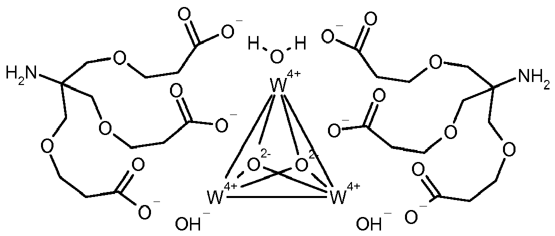

A suspension of 3-{2-amino-3-(2-carboxyethoxy)-2-[(2-carboxyethoxy)methyl]propoxy}-propanoic acid (2.94 g, 8.73 mmol) and monoaqua-κ*O*-hexakis(µ-acetato-κ²*O*)-dihydroxido-κ²*O*-di-µ₃-oxido-1:2:3κ⁶*O*-*triangulo*-tritungsten(3 *W*-*W*)(IV) (4.32 g, 4.36 mmol) in acetic acid (500 µL, 8.73 mmol) and water (136 mL) was separated into 10 pressure vessels which were irradiated in a microwave reactor for 15 minutes at 140°C. The reaction mixtures were filtrated and the combined filtrates were concentrated in vacuum. Separation on a preparative HPLC (acetonitrile / water + acetic acid) yielded monoaqua-κ*O*-bis{µ₃-3,3',3"-[aminomethylidynetris(methyleneoxy)]tripropanoato-1κ²*O*¹,*O*²:2κ²*O*^{1'},*O*^{3'}:3κ²*O*^{2'},*O*^{3'})dihydroxido-κ₂*O*-di-µ₃-oxido-1:2:3κ⁶*O*-*triangulo*-tritungsten (3 *W*-*W*)(IV).
**¹H-NMR** (300 MHz, deuterium oxide) δ = 2.83 (t, 12 H), 3.55 (s, 12 H), 3.93 (t, 12 H).
**LC/MS ESI-** m/z 1303 (M-1).

### Example 3d

### Triaqua-κ³O-bis{µ₃-3,3',3"-[aminomethylidynetris(methyleneoxy)]tripropanoato-1κ²O¹,O²:2κ²O^{1'},O³:3κ²O^{2'},O^{3'}}di-µ₃-oxido-1:2:3κ⁶O-triangulo-tritungsten(3 W-W)(IV) dichloride

For the generation of crystalline triaqua-κ³*O*-bis{µ₃-3,3',3"-[aminomethylidynetris(methyleneoxy)]tripropanoato-1κ²*O*¹,*O*²:2κ²*O*^{1'},*O*³:3κ²*O*^{3'},*O*^{3'})di-µ₃-oxido-1:2:3κ⁶*O-triangulo*-tritungsten(3 *W*-*W*)(IV) dichloride the tungsten complex was prepared in analogy to 3c starting from 3-{2-amino-3-(2-carboxyethoxy)-2-[(2-carboxyethoxy)methyl]propoxy}-propanoic acid in form of its hydrochloride prepared in analogy to 3b without ion exchange treatment. Final crystallization was done from a water acetonitile mixture.

| **Crystal data and structure refinement:**. | | |
|---|---|---|
| Empirical formula | C₂₆H₄₀Cl₂N₂O₂₈W₃ | |
| Formula weight | 1451.05 | |
| Temperature | 100(2) K | |
| Wavelength | 1.54178 Å | |
| Crystal system | Orthorhombic | |
| Space group | Pnna | |
| Unit cell dimensions | a = 8.73990(10) Å | α= 90°. |
| | b = 27.9831 (3) Å | β=90°. |
| | c = 19.0848(2) Å | γ = 90°. |
| Volume | 4667.56(9) Å³ | |
| Z | 4 | |
| Density (calculated) | 2.065 Mg/m³ | |
| Absorption coefficient | 15.220 mm⁻¹ | |
| F(000) | 2760 | |
| Crystal size | 0.20 x 0.03x 0.02 mm³ | |
| Theta range for data collection | 3.16 to 65.70°. | |
| Index ranges | -8<=h<=10, -32<=k<=31, -13<=I<=22 | |
| Reflections collected | 27656 | |
| Independent reflections | 4022 [R(int) = 0.0571] | |
| Completeness to theta = 65.70° | 99.3 % | |
| Absorption correction | None | |
| Refinement method | Full-matrix least-squares on F² | |
| Data / restraints / parameters | 4022 / 0 / 278 | |
| Goodness-of-fit on F² | 1.089 | |
| Final R indices [I>2sigma(I)] | R1 = 0.0511, wR2 = 0.1364 | |
| R indices (all data) | R1 = 0.0585, wR2 = 0.1423 | |
| Largest diff. peak and hole | 4.513 and -0.928 e.Å⁻³ | |

### Atomic coordinates (x 10⁴) and equivalent isotropic displacement parameters (Å²x 10³)

for 3d U(eq) is defined as one third of the trace of the orthogonalized U^{ij} tensor.

| | x | y | z | U(eq) |
|---|---|---|---|---|
| W(1) | -1257(1) | -2500 | 2500 | 29(1) |
| W(2) | 1519(1) | -2773(1) | 1901(1) | 30(1) |
| Cl(1) | 2500 | 0 | 2362(2) | 46(1) |
| Cl(2) | -2500 | 0 | 286(2) | 31(1) |
| O(1) | -1824(7) | -1782(2) | 2748(3) | 33(1) |
| O(2) | 415(7) | -1566(2) | 3213(3) | 34(1) |
| O(3) | -1201 (7) | -643(2) | 2082(4) | 40(2) |
| O(4) | -57(6) | -1554(2) | 305(3) | 32(1) |
| O(5) | -1802(7) | -2362(2) | 1444(3) | 36(1) |
| O(6) | 407(7) | -2594(2) | 957(3) | 32(1) |
| O(7) | 2938(6) | -972(2) | 1172(3) | 33(1) |
| O(8) | 3246(7) | -1848(2) | 2557(3) | 33(1) |
| O(9) | 3226(7) | -2282(2) | 1588(3) | 33(1) |
| O(10) | -3512(9) | -2500 | 2500 | 35(2) |
| O(11) | 599(6) | -2137(2) | 2145(3) | 28(1) |
| O(12) | 2733(7) | -3126(2) | 1144(3) | 39(2) |
| N(1) | 545(8) | -346(3) | 1050(4) | 31(2) |
| C(1) | -964(11) | -1477(3) | 3043(4) | 35(2) |
| C(2) | -1573(12) | -994(4) | 3213(5) | 43(2) |
| C(3) | -2304(10) | -744(4) | 2617(5) | 35(2) |
| C(4) | -1279(10) | -940(3) | 1484(5) | 33(2) |
| C(5) | 226(9) | -871(3) | 1082(4) | 32(2) |
| C(6) | 84(10) | -1054(3) | 334(5) | 34(2) |
| C(7) | -1582(9) | -1728(3) | 186(5) | 32(2) |
| C(8) | -1496(10) | -2265(3) | 220(5) | 32(2) |
| C(9) | -931(10) | -2419(3) | 912(5) | 32(2) |
| C(10) | 1528(9) | -1112(3) | 1479(5) | 31(2) |
| C(11) | 4231(9) | -1066(3) | 1607(5) | 32(2) |
| C(12) | 4759(10) | -1581(3) | 1603(5) | 32(2) |
| C(13) | 3655(9) | -1932(3) | 1935(4) | 29(2) |
| O(1W) | -1420(40) | 1434(14) | 5000(20) | 147(15) |
| O(1W') | -2637(18) | 1511(5) | 4753(8) | 47(6) |
| O(2W) | -549(15) | 1993(6) | 3860(9) | 128(5) |
| O(3W) | -2500 | 0 | 4273(11) | 202(16) |

Hydrogen coordinates (x 10⁴) and isotropic displacement parameters (Å²x 10³) for **3d**.

| | x | y | z | U(eq) |
|---|---|---|---|---|
| H(1A) | 1403 | -227 | 1244 | 37 |
| H(1B) | -118 | -150 | 832 | 37 |
| H(2A) | -2332 | -1026 | 3595 | 52 |
| H(2B) | -723 | -794 | 3390 | 52 |
| H(3A) | -3131 | -946 | 2422 | 42 |
| H(3B) | -2767 | -442 | 2783 | 42 |
| H(4A) | -2161 | -849 | 1187 | 39 |
| H(4B) | -1400 | -1279 | 1625 | 39 |
| H(6A) | -824 | -906 | 111 | 40 |
| H(6B) | 998 | -955 | 64 | 40 |
| H(7A) | -1956 | -1625 | -280 | 39 |
| H(7B) | -2288 | -1605 | 548 | 39 |
| H(8A) | -2524 | -2402 | 136 | 38 |
| H(8B) | -801 | -2384 | -150 | 38 |
| H(10A) | 1504 | -1017 | 1978 | 38 |
| H(10B) | 1416 | -1464 | 1453 | 38 |
| H(11A) | 3974 | -975 | 2094 | 39 |
| H(11 B) | 5090 | -861 | 1452 | 39 |
| H(12A) | 5749 | -1601 | 1853 | 39 |
| H(12B) | 4939 | -1679 | 1111 | 39 |

Figure 1 shows the crystal structure of **3d**.

### Example 4

### Monoaqua-κO-bis{µ₃-3,3',3"-[2(2-carboxyethoxy)ethyl-1,1,1-idynetris(methylene-oxy)]tripropanoato-1κ²O¹,^{d}:2κ²O^{2'},O^{1'}O³:3κ²O^{2'},O^{3'}}-dihydroxido-κ²O-di-µ₃-oxido-1:2:3κ⁶O-triangulo-tritungsten(3 W-W)(IV)

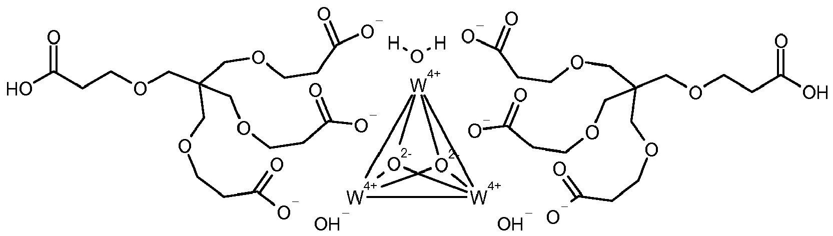

### Example 4a

### 3-{3-(2-Carboxyethoxy)-2,2-bis[(2-carboxyethoxy)methyl]propoxy}propanoic acid

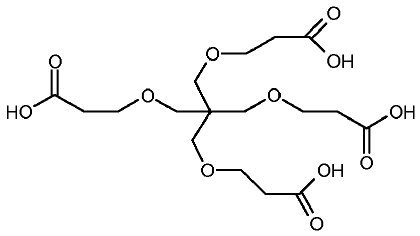

3-{3-(2-Carboxyethoxy)-2,2-bis[(2-carboxyethoxy)methyl]propoxy}propanoic acid was synthesized analogous to Weizman et al. J. Am. Chem. Soc. 1996, 118 12368-12375.
**¹H-NMR** (400 MHz, deuterium oxide) δ = 2.58 (t, 8 H), 3.34 (s, 8 H), 3.67 (t, 8 H).

### Example 4b

### Monoaqua-κO-bis{µ₃-3,3',3"-[2(2-carboxyethoxy)ethyl-1,1,1-idynetris(methylene-oxy)]tripropanoato-1κ²O¹,O²:2κ²O^{1'},O³:3κ²O^{2'},O^{3'}}-dihydroxido-κ²O-di-µ₃-oxido-1:2:3κ⁶O-triangulo-tritungsten(3 W-W)(IV)

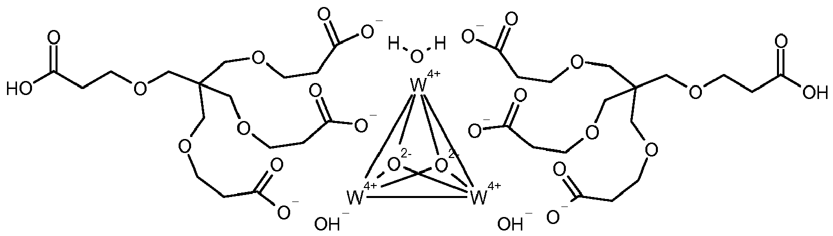

A suspension of 3-{3-(2-carboxyethoxy)-2,2-bis[(2-carboxyethoxy)methyl]propoxy}-propanoic acid (0.34 g, 808 µmol) and monoaqua-κ*O*-hexakis(µ-acetato-κ²*O*)-dihydroxido-κ²*O*-di-µ₃-oxido-1:2:3κ⁶*O*-*triangulo*-tritungsten(3 *W*-*W*)(IV) (400 mg, 404 µmol) in acetic acid (93 µL, 1.6 mmol) and water (30 mL) was irradiated in a microwave reactor for 15 minutes at 140°C. The reaction mixtures were filtrated and the filtrates was concentrated in vacuum. Separation on a preparative HPLC (acetonitrile water + acetic acid) yielded 55.1 mg monoaqua-κ*O*-bis[µ₃-3,3',3"-[2(2-carboxyethoxy)ethyl-1,1,1-idynetris(methyleneoxy)]tripropanoato-1κ²*O*¹,*O*²:2κ²*O*^{1'},*O*³:3κ²*O*^{2'},*O*^{3'})-dihydroxido-κ²*O*-di-µ₃-oxido-1:2:3κ⁶*O*-*triangulo-*tritugsten(3 *W*-*W*)(IV).
**¹H-NMR** (400 MHz, deuterium oxide) δ = 2.52 (t, 4 H), 2.72 (br. t, 12 H), 3.28 (s, 4 H), 3.32 (s, 12 H), 3.63 (t, 4 H), 3.78 (br. t, 12 H).
**LC/MS ES-** m/z 1477 (M-1).

### Example 5

### Monoaqua-κO-bis(µ₃-3,3',3"-{[(2,3-dihydroxypropyl)amino]methylidynetris(methylene-oxy)}tripropanoato-1κ²O¹,O²:2κ²O^{1'},O³:3κ²O^{3'},O^{3'})-dihydroxido-κ²O-di-µ₃-oxido-1:2:3κ⁶O-triangulo-tritungsten(3 W-W)(IV)

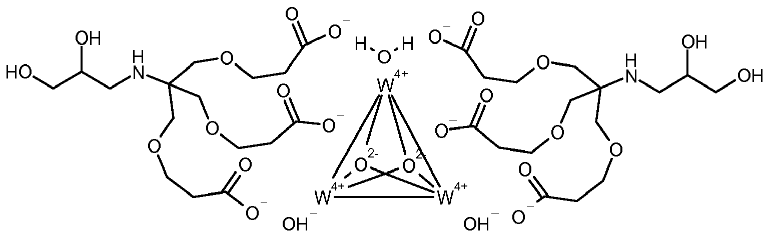

### Example 5a

### 3-{3-(2-carboxyethoxy)-2-[(2-carboxyethoxy)methyl]-2-[(2,3-dihydroxypropyl)amino]-propoxy}propanoic acid

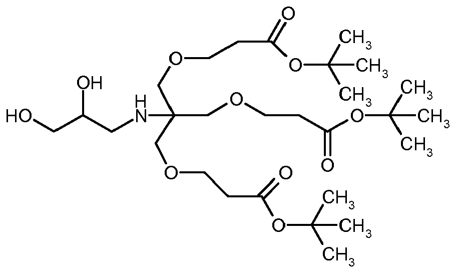

*Tert*-butyl 3-{2-amino-3-(3-*tert*-butoxy-3-oxopropoxy)-2-[(3-*tert*-butoxy-3-oxopropoxy)-methyl]propoxylpropanoate (25 g, 49.4 mmol) and *rac*-2,3-dihydroxypropanal were shaken under an hydrogen atmosphere in methanol in presence of 10% palladium on charcoal (263 mg) for 18 hours at room temperature. The mixture was filtered through cellites and concentrated in vacuum. The residue was purified by chromatography on silica gel (ethyl acetate in hexane, 20 to 100%) to yield 12.47 g of *tert*-butyl 3-{3-(3-*tert-*butoxy-3-oxopropoxy)-2-[(3-*tert*-butoxy-3-oxopropoxy)methyl]-2-[(2,3-dihydroxypropyl)-amino]propoxylpropanoate as light yellow oil.
**¹H-NMR** (400 MHz, CDCl₃): δ = 1.46 (s, 27 H), 2.47 (t, 6 H), 2.76 (dd, 1 H), 2.87 (dd, 1 H), 3.37 (s, 6 H), 3.54 - 3.79 (m, 3 H), 3.64 (t, 6H).

### Example 5b

### 3-{3-(2-Carboxyethoxy)-2-[(2-carboxyethoxy)methyl]-2-[(2,3-dihydroxypropyl)-amino]propoxy}propanoic acid

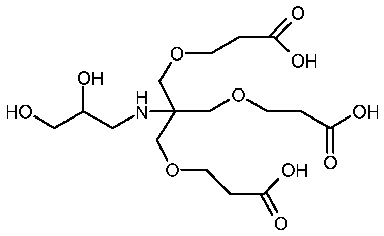

To a solution of *tert*-butyl 3-(3-(3-*tert*-butoxy-3-oxopropoxy)-2-[(3-*tert*-butoxy-3-oxo-propoxy)methyl]-2-[(2,3-dihydroxypropyl)amino]propoxy}propanoate (4.8 g, 8.3 mmol) in dioxane (48 mL) was added 6 molar aqueous hydrochloric acid (6.6 mL, 39.6 mmol) and a 4 molar solution of hydrochloric acid in dioxane (16.6 mL, 66 mmol). After stirring for 40 hours the solvent was removed under vacuum to yield the hydro chloride salt, which was treated with ion exchange resin IR 67 (200mL) in water for two hours. The ion exchange resin was washed with water followed by 15 % aqueous acetic acid. The washing solutions were fractionated and fractions with a negative chloride detection were combined and concentrated under vacuum to yield 3.18 g 3-{3-(2-carboxyethoxy)-2-[(2-carboxyethoxy)methyl]-2-[(2,3-dihydroxypropyl)amino]propoxylpropanoic acid as free base.
**¹H-NMR** (300 MHz, deuterium oxide) δ = 2.54 (t, 6 H), 3.05 (dd, 1 H), 3.23 (dd, 1 H), 3.54-3.58 (m, 2 H), 3.65 (s, 6H), 3.71 (t, 6 H), 3.83 - 3.95 (m, 1 H).

### Example 5c

### Monoaqua-κO-bis(µ₃-3,3',3"-{[(2,3-dihydroxypropyl)amino]methylidynetris(methylene-oxy)}tripropanoato-1κ²O¹,O²:2κ²O^{1'},O³:3κ²O^{2'},O^{3'})-dihydroxido-κ²O-di-µ₃-oxido-1:2:3κ⁶O-triangulo-tritungsten(3 W-W)(IV)

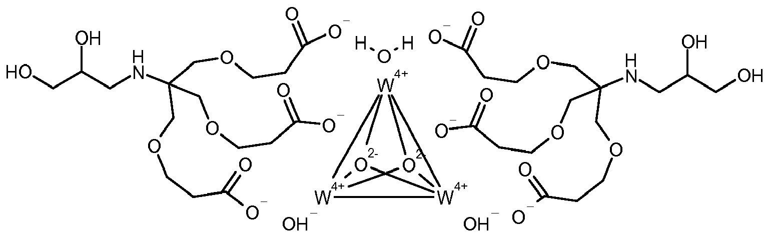

A suspension of 3-{3-(2-carboxyethoxy)-2-[(2-carboxyethoxy)methyl]-2-[(2,3-dihydroxypropyl)amino]propoxylpropanoic acid (1.58 g, 3.84 mmol) and monoaqua-κ*O*-hexakis(µ-acetato-κ²*O*)-dihydroxido-κ²*O*-di-µ₃-oxido-1:2:3κ⁶*O*-*triangulo*-tritungsten(3 *W-W*)(IV) (1.9 g, 1.92 mmol) in acetic acid (220 µL, 3.84 mmol) and water (136 mL) was separated into 5 pressure vessels which were irradiated in a microwave reactor for 15 minutes at 140°C. The reaction mixtures were filtrated and the combined filtrates were concentrated in vacuum. Separation on a preparative HPLC (acetonitrile water + acetic acid) yielded 0.24 g monoaqua-κ*O*-bis(µ₃-3,3',3"-{[(2,3-dihydroxypropyl)amino]methylidynetris(methylene-oxy)ltripropanoato-1κ²*O*¹,*O*²:2κ²*O*^{1'}, *O*³:3κ²*O*^{2'},*O*^{3'})-dihydroxido-κ²*O*-di-µ₃-oxido-1:2:3κ⁶*O-triangulo*-tritungsten(3 *W-W*)(IV).
**¹H-NMR** (300 MHz, deuterium oxide) δ = 2.80 (t, 12 H), 2.99 - 3.10 (m, 2 H), 3.14 - 3.25 (m, 2 H), 3.48 - 3.66 (m, 16 H), 3.89 (t, 14 H).
**LC/MS ES-** m/z 1451.1 (M-1).

### Example 6

### Monoaqua-κO-bis(µ₃-3,3',3"-{[(1-deoxyxyt-1-yl)amino]methylidynetris(methylene-oxy)}tripropanoato-1κ²O¹,O²:κ²O^{1'},O³:κ²O^{2'},O^{3'})-dihydroxido-κ²O-di-µ₃-oxido-1:2:3κ⁶O-triangulo-tritungsten(3 W-W)(IV)

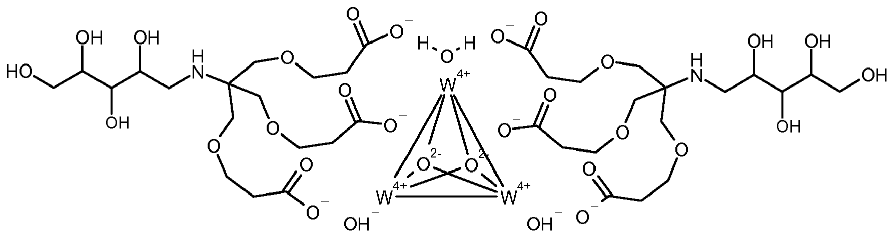

### Example 6a

### 1-({9-[(3-tert-butoxy-3-oxopropoxy)methyl]-2,2,16,16-tetramethyl-4,14-dioxo-3,7,11,15-tetraoxaheptadecan-9-yl}amino)-1-deoxyxylitol

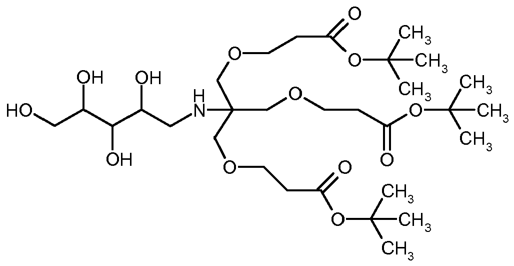

*Tert*-butyl 3-{2-amino-3-(3-*tert*-butoxy-3-oxopropoxy)-2-[(3-*tert*-butoxy-3-oxopropoxy)-methyl]propoxylpropanoate (0.5 g, 0.99 mmol) and D/L-xylose (0.3 g, were stirred in methanol for 0.5 hours at room temperature. Sodium cyano borohydride (81 mg, 1.29 mmol) was added and stirring was continued for 22 hours at room temperature and 5 days at 60°C (additional addition of 0.3 g xylose after day 4). The pH of the solution was adjusted to 4 with 2 M hydrochloric acid and the solution was concentrated under reduced pressure. The residue was purified by chromatography on silica gel (methanol in dichloromethane, 0 to 20%) to yield 310 mg of 1-({9-[(3-*tert*-butoxy-3-oxopropoxy)methyl]-2,2,16,16-tetramethyl-4,14-dioxo-3,7,11,15-tetraoxaheptadecan-9-yl}amino)-1-deoxy-xylitol.
**¹H-NMR** (400 MHz, CDCl₃): δ = 1.40 (s, 27 H), 2.40 (t, 6 H), 2.68 (br, 1 H), 3.17 (d, 2 H), 3.22 - 3.58 (m, 5 H), 3.55 (t, 6 H), 4.43 (br, 1 H).

### Example 6b

### 1-({1,3-Bis(2-carboxyethoxy)-2-[(2-carboxyethoxy)methyl] propan-2-yl}amino)-1-deoxyxylitol

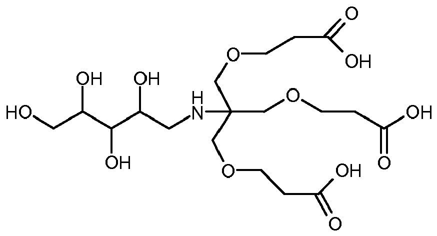

To a solution of *tert*-butyl 1-({9-[(3-*tert*-butoxy-3-oxopropoxy)methyl]-2,2,16,16-tetramethyl-4,14-dioxo-3,7,11,15-tetraoxaheptadecan-9-yl}amino)-1-deoxyxylitol (310 mg, 0.49 mmol) in dioxane (3.1 mL) was added 6 molar aqueous hydrochloric acid (0.38 mL, 2.3 mmol) and a 4 molar solution of hydrochloric acid in dioxane (1 mL, 4 mmol). After stirring for 4 hours the solvent was removed under vacuum to yield the hydro chloride salt, which was treated with ion exchange resin IR 67 (15 mL) in water for two hours. The ion exchange resin was washed with water followed by 15 % aqueous acetic acid. The washing solutions were fractionated and fractions with a negative chloride detection were combined and concentrated under vacuum to yield 120 mg 1-({1,3-bis(2-carboxyethoxy)-2-[(2-carboxyethoxy)methyl]propan-2-yl}amino)-1-deoxyxylitol acid as free base.
**¹H-NMR** (300 MHz, deuterium oxide) δ = 2.56 (br. s., 6 H), 3.14 - 3.32 (m, 2 H), 3.59 (br. s., 2 H), 3.63 - 3.80 (m, 17 H), 3.98 (br. s, 2 H).

### Example 6c

### Monoaqua-κO-bis(µ₃-3,3',3"-{[(1-deoxyxylt-1-yl)amino]methylidynetris(methyleneoxy)}-tripropanoato-1κ²O¹,O²:2κ²O^{1'}, O³:κ²O^{2'},O^{3'})-dihydroxido-κ²O-di-µ₃-oxido-1:2:3κ⁶O-tri-angulo-tritungsten(3 W-W)(IV)

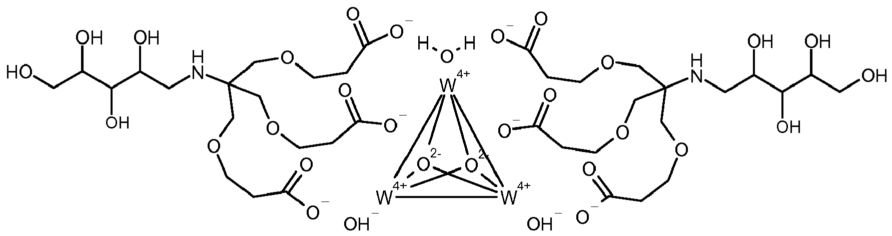

A suspension of 1-({1,3-bis(2-carboxyethoxy)-2-[(2-carboxyethoxy)methyl]propan-2-yl)amino)-1-deoxyxylitol (120 mg, 255 µmol) and monoaqua-κ*O*-hexakis(µ-acetato-κ²*O*) dihydroxido-κ²*O*-di-µ₃-oxido-1:2:3κ⁶*O*-*triangulo*-tritungsten(3 *W*-*W*)(IV) (125 mg, 127 µmol) in acetic acid (15 µL, 25 µmol) and water (9 mL) was irradiated in a microwave reactor for 15 minutes at 140°C. The reaction mixture was filtrated and the filtrate concentrated in vacuum. Separation on a preparative HPLC (acetonitrile water + acetic acid) yielded 19 mg of monoaqua-κ*O*-bis(µ₃-3,3',3"-{[(1-deoxyxylit-1-yl)amino]-methylidynetris(methyleneoxy)ltripropanoato-1κ²*O*¹,*O*²2κ²*O*^{1'},*O*³:3κ²*O*^{2'},*O*^{3'})-dihydroxido-κ²*O*-di-µ₃-oxido-1:2:3κ⁶*O*-*triangulo*-tritungsten(3 *W*-*W*)(IV) in form of its acetate salt.
**¹H-NMR** (300 MHz, deuterium oxide) δ = 2.71 (br. t, 12 H), 3.15 (br., 4 H), 3.56 (s, 12 H), 3.58 - 3.68 (m, 6 H), 3.74 (m, 2 H), 3.86 (br. t, 12 H), 3.88 - 3.95 (m, 2 H) ppm.
**LC/MS ESI-** m/z 1571 (M-1).

### Example 7

### (µ₃-3,3',3"-{Aminomethylidynetris(methyleneoxy)}tripropanoato-1κ²O¹,O²κ²O^{1'},O³: 3κ²O²,O^{3'})monoaqua-κO-dihydroxido-κ²O-di-µ₃-oxido-1:2:3κ⁶O-{µ3-3,3',3"-[propane-1,2,3-triyltris(oxy)]tripropanoato-1κ²O¹,O²:2κ²O^{1'},O³:3κ²O^{2'},O^{3'})triangulo-tritungsten (3 W-W)(IV)

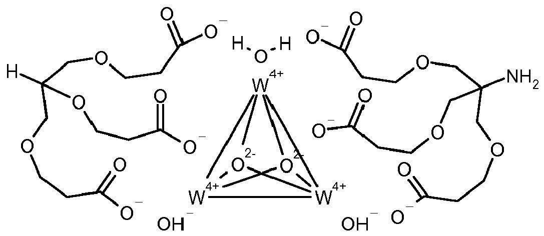

### Example 7a

### Tert-butyl 3-[2,3-bis(3-tert-butoxy-3-oxopropoxy)propoxy]propanoate

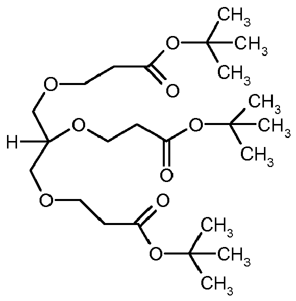

To propan-1,2,3-triol (105 g, 1.14 mol) and *tert*-butyl prop-2-enoate (835 mL, 5.7 mol) in DMSO (248 mL) was added a 5 molar aqueous sodium hydroxide solution (22.8 mL, 114 mmol) at 5°C. The mixture is quenched with aqueous NH₄Cl solution and extracted with ethyl acetate. The mixture was concentrated and the residue was purified by chromatography on silica gel (ethyl acetate in hexane, 20 to 100%) to yield 12.3 g of *tert-*butyl 3-[2,3-bis(3-*tert* butoxy-3-oxopropoxy)propoxy]propanoate.
**¹H-NMR** (400 MHz, CDCl₃): δ = 1.45 (s, 27 H), 2.48 (t, 6 H), 3.43 - 3.54 (m, 4 H), 3.58 (m, 1 H), 3.68 (t, 4 H), 3.81 (t, 2 H).

### Example 7b

### 3,3',3"-[Propane-1,2,3-triyltris(oxy)]tripropanoic acid

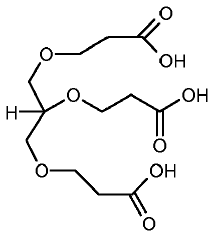

To a solution of *tert*-butyl 3-[2,3-bis(3-*tert* butoxy-3-oxopropoxy)propoxy]propanoate (12.3 g, 23.2 mmol) in dioxane (99 mL) was added 6 molar aqueous hydrochloric acid (18.6 mL, 111 mmol) and a 4 molar solution of hydrochloric acid in dioxane (46.5mL, 186mmol). After stirring for 24 hours additional 6 molar aqueous hydrochloric acid (1.86 mL, 11.1 mmol) and a 4 molar solution of hydrochloric acid in dioxane (4.65mL, 18.6mmol) was added and the solution was stirred for 2 hours at 40°C. The organic solvent was removed under vacuum, water was added to the residue and a final lyophilization yielded 8.0 g of 3,3',3"-[propane-1,2,3-triyltris(oxy)]tripropanoic acid.
**¹H-NMR** (400 MHz, deuterium oxide) δ = 2.59 (t, 2 H), 2.60 (t, 4 H), 3.50 (dd, 2 H), 3.58 (dd, 2 H), 3.67 - 3.70 (m, 1 H), 3.69 - 3.77 (m, 4 H), 3.81 (t, 2 H).

### Example 7c

### (µ₃-3,3',3"-{Aminomethylidynetris(methyleneoxy)}tripropanoato-1κ²O¹, O²:2κ²O^{1'},O³: 3κ²O^{2'},O^{3'})monoaqua-κO-dihydroxido-κ²O-di-µ₃-oxido-1:2:3κ⁶O-{µ₃-3,3',3"-[propane-1,2,3-triyltris(oxy)]tripropanoato-1κ²O¹,O²κ:²O^{1'},O³:3κO^{2'},O^{3'})triangulo-tritungsten (3 W-W)(IV)

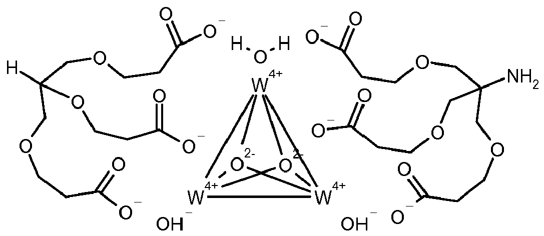

A suspension of 3,3',3"-[propane-1,2,3-triyltris(oxy)]tripropanoic acid (623 mg, 2.02 mmol), 3-{2-amino-3-(2-carboxyethoxy)-2-[(2-carboxyethoxy)methyl]propoxy}propanoic acid (682 mg, 2.02 mmol) and monoaqua-κ*O*-hexakis(µ-acetato-κ²*O*)- dihydroxido-κ²*O*-di-µ₃-oxido-1:2:3κ⁶*O*-*triangulo*-tritungsten(3 *W*-*W*)(IV) (2.0 g, 2.02 mmol) in acetic acid (47 µL, 0.81 mmol) and water (30 mL) was separated into 5 pressure vessels which were irradiated in a microwave reactor for 15 minutes at 140°C. The reaction mixtures were combined, filtrated and the filtrate was concentrated in vacuum. Separation on a preparative HPLC (acetonitrile water + acetic acid) yielded 93 mg of monoaqua-κ*O*-dihydroxido-κ²*O*-(µ₃-3,3',3"-{aminomethylidynetris(methyleneoxy)}tripropanoato-1κ²*O*¹, *O*²:κ²*O*^{1'},*O*³,3κ²*O*^{2'}, *O*^{3'})-di-µ₃-oxido-1:2:3κ⁶*O*-{µ₃-3,3',3"-[propane-1,2,3-triyltris(oxy)]tripropanoato-1κ²*O*¹, *O*²:2κ²*O*^{1'},*O*³,3κ²,*O*^{2'}*O*^{3'}}*triangulo*-tritungsten(3 *W-W*)(IV).
**¹H-NMR** (300 MHz, deuterium oxide) δ = 2.58 - 2.80 (m, 12 H), 3.24 (br., 2 H), 3.34 (m, 3 H), 3.47 (s, 6 H), 3.72 (m, 1 H), 3.78 - 3.94 (m, 6 H), 3.85 (t, 5 H) ppm.
**LC/MS ES-** m/z 1274 (M-1)

### Example 8

### {µ₃-3,3',3"-[Aminomethylidynetris(methyleneoxy)]tripropanoato-1κ²O¹,O²:2κ²O^{1'},O³: 3κ²O^{2'},O^{3'}}monoaqua-κO-dihydroxido-κ²O-{µ₃-3,3',3"-[methylidynetris(methylene-oxy)]tripropanoato-1κ²O¹,O²:2κ²O^{1'},O³:3κ²O^{3'},}di-µ₃-oxido-1:2:3κ⁶O-triangulo-tritungsten(3 W-W)(IV)

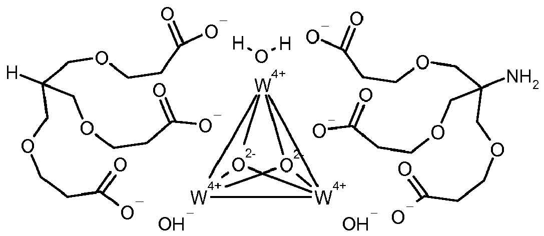

A suspension of 3-{3-(2-carboxyethoxy)-2-[(2-carboxyethoxy)methyl]propoxy}propanoic acid (260 mg, 808 µmol), 3-{2-amino-3-(2-carboxyethoxy)-2-[(2-carboxyethoxy)methyl]-propoxylpropanoic acid (273 mg, 808 µmol) and monoaqua-κ*O*-hexakis(µ-acetato-κ²*O*)-dihydroxido-κ²*O*-di-µ₃-oxido-1:2:3κ⁶*O*-*triangulo*-tritungsten(3 *W-W*)(IV) (800 mg, 808 µmol) in acetic acid (93 µL, 1.62 mmol) and water (60 mL) was separated into two portions, which were irradiated in a microwave reactor for 15 minutes at 140°C. The reaction mixtures were filtrated and the filtrate was concentrated in vacuum. Separation on a preparative HPLC (acetonitrile water + acetic acid) yielded 87 mg of {µ₃-3,3',3"-[Aminomethylidynetris(methyleneoxy)]tripropanoato-1κ²*O*¹,*O*²:2κ²*O*^{1'},*O*³:3κ²*O*³}mono-aqua-κ*O*-dihydroxido-κ²*O*-{µ₃-3,3',3"-[methylidynetris(methylene-oxy)]tripropanoato-1κ²*O*¹,*O*²:2κ*O*^{1'},*O*³:3κ,*O*^{2'},*O*^{3'}}di-µ₃-oxido-1:2:3κ⁶*O*-*triangulo*-tritungsten(3 *W*-*W*)(IV).
**¹H-NMR** (300 MHz, deuterium oxide) δ = 1.71 (m, 1 H), 2.71 (br. t, 6 H), 2.73 (br. t, 6 H), 3.38 (d, 6 H), 3.49 (s, 6 H), 3.82 (t, 6 H), 3.87 (t, 6 H) ppm.
**LC/MS ES-** m/z 1287.93 (M-1)

### Example 9

### Monoaqua-κO-dihydroxido-κ²O-(µ₃-3,3',3"-{[(2,3-dihydroxypropyl)amino]-methylidynetris(methyleneoxy)}tripropanoato-1κ²O¹,O²:2κ²O^{1'},O³:3κ²O^{2'},O^{3'})di-µ₃-oxido-1:2:3κ⁶O-{µ₃-3,3',3"-[propane-1,2,3-triyltris(oxy)]tripropanoato-1κ²O¹,O²:2κ²O^{1'},O³: 3κ²O^{2'},O^{3'}}triangulo-tritungsten(3 W-W)(IV)

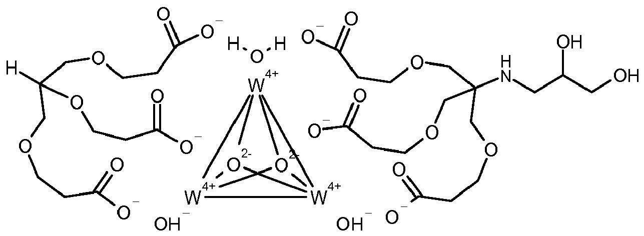

A suspension of 3,3',3"-[propane-1,2,3-triyltris(oxy)]tripropanoic acid (98 mg, 316 µmol), 3-{3-(2-carboxyethoxy)-2-[(2-carboxyethoxy)methyl]-2-[(2,3-dihydroxypropyl)amino]-propoxylpropanoic acid (174 mg, 422 µmol) and sodium hexakis(µ-acetato-k²*O*)-tris(acetatO-κ*O*)-di-µ₃-oxido-1:2:3κ⁶*O*-*triangulo*-tritungsten(3 *W*-*W*)(IV) (400 mg, 0.37 mmol) in water (60 mL) was irradiated in a microwave reactor for 15 minutes at 140°C. The reaction mixture was filtrated and the filtrate was concentrated in vacuum. Separation on a preparative HPLC (acetonitrile water + acetic acid) yielded mg *rac*-monoaqua-κ*O-*dihydroxido-κ²*O*-(µ3-3,3',3"-{[(2,3-dihydroxypropyl)amino]methylidynetris(methyleneoxy)} tripropanoato-1κ²*O*¹,*O*²:2κ²*O*^{1'}, *O*³,3κ²*O*^{2'}, *O*^{3'})di-µ₃-oxido-{µ₃-3,3',3"-[propane-1,2,3-triyltris-(oxy)]tripropanoato-1κ²*O*¹, *O*²:2κ²*O*^{1'},*O*³:3κ²*O*^{2'}*O*^{3'}}-1:2:3κ⁶*O*-*triangulo*-tritungsten(3 *W-W*)(IV).
**¹H-NMR** (400 MHz, deuterium oxide) δ = 2.64 - 2.78 (m, 12 H), 3.00 (dd, 1 H), 3.15 (dd, 1 H), 3.19 - 3.28 (m, 2 H), 3.31 - 3.41 (m, 3 H), 3.46 - 3.62 (m, 2 H), 3.56 (s, 6H), 3.73 (m, 1 H), 3.79 - 3.93 (m, 6 H), 3.86 (t, 6 H) ppm.
**LC/MS ES-** m/z 1347.66 (M-1).

### Example 10

### Monoaqua-κO-bis{µ₃-3,3',3"-[(1α,3α,5α)-cyclohexane-1,3,5-triyltris(oxy)]tri-propanoato-1κ²O¹,O²:2κ²O^{1'},O³:3κ²O^{2'},O^{3'}}dihydroxido-κ²O-di-µ₃-oxido-1:2:3κ⁶O-triangulo-tritungsten(3 W-W(IV)

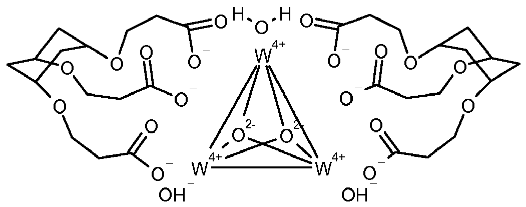

### Example 10a

### Tri-tert-butyl 3,3',3"-[(all-cis)-cyclohexane-1,3,5-triyltris(oxy)]tripropanoate

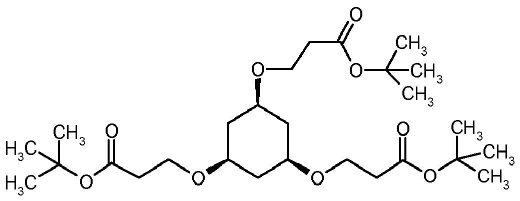

To a suspension of cis,cis-1,3,5-cyclohexanetriol-dihydrate (2.5g, 14.9 mmol) and *tert-*butyl prop-2-enoate (10.9 mL, 74.3 mmol) in DMSO (4.2 mL) at 15°C was added a 5 molar aqueous sodium hydroxide solution (0.3 mL). Stirring was continued at room temperature for 36 hours. The mixture was directly purified by chromatography on silica gel (ethyl acetate in hexane, 20 to 80%) to yield 2.46 g of tri-tert-butyl 3,3',3"-[(all-cis)-cyclohexane-1,3,5-triyltris(oxy)]tripropanoate.
**¹H-NMR** (400 MHz, CDCl₃): δ = 1.16 (q, 3 H), 1.46 (s, 27 H), 2.27 - 2.42 (m, 3 H), 2.47 (t, 6 H), 3.23 (tt, 3 H), 3.61 - 3.77 (m, 6 H) ppm.

### Example 10b

### 3,3',3"-[(all-cis)-cyclohexane-1,3,5-triyltris(oxy)]tripropanoic acid

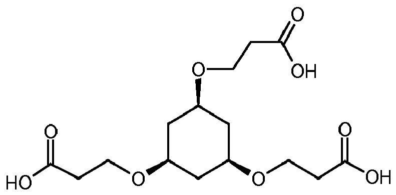

To a solution of tri-*tert*-butyl 3,3',3"-[cyclohexane-1,3,5-triyltris(oxy)]tripropanoate (2.46 g, 4.76 mmol) in dioxane (24.6 mL) was added 6 molar aqueous hydrochloric acid (3.8 mL, 22.8 mmol) and a 4 molar solution of hydrochloric acid in dioxane (9.5 mL, 38 mmol). After stirring for 17 hours the solvent was removed under vacuum to yield 2.0 g of 3,3',3"-[(all-cis)-cyclohexane-1,3,5-triyltris(oxy)]tripropanoic acid as a light yellow oil.
**¹H-NMR** (400 MHz, deuterium oxide) δ = 1.06 (ddd, 3 H), 2.39 (m, 3 H), 2.59 (t, 6 H), 3.33 - 3.67 (m, 3 H), 3.78 (t, 6 H).

### Example 10c

### Monoaqua-κO-bis{µ₃-3,3',3"-[(1α,3α,5α)-cyclohexane-1,3,5-triyltris(oxy)]tri-propanoato-1κ²O¹,O²:2κ²O^{1'},O^{2'}:3κ²O^{2'},O^{3'}}dihydroxido-κ²O-di-µ₃-oxido-1:2:3κ⁶O-triangulo-tritungsten(3 W-W)(IV)

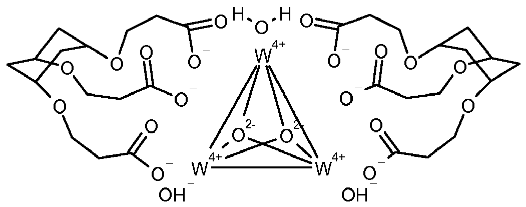

A suspension of 3,3',3"-[(all-cis)-cyclohexane-1,3,5-triyltris(oxy)]tripropanoic acid (1.58 g, 3.84 mmol) and monoaqua-κ*O*-hexakis(µ-acetato-κ²*O*)-dihydroxido-κ²*O*-di-κ₃-oxido-1:2:3κ⁶*O*-*triangulo*-tritungsten(3 W-W)(IV) (2.0 g, 2.02 mmol) in acetic acid (232 µL, 4.05 mmol) and water (150 mL) was separated into 5 pressure vessels which were irradiated in a microwave reactor for 15 minutes at 140°C. The reaction mixtures were filtrated and the combined filtrates were concentrated in vacuum. Separation on a preparative HPLC (acetonitrile water + acetic acid) yielded 1 mg monoaqua-κ*O*-dihydroxido-di-κ₃-oxido-bis{µ₃-3,3',3"-[(*1*α,*3*α,*5*α)-cyclohexane-1,3,5-triyltris(oxy)]tripropanoato-1κ²*O*¹,*O*²:2κ²*O*^{1'},*O*³:3κ²*O*^{2'},*O*^{3'}}-1:2:3κ⁶*O*-*triangulo*-tritungsten(3 *W*-*W*)(IV).
**¹H-NMR** (400 MHz, deuterium oxide) δ = 1.06 - 1.21 (m, 6 H), 2.11 - 2.26 (m, 6 H), 2.51 - 2.63 (m, 6 H), 2.68 - 2.84 (br., 12 H), 3.97 (br. , 12 H).
**LC/MS ES-** m/z 1325.18 (M-1).

### Example 11

### Monoaqua-κO-bis{µ₃-3,3',3"-[2-hydroxyethyl-1,1,1-idynetris(methyleneoxy)-tripropanoato-1κ²O¹,O²:2κ²O^{1'},O³:3κ²O^{3'}}dihydroxido-κ²O-di-µ₃-oxido-1:2:3κ⁶O-triangulo-tritungsten(3 W-W)(IV)

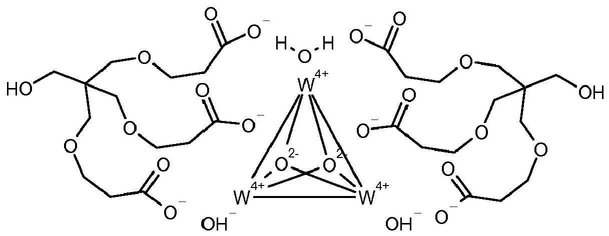

### Example 11a

### Tert-butyl 3-[3-(3-tert-butoxy-3-oxopropoxy)-2-[(3-tert-butoxy-3-oxopropoxy)methyl]-2-(hydroxymethyl)propoxy]propanoate

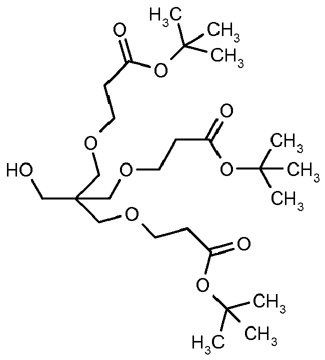

To pentaerythritol (5.5 g, 40.4 mmol) in *tert*-butanol (30 mL) was added potassium *tert-*butoxide (1.36 g, 12.1 mmol) and *tert*-butyl acrylate (19.5 mL, 133.3 mmol). The suspension was stirred for 30 min at 100°C and overnight at room temperature. After removal of the solvent *in vacuo,* the residue was taken up in ethyl acetate and water, the organic phase was washed two times with water and dried over sodium sulfate. The mixture was concentrated *in vacuo* and the residue was purified by chromatography on silica gel (dichloromethane/methanol gradient, 0 to 100%) to yield 3.67 g (16.75 %) of *tert-*butyl 3-[3-(3-*tert*-butoxy-3-oxopropoxy)-2-[(3-*tert-*butoxy-3-oxopropoxy)methyl]-2-(hydroxymethyl)propoxy]propanoate.
**¹H-NMR** (500 MHz, DMSO-*d6*): δ = 1.40 (s, 27 H), 2.38 (t, 6 H), 3.26 - 3.31 (m, 8 H), 3.51 (t, 6 H), 4.11 (m, 1 H).
ESI+ m/z 521 (M+1).

### Example 11 b

### 3-[3-(2-Carboxyethoxy)-2-[(2-carboxyethoxy)methyl]-2-(hydroxymethyl)propoxy]-propanoic acid

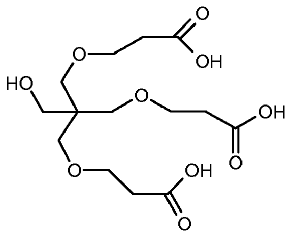

To a solution of *tert*-butyl 3-[3-(3-*tert-*butoxy-3-oxopropoxy)-2-[(3-tert butoxy-3-oxoprop-oxy)methyl]-2-(hydroxymethyl)propoxy]propanoate (34.7 g, 66.7 mmoL) in dioxane (400 mL) was added 2 molar aqueous hydrochloric acid (200 mL, 400 mmol). The mixture was refluxed for 6h and stirred at room temperature overnight. After removal of the solvent *in vacuo*, the residue was chromatographed with acetonitrile / water on C18-silica gel, the resulting fractions were combined and finally lyophilized to yield 9.07 g (38.6 %) of 3-[3-(2-carboxyethoxy)-2-[(2-carboxyethoxy)methyl]-2-(hydroxymethyl)propoxy]propanoic acid.
**¹H-NMR** (400 MHz, CDCl₃) δ = 2.60 (t, 6H), 3.46 (s, 6H), 3.65 (s, 2H), 3.70 (t, 6H).
ESI+ m/z 353 (M+1).

### Example 11c

### Monoaqua-κO-bis{µ₃-3,3',3"-[2-hydroxyethyl-1,1,1-idynetris(methyleneoxy)-tripropanoato-1κ²O¹,O²:2κ²O^{1'},O³:3κ²O^{2'},O^{3'}}dihydroxido-κ²O-di-µ₃-oxido-1:2:3κ⁶O-triangulo-tritungsten(3 W-W)(IV)

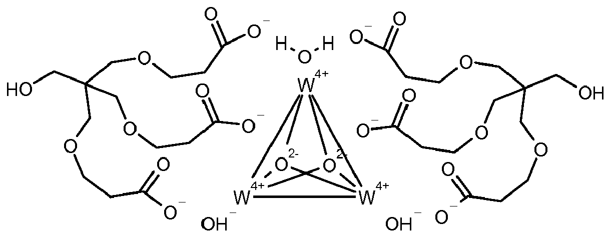

3.52 g (10 mmol) of 3-[3-(2-carboxyethoxy)-2-[(2-carboxyethoxy)methyl]-2-(hydroxymethyl)propoxy]propanoic acid and 2.28 g, (2 mmol) of sodium hexakis(µ-acetato-κ²*O*)-tris(acetato-κ*O*)-di-µ₃-oxido-1:2:3κ⁶*O*-*triangulo*-tritungsten(3 *W*-*W*)(IV) in 40 mL water were irradiated in a micowave reactor for 8h at 125°C. The reaction mixture was filtrated and the solution was concentrated *in vacuo* and the resulting crude product was chromatographed with acetonitrile / water on C18-silica gel and the resulting fractions were combined and finally lyophilized. Monoaqua-κ*O*-bis{µ₃-3,3',3"-[2-hydroxyethyl-1,1,1-idynetris(methyleneoxy)-tripropanoato-1κ²*O*¹,*O*²:2κ²*O*^{1'},*O*³:3κ²*O*^{2'},*O*^{3'})dihydroxido-κ²*O*-di-µ₃-oxido-1:2:3µ⁶*O*-*triangulo*-tritungsten(3 *W*-*W*)(IV) was obtained (80 mg, 3 %) as a curry-colored amorphous powder.
**¹H-NMR** (400 MHz, deuterium oxide) δ = 2.75 (t, 12H), 3.39 (s, 12H), 3.46 (s, 4H), 3.85 (t, 12H).
**LC/MS ES-** m/z 1333 (M-1).

### Example 12

### Monoaqua-κO-bis(µ₃-3,3',3"-[2-methoxyethyl-1,1,1-idynetris(methyleneoxy)-tripropanoato-1κ²O¹,O²:2κ²O^{1'},O³,3κ²O^{2'},O^{3'}]-dihydroxido-κ²O-di-µ₃-oxido-1:2:3κ⁶O-triangulo-tritungsten(3 W-W)(IV)

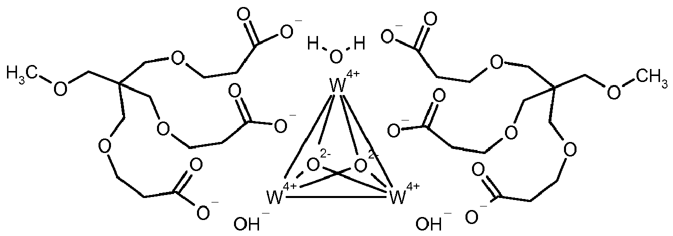

### Example 12a

### Tert-butyl 3-[3-(3-tert-butoxy-3-oxopropoxy)-2-[(3-tert-butoxy-3-oxopropoxy)methyl]-2-(methoxymethyl)propoxy]propanoate

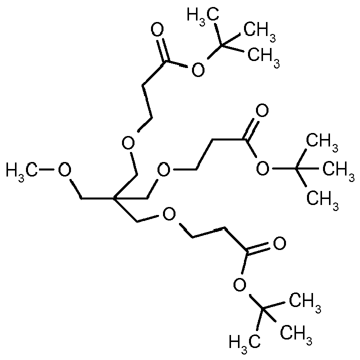

To 2-(hydroxymethyl)-2-(methoxymethyl)propane-1,3-diol (Journal of the American Chemical Society (1955), 77, 6382-3), (2.8 g, 20.2 mmol) in *tert-*butanol (15 mL) were added potassium *tert*-butoxide (0.68 g, 6 mmol) and *tert*-butyl acrylate (9.8 mL, 66.6 mmol). The suspension was stirred for 30 min at 100°C and overnight at room temperature. After removal of the solvent *in vacuo,* the residue was taken up in ethyl acetate and water, the organic phase was washed two times with water, dried over sodium sulfate. The mixture was concentrated *in vacuo* and the residue was purified by chromatography on silica gel (dichloromethane/methanol gradient, 0 to 100%) to yield 2.0 g (18.4 %) of *tert-*butyl 3-[3-(3-*tert-*butoxy-3-oxopropoxy)-2-[(3-tert butoxy-3-oxopropoxy)-methyl]-2-(methoxymethyl)propoxy]propanoate.
**¹H-NMR** (400 MHz, CDCl₃): δ = 1.45 (s, 27H), 2.44 (t, 6H), 3.27 (s, 3H), 3.31 (s, 2H), 3.37 (s, 6H), 3.61 (t, 6H).
**ESI+** m/z 535 (M+1).

### Example 12b

### 3-[3-(2-Carboxyethoxy)-2-[(2-carboxyethoxy)methyl]-2-(methoxymethyl)propoxy]-propanoic acid

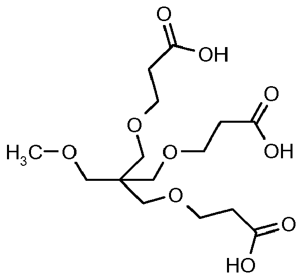

To a solution of *tert*-butyl 3-[3-(3-*tert-*butoxy-3-oxopropoxy)-2-[(3-*tert*-butoxy-3-oxoprop-oxy)methyl]-2-(methoxymethyl)propoxy]propanoate (1.34 g, 2.5 mmoL) in dioxane (20 mL) was added 2 molar aqueous hydrochloric acid (10 mL, 20 mmol). The mixture was refluxed for 6h and stirred at room temperature overnight. After removal of the solvent *in vacuo*, the residue was chromatographed with acetonitrile / water on C18-silica gel, the resulting fractions were combined and finally lyophilized to yield 0.9 g (98.2 %) of 3-[3-(2-carboxyethoxy)-2-[(2-carboxyethoxy)methyl]-2-(methoxymethyl)propoxy]propanoic acid.
**¹H-NMR** (400 MHz, deuterium oxide) δ = 2.50 (t, 6H), 3.64 (s, 6H), 4.65 (m, 11 H).
**ESI+** m/z 367 (M+1).

### Example 12c

### Monoaqua-κO-bis(µ₃-3,3',3"-[2-methoxyethyl-1,1,1-idynetris(methyleneoxy)-tripropanoato-1κ²O¹,O²:2κ²O^{1'},O³:3κ²O^{2'},O^{3'}]-dihydroxido-κ²O-di-µ₃-oxido-1:2:3κ⁶O-triangulo-tritungsten(3 W-W)(IV)

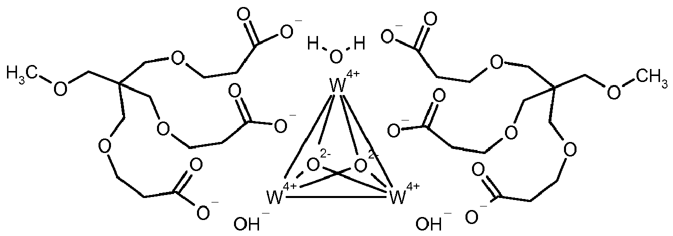

0.92 g (2.5 mmol) of 3-[3-(2-carboxyethoxy)-2-[(2-carboxyethoxy)methyl]-2-(methoxymethyl)propoxy]propanoic acid and 0.57 g, (0.5 mmol) of sodium hexakis(µ-acetato-κ²*O*)-tris(acetato-κ*O*)-di-µ₃-oxido-1:2:3κ⁶*O*-*triangulo*-tritungsten(3 *W*-*W*)(IV) in 40 mL water were irradiated in a micowave reactor for 8h at 125°C. The reaction mixture was filtrated and the solution was concentrated *in vacuo* and the resulting crude product was chromatographed with acetonitrile / water on C18-silica gel and the resulting fractions were combined and finally lyophilized. Monoaqua-κ*O*-bis(µ₃-3,3',3"-[2-methoxyethyl-1,1,1-idyne tris(methyleneoxy)tripropanoato-1 K²*O*¹,*O*²:2κ²*O*^{1'},*O*³:3κ²*O*^{2'},*O*^{3'}]dihydroxido-κ²*O*-di-µ₃-oxido-1:2:3κ⁶*O*-*triangulo*-tritungsten(3 *W*-*W*)(IV) was obtained (78 mg, 11.5 %) as a curry-colored amorphous powder.
**¹H-NMR** (600MHz, deuterium oxide) δ = 2.73-2.77 (m, 12H), 3.28 (s, 6H), 3.30 (s, 4H), 3.38 (s, 12H), 3.81-3.87 (m, 12H).
**LC/MS ES-** m/z 1361 (M-1).

### Example 13

### Monoaqua-κO-dihydroxido-κ²O-bis(µ₃-3,3',3"-[hydroxymethylidyne tris(methylene-oxy)tripropanoato-1κ²O¹,O²:2κ²O^{1'},O³,3κ²O^{2'},O^{3'}]-di-µ₃-oxido-1:2:3κ⁶O-triangulo-tritungsten(3 W-W(IV)

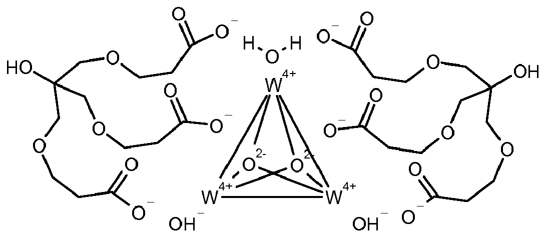

### Examle 13a

### Tert-butyl 3-({2-[(3-tert-butoxy-3-oxopropoxy)methyl]prop-2-en-1-yl}oxy)propanoate

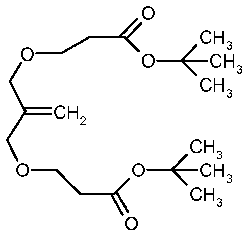

2-Methylidenepropane-1,3-diol (5.0 g, 56.7 mmol) and *tert*-butyl prop-2-enoate (36.4 g, 284 mmol) are stirred in DMSO (11.4 mL) and 5 molar aqueous sodium hydroxide solution (1.14 mL) at 20°C for 72 hours. The mixture was concentrated and the residue was purified by chromatography on silica gel (ethyl acetate in hexane, 0 to 40%) to yield 9.03 g of tert-butyl 3-({2-[(3-tert-butoxy-3-oxopropoxy)methyl]prop-2-en-1-yl}oxy)propanoate.
**¹H-NMR** (300 MHz, CDCl₃): δ = 1.46 (s, 18 H), 2.50 (t, 4 H), 3.66 (t, 4 H), 3.98 (s, 4 H), 5.18 (s, 2 H).

### Examle 13b

### Di-tert-butyl 3,3'-{[2-hydroxy-2-(hydroxymethyl)propane-1,3-diyl]bis(oxy)}dipropanoate

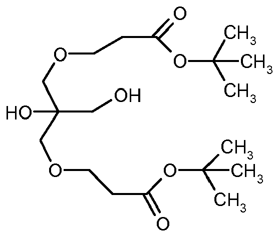

To a solution of tert-butyl 3-({2-[(3-tert-butoxy-3-oxopropoxy)methyl]prop-2-en-1-yi}oxy)-propanoate (4.74 g, 13.8 mmol) in acetone (70 mL) and water (7 mL) was added 4-methylmorpholine 4-oxide (3.22 g, 27.5 mmol) and 2.5% solution of tetraoxoosmium in tert.-butanol (5.2 mL) at 0°C. The mixture was warmed to room temperature, stirred for 48 hours and quenched with an aqueous sodium disulfite solution. After vigorous stirring the mixture was extracted with ethyl acetate and the combined organic phases were washed with 1 M hydrochloric acid, aqueous sodium hydrogen carbonate solution and brine. The solution was dried over sodium sulfate and concentrated to yield 4.3g of di-tert-butyl 3,3'-{[2-hydroxy-2-(hydroxymethyl)propane-1,3-diyl]bis(oxy)}dipropanoate.
**¹H-NMR** (400 MHz, CDCl₃): δ = 1.46 (m, 18 H), 2.49 (t, 4 H), 2.59 (t, 1 H), 3.20 (s, 1 H), 3.50 (s, 4 H), 3.59 (d, 2 H), 3.71 (t, 4 H).

### Example 13c

### 3-{3-(2-Carboxyethoxy)-2-[(2-carboxyethoxy)methyl]-2-hydroxypropoxy}propanoic acid

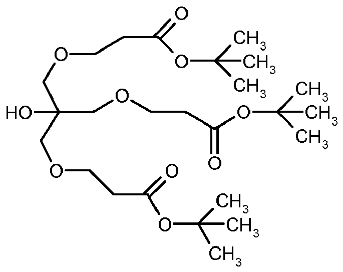

To di-*tert*-butyl 3,3'-{[2-hydroxy-2-(hydroxymethyl)propane-1,3-diyl]bis(oxy)}dipropanoate (1 g, 2.64 mmol) and *tert*-butyl prop-2-enoate (1.42 mL, 9.72 mmol) in tert-butanol (1.9 mL) were added four times potassium *tert.*-butanolate (4 x 22 mg, all 15 minutes at room temperature. 30 minutes after the last addition the mixture was quenched with saturated aqueous NH₄Cl solution and extracted with ethyl acetate. The combined organic phases were dried over sodium sulfate and concentrated. The residue was purified by chromatography on silica gel (ethyl acetate in hexane, 0 to 40%) to yield 0.77 g of 3-{3-(2-Carboxyethoxy)-2-[(2-carboxyethoxy)methyl]-2-hydroxypropoxylpropanoic acid as a colorless oil.
**¹H-NMR** (300 MHz, CDCl₃): δ = 1.45 (s, 27 H), 2.49 (t, 6 H), 3.05 (s, 1 H), 3.44 (s, 6 H), 3.70 (m, 6 H).

### Example 13d

### 3-{3-(2-Carboxyethoxy)-2-[(2-carboxyethoxy)methyl]-2-hydroxypropoxy}propanoic acid

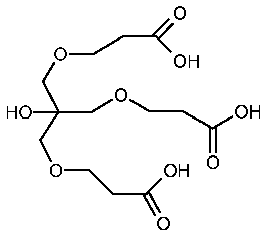

To a solution of *tert*-butyl 3-{3-(3-*tert*-butoxy-3-oxopropoxy)-2-[(3-*tert*-butoxy-3-oxopropoxy)-methyl]-2-hydroxypropoxy}propanoate (1.64 g, 3.24 mmol) in dioxane (16.4mL) was added 6 molar aqueous hydrochloric acid (2.6 mL, 15.5 mmol) and a 4 molar solution of hydrochloric acid in dioxane (6.5 mL, 25.9 mmol). After stirring for 96 hours the solvent was removed under vacuum to yield 1.22 g of 3-{3-(2-carboxyethoxy)-2-[(2-carboxyethoxy)methyl]-2-hydroxy-propoxylpropanoic acid.
**¹H-NMR** (400 MHz, deuterium oxide) δ = 2.59 (t, 6 H), 3.42 (s, 6 H), 3.71 (t, 6 H).
**LC/MS ES+** m/z 339.17 (M+1).

### Example 13e

### Monoaqua-κO-dihydroxido-κ²O-bis(µ₃-3,3',3"-[hydroxymethylidyne tris(methylene-oxy)tripropanoato-1κ²O¹, O²:2κ²O^{1'}, O³3κ²O^{2'}, O^{3'}]-di-µ₃-oxido-1:2:3κ⁶O-triangulo-tritungsten(3 W-W(IV)

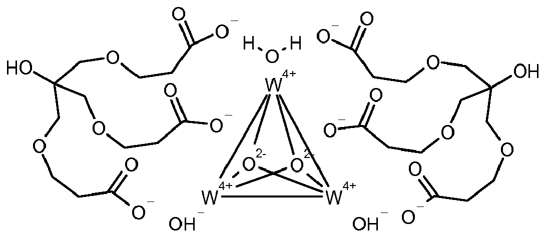

5.3 mg of the title compound monoaqua-κ*O*-dihydroxido-κ²*O*-bis(µ₃-3,3',3"-[hydroxymethylidyne tris(methyleneoxy)tripropanoato-1κ²*O*¹,*O*²:2κ²*O*¹,*O*³:3κ²*O*²,*O*³]-di-µ₃-oxido-1:2:3κ⁶*O*-*triangulo*-tritungsten(3 *W*-*W*)(IV) was isolated as a byproduct of example 16.
**¹H-NMR** (400 MHz, deuterium oxide) δ = 2.70 (br. t, 12 H), 3.31 (s, 12 H), 3.81 (br. t, 12 H).
**MS ESI-** m/z 1305 (M-1).

### Example 14

### Monoaqua-κO-dihydroxido-κ²O-(µ₃-3,3',3"-{[(2,3-dihydroxypropyl)amino]-methylidynetris(methyleneoxy)}tripropanoato-1κ²O¹,O²:2κ²O^{1'},O³:3κ²O^{2'},O^{3'})(µ₃-3,3',3"-[hydroxymethylidynetris(methyleneoxy)tripropanoato-1κ²O¹,O²:2κ²O^{1'},O³:3κ²O^{2'},O^{3'}]-di-µ₃-oxido-1:2:3κ⁶O-triangulo-tritungsten(3 W-W)(IV)

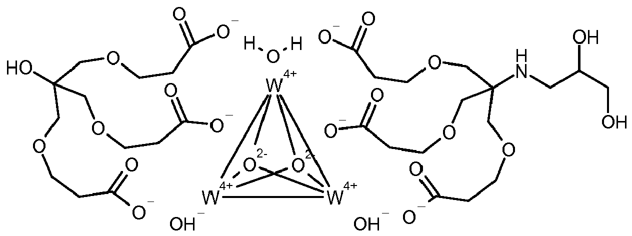

A suspension of 3-{3-(2-carboxyethoxy)-2-[(2-carboxyethoxy)methyl]-2-hydroxypropoxy}-propanoic acid (121 mg, 0.37 mmol), 3-{3-(2-carboxyethoxy)-2-[(2-carboxyethoxy)methyl]-2-[(2,3-dihydroxypropyl)amino]propoxy}propanoic acid (147 mg, 0.37 mmol) and sodium hexakis(µ-acetato-κ²*O*)-tris(acetato-κ²*O)*-di-µ3-oxido-1:2:3κ⁶*O*-*triangulo*-tritungsten(3 *W-W)*(IV) (400 mg, 0.37 mmol) in water (30 mL) was irradiated in a microwave reactor for 15 minutes at 140°C. The reaction mixture was filtrated and the filtrate was concentrated in vacuum. Separation on a preparative HPLC (acetonitrile water + acetic acid) yielded 57 mg monoaqua-κ*O*-dihydroxido-κ²*O*-(µ3-3,3',3"-{[(2,3-dihydroxypropyl)amino]methylidyne-tris(methyleneoxy)ltripropanoato-1κ²*O*¹,*O*²:2κ²*O*^{1'},*O*³,3κ²*O*^{2'},*O*^{3'})(µ₃-3,3',3"-[hydroxyl-methylidynetris(methyleneoxy)tripropanoato-1κ²*O*¹,*O*²:2κ²*O*¹,*O*³,3κ²*O*^{2'},*O*^{3'}]-di-µ₃-oxido-1:2:3κ⁶*O*-*triangulo*-tritungsten(3 *W*-*W*)(IV).
**¹H-NMR** (300 MHz, deuterium oxide) δ = 2.72 (br. t, 6 H), 2.74 (br. t, 6 H), 3.02 (dd, 1 H), 3.17 (dd, 1 H), 3.33 (s, 6 H), 3.47 - 3.61 (m, 2 H), 3.57 (s, 6 H), 3.76 - 3.94 (m, 13 H).
**LC/MS ES-** m/z 1378 (m-1)

### Example 15

### Monoaqua-κO-dihydroxido-κ²O-bis(µ₃-3,3',3"-{[(2-hydroxyethyl)amino]methylidyne-tris(methyleneoxy)}tripropanoato-1κ²O¹,O²:2κ²O^{1'},O³:3κ²O^{2'},O^{3'})-di-µ₃-oxido-1:2:3κ⁶O-triangulo-tritungsten(3 W-W)(IV)

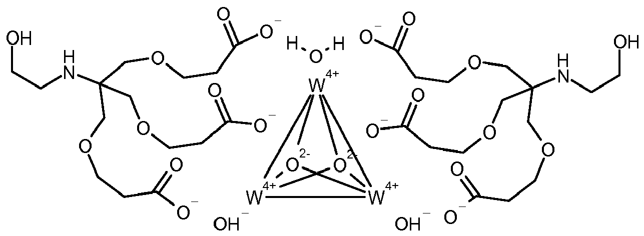

### Example 15a

### Tert-butyl 3-{3-(3-tert-butoxy-3-oxopropoxy)-2-[(3-tert-butoxy-3-oxopropoxy)methyl]-2-[(2-hydroxyethyl)amino]propoxy}propanoate

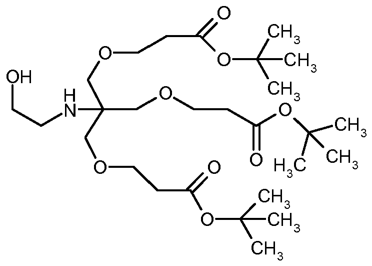

*Tert*-butyl 3-{2-amino-3-(3-*tert*-butoxy-3-oxopropoxy)-2-[(3-*tert*-butoxy-3-oxopropoxy)-methyl]propoxylpropanoate (530 mg, 1.05 mmol), palladium on charcoal 10% (6 mg) and 1,4-dioxane-2,5-diol (94.4 mg, 1.57 mmol) was stirred under an hydrogen atmosphere of 50 bar in methanol for for 20 hours. Additional 1,4-dioxane-2,5-diol (94.4 mg, 1.57 mmol) was added two times and stirring under 50 bar hydrogen pressure was continued for 7 hours after each addition. The reaction mixture was filtered through cellites, concentrated and purified via chromatography on silica gel (methanol in dichloromethane 0 to 20%) to yield 460 mg of *tert*-butyl 3-{3-(3-*tert*-butoxy-3-oxopropoxy)-2-[(3-*tert*-butoxy-3-oxopropoxy)methyl]-2-[(2-hydroxyethyl)amino]propoxy}propanoate.
**¹H-NMR** (300 MHz, CDCl₃): δ = 1.46 (s, 27 H), 2.47 (t, 6 H), 2.81 (t, 2 H), 3.39 (s, 6 H), 3.53 (t, 2 H), 3.64 (t, 6 H).

### Example 15b

### 3-{3-(2-Carboxyethoxy)-2-[(2-carboxyethoxy)methyl]-2-[(2-hydroxyethyl)amino]-propoxy}propanoic acid

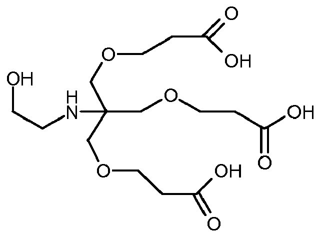

To 3-{3-(3-*tert*-butoxy-3-oxopropoxy)-2-[(3-*tert*-butoxy-3-oxopropoxy)methyl]-2-[(2-hydroxyethyl)amino]propoxylpropanoate (4.13 g, 7.51 mmol) in dioxane was added 6 M aqueous hydrochoric acid (6 mL, 36 mmol) and a 4 M solution of hydrochloric acid in dioxane (15 mL, 60 mmol). After stirring for 15 hours the solvent was removed under vacuum and the residue was solved in water (300 mL) and a volume of 200 mL of weakly basic anionic exchanger Amberlite IRA 67 in its OH⁻-form was added. The suspension was stirred for 2 hours and the exchange resin was filtered and washed with water (1000 mL). The free base of the product was eluted from the resin by fractionated washing with 15% aqueous acetic acid to yield 2.29 g of 3-{3-(2-carboxyethoxy)-2-[(2-carboxyethoxy)methyl]-2-[(2-hydroxyethyl)amino]propoxy}propanoic acid after lyophilization.
**¹H-NMR** (400 MHz, deuterium oxide) δ = 2.54 (t, 6 H), 3.20 (t, 2 H), 3.65 (s, 6 H), 3.71 (t, 6 H), 3.76 (t, 2 H).

### Example 15c

### Monoaqua-κO-dihydroxido-κ²O-bis(µ₃-3,3',3"-{[(2-hydroxyethyl)amino]methylidyne-tris(methyleneoxy)}tripropanoato-1κ²O¹,O²:2κ²O^{1'},O³:3κ²O¹,O^{2'},O^{3'})-di-µ₃-oxido-1:2:3κ⁶O-triangulo-tritungsten(3 W-W)(IV)

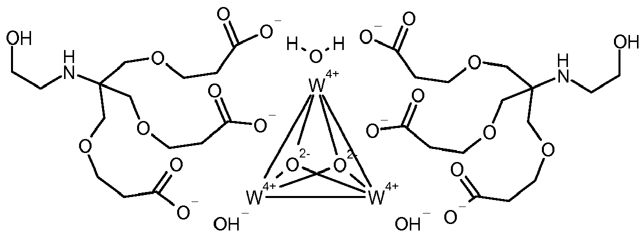

A suspension of 3-{3-(2-carboxyethoxy)-2-[(2-carboxyethoxy)methyl]-2-[(2-hydroxyethyl)-amino]propoxylpropanoic acid (80 mg, 210 µmol) and hexakis(µ-acetato-κ²*O*)-monoaqua-κ*O*-dihydroxido-κ²*O-*di-µ₃-oxido-1:2:3µ⁶*O*-*triangulo*-tritungsten(3 *W*-*W*)(IV) (103 mg, 105 µmol) in acetic acid (12 µL, 0.81 mmol) and water (8 mL) was irradiated in a microwave reactor for 15 minutes at 140°C. The reaction mixtures were filtrated and the filtrate was concentrated in vacuum. Separation on a preparative HPLC (acetonitrile water + acetic acid ) yielded 9.4 mg of monoaqua-κ*O*-bis(µ₃-3,3',3"-{[(2-hydroxyethyl)amino]-methylidynetris(methyleneoxy)ltripropanoato-1κ²*O*¹, *O*²:2κ²*O*^{1'}*O*³:3κ²*O*^{2'},*O*^{3'})dihydroxido-κ²*O*-di-µ₃-oxido-1:2:3κ⁶*O*-triangulo-tritungsten(3 *W*-*W*)(IV) after ultrafiltration through a YC05 NMWL 500 membrane and final lyophilization.
**¹H-NMR** (300 MHz, deuterium oxide) δ = 2.70 (t, 12 H), 3.03 (br. , 4 H), 3.51 (s, 12 H), 3.68 (br. t, 4 H), 3.84 (br. t, 12 H).
**LC/MS ESI-** m/z 1391 (M-1).

### Example 16

### Monoaqua-κO-dihydroxido-κ²O-(µ₃-3,3',3"-{[(2-hydroxyethy)amino]methylidynetris-(methyleneoxy)}tripropanoato-1κ²O¹, O²:2κ²O^{1'}, O³:3κ²O^{2'},O³,)(µ₃-3,3',3"-[hydroxy-methylidynetris(methyleneoxy)tripropanoato-1κ²O¹,O²:2κ²O^{1'},O³:3κ²O^{2'},O^{3'}]-di-µ₃-oxido-1:2:3κ₆O-triangulo-tritungsten(3 W-W)(IV)

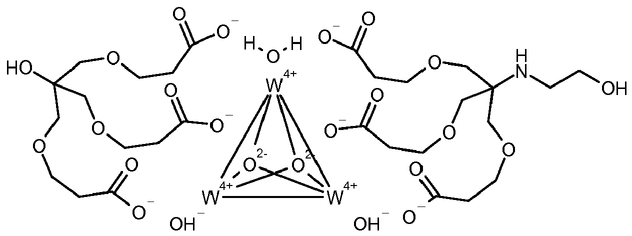

A suspension of 3-{3-(2-carboxyethoxy)-2-[(2-carboxyethoxy)methyl]-2-hydroxypropoxy}-propanoic acid (137 mg, 404 µmol) 3-{3-(2-carboxyethoxy)-2-[(2-carboxyethoxy)methyl]-2-[(2-hydroxyethyl)amino]propoxylpropanoic acid (155 mg, 404 µmol) and hexakis(µ-acetato-κ²*O*)-monoaqua-κ*O*-dihydroxido-κ²*O*-di-µ₃-oxido-1:2:3κ⁶*O*-triangulo-tritungsten(3 W-W)(IV) (400 mg, 404 µmol) in acetic acid (47 µL, 0.81 mmol) and water (30 mL) was irradiated in a microwave reactor for 15 minutes at 140°C. The reaction mixtures were filtrated and the filtrate was concentrated in vacuum. Separation on a preparative HPLC (acetonitrile, water + acetic acid) yielded 5.0 mg of monoaqua-κ*O-*dihydroxido-κ*²O-*(µ₃-3,3',3"-{[(2-hydroxyethy)amino]methylidynetris(methyleneoxy)}-tripropanoato-1κ²*O*¹,*O*²:2κ²*O*^{1'},*O*³*,*3κ²*O*^{2'},*O*^{3'})(µ₃-3,3',3"-[hydroxymethylidynetris-(methyleneoxy)tripropanoato-1 κ²*O*¹,*O*¹:2κ*²O^{1'}*,*O*³,3κ²*O*^{2'},*O*^{3'}]-di-µ₃-oxido-1:2:3κ⁶*O-triangulo*-tritungsten(3 *W*-*W*)(IV).
**¹H-NMR** (400 MHz, deuterium oxide) δ = 2.68 - 2.80 (m, 12 H), 3.14 (br. t, 2 H), 3.32 (s, 6 H), 3.56 (s, 6 H), 3.71 (t, 2 H), 3.82 (br. t, 6 H), 3.86 (br. t, 6 H) ppm.
**LC/MS ESI-** m/z 1348 (M-1).

### Example 17

### Monoaqua-κO-bis{µ₃-3,3',3"-[(methoxyacetyl)aminomethylidynetris(methyleneoxy)] tripropanoato-1κ²O¹,O²:2κ²O^{1'},O³:3κ²O^{2'},O^{3'}}dihydroxido-κ²O-di-µ₃-oxido-1:2:3κ⁶O-triangulo-tritungsten(3 W-W)(IV)

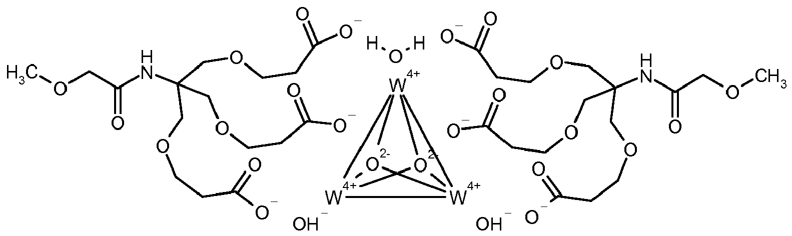

### Example 17a

### Tert-butyl 3-{3-(3-tert-butoxy-3-oxopropoxy)-2-[(3-tert-butoxy-3-oxopropoxy)methyl]-2-[(methoxyacetyl)amino]propoxy}propanoate

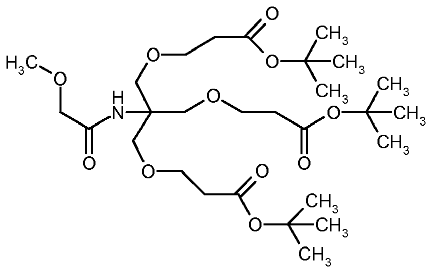

*To tert*-butyl 3-{2-amino-3-(3-*tert*-butoxy-3-oxopropoxy)-2-[(3-*tert*-butoxy-3-oxopropoxy)-methyl]propoxylpropanoate (10 g, 19.8 mmol) in dichloromethane (50 mL) and triethylamine (8.3 ML, 59.3 mmol) was added methoxyacetyl chloride (2.15g, 19.8 mmol). While addition the temperature of the reaction was allowed to warm to 35°C and stirring at room temperature was continued for 30 minutes. The reaction mixture was concentrated and purified via chromatography on silica gel (ethyl acetate in hexane 10 to 100%) to yield 9.2 g of *tert*-butyl 3-{3-(3-tert-butoxy-3-oxopropoxy)-2-[(3-tert-butoxy-3-oxopropoxy)methyl]-2-[(methoxyacetyl)amino]propoxy}propanoate as a colorless oil.
**¹H-NMR** (400 MHz, CDCl₃) δ = 1.45 (s, 27 H), 2.46 (t, 6H), 3.39 (s, 3 H), 3.66 (t, 6 H), 3.73 (s, 6 H), 3.79 (s, 2 H), 6.71 (s, 1 H) ppm.

### Example 17b

### 3-{3-(2-Carboxyethoxy)-2-[(2-carboxyethoxy)methyl]-2-[(methoxyacetyl)amino]-propoxy}propanoic acid

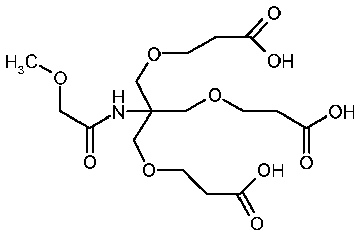

To a solution of *tert*-butyl 3-{3-(3-tert-butoxy-3-oxopropoxy)-2-[(3-tert-butoxy-3-oxo-propoxy)methyl]-2-[(methoxyacetyl)amino]propoxylpropanoate (10.2 g, 17.6 mmol) in dioxane (75 mL) was added a 4 molar solution of hydrochloric acid in dioxane (26.5 mL, 106 mmol) and 6 M aqueous hydrochloric acid (17.6 mL, 105 mmol). After stirring for 15 hours at 40°C the solvent was removed under vacuum an d the residue was two times codestillated with toluene to yield 8.24 g of 3-{3-(2-carboxyethoxy)-2-[(2-carboxy-ethoxy)methyl]-2-[(methoxyacetyl)amino]propoxy}propanoic acid.
**¹H-NMR** (300 MHz, deuterium oxide) δ = 2.58 (t, 6 H), 3.34 (s, 3 H), 3.67 (s, 6 H), 3.70 (t, 6 H), 3.85 (s, 2 H) ppm.

### Example 17c

### Monoaqua-κO-bis{µ₃-3,3',3"-[(methoxyacetyl)aminomethylidynetris(methyleneoxy)] tripropanoato-1κ²O¹,O²:2κ²O^{1'},O³:3κ²O²,O^{3'}}dihydroxido-κ²O-di-µ₃-oxido-1:2:3κ⁶O-triangulo-tritungsten(3 W-W)(IV)

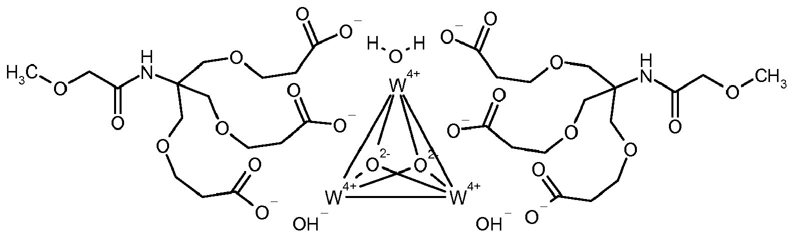

55 mg of the title compound monoaqua-κ*O*-dihydroxido-κ²*O*-bis{µ₃-3,3',3"-[(methoxyacetyl)aminomethylidynetris(methyleneoxy)]tripropanoato-1κ²*O*¹,*O*²:2κ²*O*^{1'},*O*³:3κ²*O*^{2'},*O*^{3'}}-di-µ₃-oxido-1:2:3κ⁶*O-triangulo*-tritungsten(3 *W*-*W*)(IV) was isolated as a byproduct of example 22 after HPLC separation.
**¹H-NMR** (400 MHz, deuterium oxide) δ = 2.75 (br. t, 12 H), 3.35 (s, 6 H), 3.59 (s, 12 H), 3.83 - 3.92 (m, 16 H) ppm.
**LC/MS ESI-** m/z 1447 (M-1).

### Example 18

### Monoaqua-κO-dihydroxido-κ²O-(µ₃-3,3',3"-{[(2,3-dihydroxypropyl)amino]-methylidynetris(methyleneoxy)}tripropanoato-1κ²O¹,O²:2κ²O^{1'},O³:3κ²O^{2'},O^{3'}){µ₃-3,3',3"-[2-hydroxyethyl-1,1,1-idynetris(methyleneoxy)-tripropanoato-1κ²O¹,O²: 2κ²O^{1'},O³:3κ²O^{2'},O³}di-µ₃-oxido-1:2:3κ⁶O-triangulo-tritungsten(3 W-W)(IV)

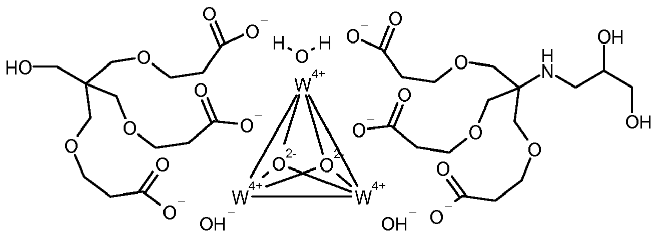

A suspension of 3-[3-(2-carboxyethoxy)-2-[(2-carboxyethoxy)methyl]-2-(hydroxylmethyl)-propoxy]propanoic acid (142 mg, 0.40 mmol) 3-{3-(2-carboxyethoxy)-2-[(2-carboxy-ethoxy)methyl]-2-[(2,3-dihydroxypropyl)amino]propoxylpropanoic acid (142 mg, 0.40 mmol) and monoaqua-κ*O*-hexakis(µ-acetato-κ²*O*)-dihydroxido-κ²*O*-di-µ₃-oxido-1:2:3κ⁶*O-triangulo*-tritungsten(3 W-W)(IV) (400 mg, 0.40 mmol) in water (60 mL) was irradiated in a microwave reactor for 15 minutes at 140°C. The reaction mixture was filtrated and the filtrate was concentrated in vacuum. Separation on a preparative HPLC (acetonitrile water + acetic acid) yielded 19 mg monoaqua-κ*O*-dihydroxido-κ²*O*-(µ₃-3,3',3"-{[(2,3-dihydroxypropyl)amino]methylidynetris(methyleneoxy)ltripropanoato-1κ²*O*¹,*O*²:2κ²*O*^{1'},*O*³:3κ²*O*^{2'},*O*^{*3*'}) {µ₃-3,3',3"-[2-hydroxyethyl-1,1,1 -idynetris(methyleneoxy)-tripropanoato-1κ²*O*¹,*O*²: 2κ²*O*^{1'},*O*³:3κ²*O*²,*O*^{3'}}di-µ3-oxido-1:2:3κ⁶*O*-*triangulo*-tritungsten(3 *W*-*W*)(IV).
**¹H-NMR** (300 MHz, deuterium oxide) δ = 2.62 - 2.74 (m, 12 H), 2.98 (dd, 1 H), 3.13 (m, 1 H), 3.32 (s, 6 H), 3.39 (s, 2 H), 3.45 - 3.61 (m, 2 H), 3.55 (s, 6 H), 3.78 (t, 6H), 3.84 (m, 7 H)
**LC/MS ESI-** m/z 1392 (m-1)

### Example 19

### {µ₃-3,3',3"-[aminomethylidynetris(methyleneoxy)}]tripropanoato-1κ²O¹,O²:2κ²O^{1'},O³ :3κ²O^{2'},O^{3'}}monoaqua-κO-dihydroxido-κ²O-{µ₃-3,3',3"-[(methoxyacetyl)-amino-methylidynetris(methyleneoxy)]tripropanoato-1κ²O¹,O²:2κ²O^{1'},O³:3κ²O^{2'},O^{3'}}di-µ₃-oxido-1:2:3κ⁶O-triangulo-tritungsten(3 W-W)(IV)

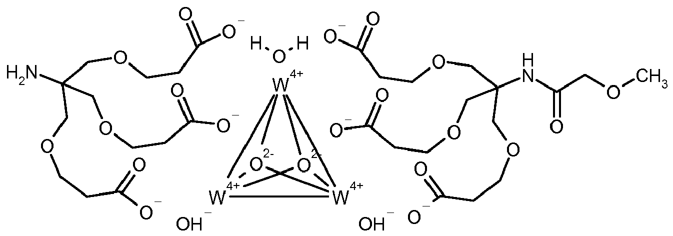

To a solution off monoaqua-κ*O*-bis{µ₃-3,3',3"-[aminomethylidynetris(methyleneoxy)]-tripropanoato-1κ²*O*¹,*O*²:2κ²*O*^{1'},*O*³:3κ²*O*^{2'},*O*^{3'}}dihydroxido-κ²*O*-di-µ₃-oxido-1:2:3κ⁶*O*-*triangulo*-tritungsten(3 *W*-*W*)(IV) (400 mg, 290 µmol) in triethylamine (162 µL, 1.16 mmol) and water (30 mL) was added 1-[(methoxyacetyl)oxy]pyrrolidine-2,5-dione (163 mg, 0.87 mmol, J. Org. Chem. 1995 60, 331 - 336) After stirring for 3 days at room temperature additional 1-[(methoxyacetyl)oxy]pyrrolidine-2,5-dione (163 mg, 0.87 mmol) was added and stirring was continued for one day at room temperature and four hours at 50°C. Concentration in vacuum and subsequent separation on a preparative HPLC (acetonitrile water + acetic acid) yielded 15 mg {µ₃-3,3',3"-[aminomethylidynetris(methyleneoxy)}]tripropanoato-1κ²*O*¹,*O*²:2κ²*O*^{1'},*O*³,3κ²*O*^{2'},*O*^{3'}}monoaqua-κ*O*-dihydroxido-κ²*O*-{µ₃-3,3',3"-[(methoxyacetyl)aminomethylidynetris(methyleneoxy)]tripropanoato-1κ²*O*¹,*O*²:2κ²*O*^{1'},*O*³: 3κ²*O*^{2'},*O*^{3'}}di-µ₃-oxido-1:2:3κ⁶*O*-*triangulo*-tritungsten(3 *W*-*W*)(IV).
**¹H-NMR** (400 MHz, deuterium oxide) δ = 2.76 (m, 12 H), 3.35 (s, 3 H), 3.50 (s, 6 H), 3.59 (s, 6 H), 3.81 3.95 (m, 14 H).
**LC/MS ESI-** m/z 1375 (M-1).

### Example 20

### Monoaqua-κO-dihydroxido-κ²O-(µ₃-3,3',3"-{[(1-deoxyerythrito)-1-yl)amino]-methylidynetris(methyleneoxy)}tripropanoato-1κ²O¹,O²:2κ²O^{1'}O³:3κ²O^{2'}O^{3'})di-µ₃-oxido-{µ₃-3,3',3"-[propane-1,2,3-triyltris(oxy)]tripropanoato-1κ²O¹,O²: 2κ²O^{1'},O³:3κ²,O^{2'},O^{3'}}-1:2:3κ⁶O-triangulo-tritungsten(3 W-W)(IV)

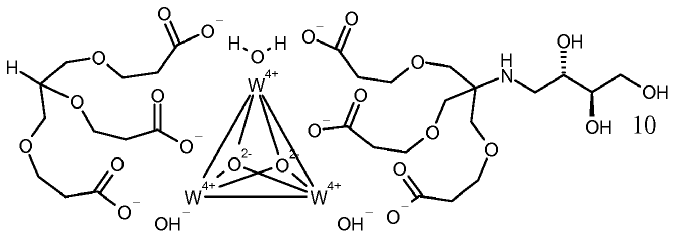

### Example 20a

### 1-({9-[(3-Tert-butoxy-3-oxopropoxy)methyl]-2,2,16,16-tetramethyl-4,14-dioxo-3,7,11,15-tetraoxaheptadecan-9-yl}amino)-1-deoxerythrol

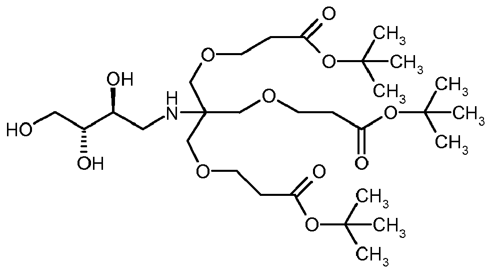

*Tert*-butyl 3-{2-amino-3-(3-*tert*-butoxy-3-oxopropoxy)-2-[(3-*tert*-butoxy-3-oxopropoxy)-methyl]propoxylpropanoate (0.99 g, 1.95 mmol), D(-)-erythrose (0.23 g, 1.95 mmol) and 2-methylpyridine borane complex (0.21 g, 1.95 mmol) in methanol (25 mL) and acetic acid (2.5 mL) were irradiated in a microwave reactor for 5 minutes at 100°C. Additional D(-)-erythrose (0.23 g, 1.95 mmol) and 2-methylpyridine borane complex (0.21g, 1.95 mmol) were added and irradiation in a microwave reactor at 100°C was repeated for for 5 minutes. The solution was concentrated under reduced pressure and residue was purified by chromatography on silica gel (methanol in ethyl acetate, 0 to 20%) to yield 0.92 g of 1-({9-[(3-tert-butoxy-3-oxopropoxy)methyl]-2,2,16,16-tetramethyl-4,14-dioxo-3,7,11,15-tetraoxaheptadecan-9-yl}amino)-1-deoxyerythrol.
**¹H-NMR** (400 MHz, CDCl₃): δ = 1.46 (s, 27 H), 2.47 (t, 6 H), 2.86 - 2.95 (m, 2 H), 2.98 (br., 6 H), 3.41 (s, 6 H), 3.60 - 3.80 (m, 4 H), 3.64 (t, 6 H) ppm.

### Example 20b

### 1-({1,3-Bis(2-carboxyethoxy)-2-[(2-carboxyethoxy)methyl]propan-2-yl}amino)-1-deoxyerythrol

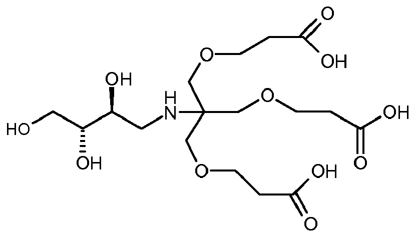

To a solution of *tert*-butyl 1-({9-[(3-tert-butoxy-3-oxopropoxy)methyl]-2,2,16,16-tetramethyl-4,14-dioxo-3,7,11,15-tetraoxaheptadecan-9-yl}amino)-1-deoxyerythol (920 mg, 1.51 mmol) in dioxane (10 mL) was added 6 molar aqueous hydrochloric acid (1.2 mL, 7.2 mmol) and a 4 molar solution of hydrochloric acid in dioxane (3 mL, 12 mmol). After stirring for 3 hours the solvent was removed under vacuum to yield the hydro chloride salt, which was treated with ion exchange resin IR 67 (40 mL) in water for 14 hours. The ion exchange resin was washed with water followed by 15 % aqueous acetic acid. The washing solutions were fractionated and fractions with a negative chloride detection were combined and concentrated under vacuum to yield 690 mg 1-({1,3-bis(2-carboxyethoxy)-2-[(2-carboxyethoxy)methyl]propan-2-yl}amino)-1-deoxyerythrol as free base.
**¹H-NMR** (400 MHz, deuterium oxide) δ = 2.55 (t, 6 H), 3.12 (dd, 1 H), 3.37 (dd 2 H), 3.58 - 3.84 (m, 4 H), 3.67 (s, 6 H), 3.72 (t, 6 H).

### Example 20c

### Monoaqua-κO-dihydroxido-κ²O-(µ₃-3,3',3"-{[(1-deoxyerythritol-1-yl)amino]-methylidynetris(methyleneoxy)}tripropanoato-1κ²O¹,O²:2κ²O^{1'},O³:3κ²O^{2'},O^{3'})di-µ₃-oxido-{µ₃-3,3',3"-[propane-1,2,3-triyltris(oxy)]tripropanoato-1κ²O¹,O²:2κ²O^{1'},O³: 3κ²O^{2'},O^{3'}}-1:2:3κ⁶O-triangulo-tritungsten(3 W-W)(IV)

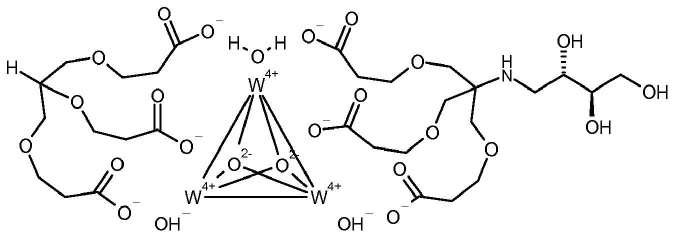

A suspension of 3,3',3"-[propane-1,2,3-triyltris(oxy)]tripropanoic acid (114 mg, 0.37 mmol), 1-({1,3-bis(2-carboxyethoxy)-2-[(2-carboxyethoxy)methyl]propan-2-yl}amino)-1-deoxyerythrol (164 mg, 0.37 mmol) and sodium hexakis(µ-acetato-κ²*O*)-tris(acetato-κ*O*)-di-µ₃-oxido-1:2:3κ⁶*O*-triangulo-tritungsten(3 *W*-*W*)(IV) (400 mg, 0.37 mmol) in water (30 mL) was irradiated in a microwave reactor for 15 minutes at 140°C. The reaction mixture was filtrated and the filtrate was concentrated in vacuum. Separation on a preparative HPLC (acetonitrile water + acetic acid) yielded 35 mg monoaqua-κ*O*-dihydroxido-κ²*O*-(µ₃-3,3',3"-{[(1-deoxyerythritol-1-yl)amino]methylidynetris(methyleneoxy)}tripropanoato-1κ²*O*¹,*O*²:2κ²*O*^{1'},*O*³:3κ²*O*^{2'},*O*^{3'})di-µ₃-oxido-{µ3-3,3',3"-[propane-1,2,3-triyltris(oxy)]tripropanoato-1κ²*O*¹,*O*²:2κ²*O*^{1'},*O*³:3κ²*O*^{2'},*O*^{3'}}-1:2:3κ⁶*O*-*triangulo*-tritungsten(3 *W*-*W*)(IV).
**¹H-NMR** (300 MHz, deuterium oxide) δ = 2.72 (m, 12 H), 3.04 (dd, 1 H), 3.15 (m, 1 H), 3.20 - 3.27 (m, 2 H), 3.35 (m, 3 H), 3.53 - 3.61 (m, 2 H), 3.56 (s, 6H), 3.67 -3.83 (m, 5 H), 3.86 (t, 6 H), 3.89 - 3.97 (m, 3 H).
**LC/MS ESI-** m/z 1378 (M-1)

### Example 21

### Monoaqua-κO-dihydroxido-κ²O-(µ₃-3,3',3"-{[(2,3-dihydroxypropan-1-yl)amino]-methylidynetris(methyleneoxy)}tripropanoato-1κ²O¹,O²:2κ¹O^{1'},O³:3κ²O^{2'}O^{3'}){µ_{3*}-3,3',3"-[(methoxyacetyl)aminomethylidynetris(methyleneoxy)]tripropanoato-1κ²O¹,O²:2κ²O^{1'},O³:3κ²O^{2'},O^{3'},}-di-µ₃-oxido-1:2:3κ⁶O-triangulo-tritungsten(3 W-W)(IV)

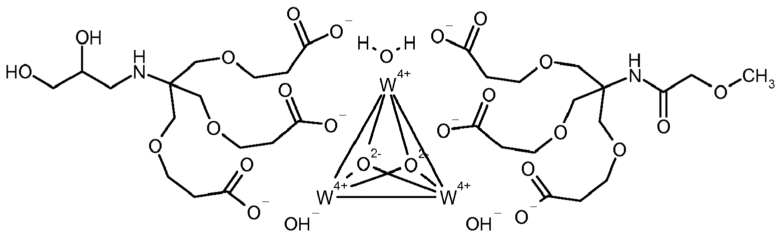

A mixture off 3-{3-(2-carboxyethoxy)-2-[(2-carboxyethoxy)methyl]-2-[(2,3-dihydroxypropyl)-amino]propoxylpropanoic acid (1.47 g, 3.59 mmol), 3-{3-(2-carboxyethoxy)-2-[(2-carboxyethoxy)methyl]-2-[(methoxyacetyl)amino]propoxy}propanoic acid (1.47 g, 3.59 mmol) and sodium hexakis(µ-acetato-κ²*O*)-tris(acetato-κ*O*)-di-µ₃-oxido-1:2:3κ⁶*O-triangulo-*tritungsten(3 *W*-*W*)(IV) (4.0 g, 3.59 mmol) in water (300 mL) was separated into 10 pressure vessels which were irradiated in a microwave reactor for 15 minutes at 140°C. The reaction mixtures were filtrated and the combined filtrates were concentrated in vacuum. Separation on a preparative HPLC (acetonitrile water + acetic acid) yielded 0.56 g Monoaqua-κ*O*-dihydroxido-κ²*O*-(µ3-3,3',3"-{[(2,3-dihydroxypropan-1-yl)amino]-methylidynetris(methyleneoxy)}tripropanoato-1κ²*O*¹,*O*²:2κ²*O*¹*O*³,*O*³:3κ²*O*^{2'},*O*^{3'}){µ₃-3,3',3"-[(methoxyacetyl)aminomethylidynetris(methyleneoxy)]tripropanoato-1κ²*O*¹,*O*²:2κ²*O*^{1'},*O*³: 3κ²*O*^{2'}*O*^{3'}}-di-µ₃-oxido-1:2:3κ⁶*O*-*triangulo*-tritungsten(3 *W*-*W*)(IV).
**¹H-NMR** (400 MHz, deuterium oxide) δ = 2.76 (br, 12 H), 3.03 (dd, 1 H), 3.17 (dd, 1 H), 3.35 (s, 3 H), 3.50 - 3.60 (m, 2 H), 3.58 (s, 12H), 3.82 - 3.92 (m, 15 H).
**LC/MS ESI-** m/z 1449 (M-1).

### Example 22

### Monoaqua-κO-dihydroxido-κ²O-(µ₃-3,3',3"-{[(1-deoxyerythritol-1-yl)amino]-methylidynetris(methyleneoxy)}tripropanoato-1κ²O¹,O²:2κ²O^{1'},O³:3κ²O^{1'},O^{3'}){µ₃-3,3',3"-[(methoxyacetyl)aminomethylidynetris(methyleneoxy)]tripropanoato-1κ²O¹,O²:2κ²O^{1'}O³:3κ²O^{2'},O^{3'}}-di-µ₃-oxido-1:2:3κ⁶O-triangulo-tritungsten(3 W-W)(IV)

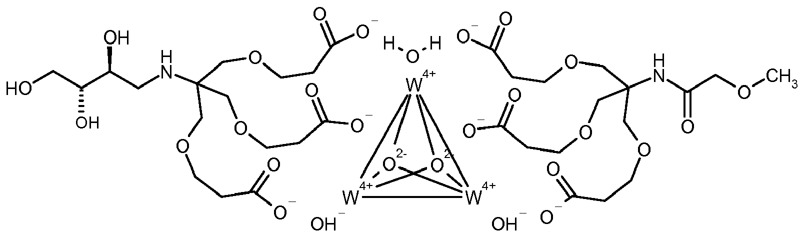

A mixture off 1-({1,3-bis(2-carboxyethoxy)-2-[(2-carboxyethoxy)methyl]propan-2-yl}amino)-1-deoxyerythrol (164 mg, 0.37 mmol), 3-{3-(2-carboxyethoxy)-2-[(2-carboxy-ethoxy)methyl]-2-[(methoxyacetyl)amino]propoxy}propanoic acid (151 mg, 0.37 mmol) and sodium hexakis(µ-acetato-κ²*O*)-tris(acetato-κ*O*)-di-µ₃-oxido-1:2:3κ⁶*O*-*triangulo*-tritungsten(3 *W*-*W*)(IV) (400 mg, 0.37 mmol) in water (30 mL) was irradiated in a microwave reactor for 15 minutes at 140°C (300W). The reaction mixture was filtrated and the filtrate concentrated in vacuum. Separation on a preparative HPLC (acetonitrile, water + acetic acid) yielded 32 mg monoaqua-κ*O*-dihydroxido-κ²O-(µ-3,3',3"-{[(1-deoxyerythritol-1-yl)amino]methylidynetris(methyleneoxy)=tripropanoato-1κ²*O*¹,*O*²:2κ²*O*^{1'},*O*³,3κ²*O*², *O*^{3'}){µ₃-3,3',3"-[(methoxyacetyl)aminomethylidynetris(methyleneoxy)]tripropanoato-1κ²*O*¹,*O*²:2κ²*O*^{1'},*O*³:3κ²*O*^{2'},*O*³'}-di-µ₃-oxido-1:2:3κ⁶*O*-*triangulo*-tritungsten(3 *W*-*W*)(IV).
**¹H-NMR** (300 MHz, deuterium oxide) δ = 2.70 (br., 12 H), 3.04 (dd, 1 H), 3.27 (dd, 1 H), 3.32 (s, 3 H), 3.47-3.69 (m, 2 H), 3.55 (s, 12 H), 3.73 - 3.90 (m, 15 H).
**LC/MS ESI-** m/z 1479 (M-1).

### Example 23a

### Sodium hexakis(µ-methoxyacetato-µ²O)-tris(methoxyacetato-κO)-di-µ₃-oxido-1:2:3κ⁶O-triangulo-tritungsten(3 W-W)(IV)

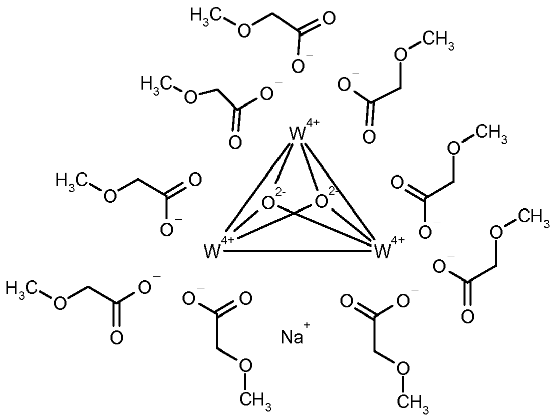

Acording to WO 97/03993 and WO 97/03994 the literature example 6 sodium hexakis(µ-methoxyacetato-κ²*O*)-tris(methoxyacetato-κ*O*)-di-µ₃-oxido-1:2:3κ*⁶O-triangulo*-tri-tungsten(3 *W*-*W*)(IV) was synthesized as reference compound.
**¹H-NMR** (300 MHz, deuterium oxide) δ = 3.36 (s, 18 H), 3.39 (s, 9 H), 4.12 (s, 6 H), 4.38 (m, 12 H).
**LC/MS ES-** m/z 1385.66 (M-23).

### Example 23b

### Monoaqua-κO-hexakis(µ-methoxyacetato-κ²O)-dihydroxido-κ²O-di-µ₃-oxido-1:2:3κ⁶O-triangulo-tritungsten(3 W-W)(IV)

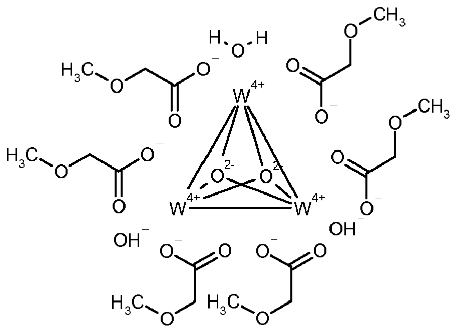

In analogy to Example 1d sodium hexakis(µ-methoxyacetato-κ²*O*)-tris(acetato-κ*O*)-di-µ₃-oxido-1:2:3κ⁶*O*-*triangulo*-tritungsten(3 *W*-*W*)(IV) was transformed into monoaqua-κ*O-*hexakis(µ-acetato-κ²*O*)-dihydroxido-κ²*O*-di-p₃-oxido-1:2:3κ⁶*O*-*triangulo*-tritungsten(3 *W-W*)(IV) as a reference compound.
**¹H-NMR** (300 MHz, deuterium oxide) δ = 3.36 (s, 18 H), 4.33 (s, 12 H).
**LC/MS ESI-** m/z 1168 (M-1).

### Example 24

### Stability of W₃O₂ Clusters

The stability of W₃O₂ clusters was determined in aqueous, buffered solution at pH 7.4. The solution containing 5 mmol/L of the compound in a tightly sealed vessel in which the air had been replaced by an argon atmosphere was heated to 121 °C for 45 min in a steam autoclave. Only in cases of the reference example compounds 1 c, 1 d, 23a and 23b it was necessary to remove precipitated material by centrifugation. The tungsten concentration of the solution was determined by ICP-OES before and after heat treatment. The integrity of the compound was determined by HPLC analysis before and after heat treatment. Absolute stability was calculated as the ratio of the peak area of the compound after and before the heat treatment multiplied with the ratio of the tungsten concentration of the solution after and before heat treatment. The relative stability was calculated with respect to example compound 23b (W₃O₂-hexa-methoxy acetate) which was set to 1.

### HPLC system:

Column: Symmetry C18, 4.6 x 75 mm (Waters).
Solvent A1: 0.1 mM Na-citrate, pH 6
Solvent A2: 5 mM Na-heptanesulfonate + 5 mM acetate pH 4.5
Solvent A3: 5 mM NH4-acetate pH 6
The use of solvent A1 to A4 depends on the structure of the compound (see table).
Solvent B: methanol, HPLC grade
Gradient: linear gradients starting from 100 % A and 0% B were used. Details are given in the table.
Flow: 1 mL/min
Detector D1: DAD: UV-vis, 200-600 nm, at 254 nm and specific absorption of W₃O₂ clusters at 460 nm.
Detector D2: element specific detection by ICP-MS running at m/z 184 for ¹⁸⁴W, the most abundant isotope of tungsten.
Detector D3: element specific detection by ICP-OES running at 239.7 nm, the most intense emission wavelength of tungsten.
Detector D1 was always used and detector D2 or D3, respectively, were coupled to the outlet of D1. Refer to the table for the detectors used.

| Example No | Stability | | Chromatographic conditions | | |
|---|---|---|---|---|---|
| | absolute | relative | Solvent A | Gradient | Detector |
| 1 | 104% | 7.4 | A2 | 0-95% B in 10 min | D1, D2 |
| 2 | 88% | 6.3 | A1 | 0-95% B in 10 min | D1, D2 |
| 3 | 102% | 7.3 | A2 | 0-95% B in 10 min | D1, D2 |
| 4 | 107% | 7.6 | A2 | 0-95% B in 10 min | D1, D2 |
| 5 | 103% | 7.4 | A2 | 0-95% B in 10 min | D1, D2 |
| 6 | 98% | 7.0 | A2 | 0-95% B in 10 min | D1, D2 |
| 7 | 102% | 7.3 | A1 | 0-95% B in 10 min | D1, D2 |
| 8 | 101% | 7.2 | A2 | 0-95% B in 10 min | D1, D2 |
| 9 | 102% | 7.3 | A2 | 0-95% B in 10 min | D1, D2 |
| 11 | 101% | 7.2 | A1 | 0-95% B in 10 min | D1, D2 |
| 12 | 101% | 7.2 | A1 | 0-95% B in 10 min | D1, D2 |
| 14 | 101% | 7.2 | A1 | 0-95% B in 10 min | D1, D2 |
| 15 | 104% | 7.4 | A1 | 0-95% B in 10 min | D1, D2 |
| 16 | 99% | 7.1 | A1 | 0-95% B in 10 min | D1, D2 |
| 17 | 102% | 7.3 | A1 | 0-95% B in 10 min | D1, D2 |
| 18 | 105% | 7.4 | A1 | 0-95% B in 10 min | D1, D2 |
| 19 | 99% | 7.1 | A1 | 0-95% B in 10 min | D1, D2 |
| 20 | 99% | 7.1 | A1 | 0-95% B in 10 min | D1, D2 |
| 21 | 102% | 7.3 | A1 | 0-95% B in 10 min | D1, D2 |
| 1d | 0% | 0 | A3 | 0-50% B in 10 min | D1, D3 |
| 1c | 55% | 3.9 | A3 | 0-50% B in 10 min | D1, D3 |
| 23a | 0% | 0 | A3 | 0-50% B in 10 min | D1, D3 |
| 23b | 14% | 1 | A3 | 0-50% B in 10 min | D1, D3 |

### Example 25

### Preclinical x-ray imaging

To demonstrate the efficacy of the x-ray diagnostic agent a preclinical animal investigation was performed using x-ray computed tomography (CT). The study was performed on a clinical CT unit (Sensation 64, Siemens Medical Solutions, Erlangen, Germany) with an anaesthetized rat. The compound described in example 1 was used as tungsten based contrast agents. The aim was to visualize the vascular system of the rat by using contrast enhanced CT-angiography.

The study was performed on healthy Han-Wistar rats. Initial anaesthesia was induced by inhalation of 4% Isoflurane (Baxter Deutschland GmbH, Unterschleißheim, Germany) and maintained by 1.5% Isoflurane. The tungsten based contrast agent (**Example 1**) at a concentration of 66 mg W/mL was administered intravenously via the tail vein by the help of a dedicated injection pump (flow rate = 0.8 ml/s). A dosage of 400 mg W/kg body weight was used.

An x-ray projection image was acquired to adjust the measurement range to the size of the animal. The subsequent contrast enhanced angiographic measurement was done with following CT parameter settings: Spiral mode (pitch = 1.4), x-ray tube voltage = 120 kV, effective mAs-product = 80 mAs, tube rotation time = 0.37s, slice thickness = 1.5 mm. The delay between the administration of the x-ray diagnostic agent and the start of the measurement was 2s.

The images showed a high CT signal for the heart, the major blood vessels and the kidneys. It also contains the intrinsic high CT signal of the skeleton and a low signal for tissue. All images showed a high contrast between enhanced blood vessels and surrounding tissue. The axial CT images were reformatted to sagital (**Fig.2A**) and coronar (Fig.2B) views by the software of the CT unit. The resulting images were displayed in maximum intensity projection (MIP) with a thickness of 7 mm. The high contrast for the blood vessels is clearly demonstrated in the zoomed area of the thorax, showing the heartchambers and the aortic arch with the branches of the brachiocephalic, left common carotid and left subclavian arteries (**Fig.3**). The images were analyzed quantitatively by drawing a region of interest at representative anatomical positions; the aorta ascendens, the aorta descendens, the aorta abdominals and the ateria carotis. A high CT-signal, demonstrating the highly effective x-ray attenuation of the tungsten based contrast agent was detected at all anatomical positions (**Fig.4**).

## Claims

1. Trinuclear tungsten clusters comprising tridentate tri- or tetracarboxylic acid ligands.

2. Trinuclear tungsten cluster comprising two tridentate carboxylic acid ligands of the general formula I, 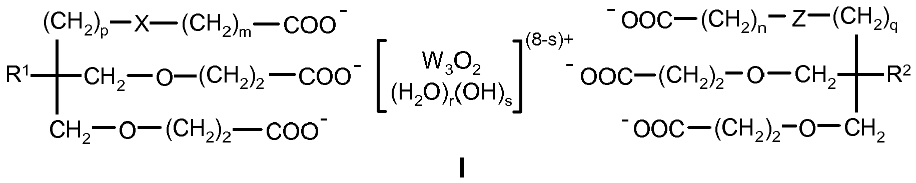 wherein
R¹ is H, CH₃, CH₂OH CH₂OCH₃ CH₂O(CH₂)₂COOH, NH₂, NH(CH₂)₂OH, NHCH₂CH(OH)CH₂OH, NHCH(CH₂OH)₂, NHCH₂(CH(OH))₂CH₂OH, NHCH₂(CH(OH))₃CH₂OH, NH(CO)CH₂OCH₃ or OH;
R² is H, CH₃, CH₂OH, CH₂OCH₃, CH₂O(CH₂)₂COOH, NH₂, NH(CH₂)₂OH, NHCH₂CH(OH)CH₂OH, NHCH(CH₂OH)₂, NHCH₂(CH(OH))₂CH₂OH, NHCH₂(CH(OH))₃CH₂OH, NH(CO)CH₂OCH₃ or OH;
X is O or NR³;
wherein R³ is H, (CH₂)₂OH, CH₂CH(OH)CH₂OH, CH(CH₂OH)₂, CH₂(CH(OH))₂CH₂OH or CH₂(CH(OH))₃CH₂OH;
Z is O or NR⁴;
wherein R⁴ is selected from the group comprising H, (CH₂)₂OH, CH₂CH(OH)CH₂OH, CH(CH₂OH)₂, CH₂(CH(OH))₂CH₂OH or CH₂(CH(OH))₃CH₂OH;
m is 2, 3;
n is 2, 3;
p is 0, 1;
q is 0, 1;
r is 0, 1, 2, 3;
and
s is 0, 1, 2, 3;
with the proviso that r + s is 3;
if necessary any protonated species and any deprotonated species of said compounds, including all isomeric forms of said compounds, including but not limited to enantiomers, diastereomers, regioisomers and mixtures thereof, and any pharmaceutically acceptable salt of such compounds.

3. Trinuclear tungsten cluster comprising two tridentate tricarboxylic acid ligands compounds of the general formula II, 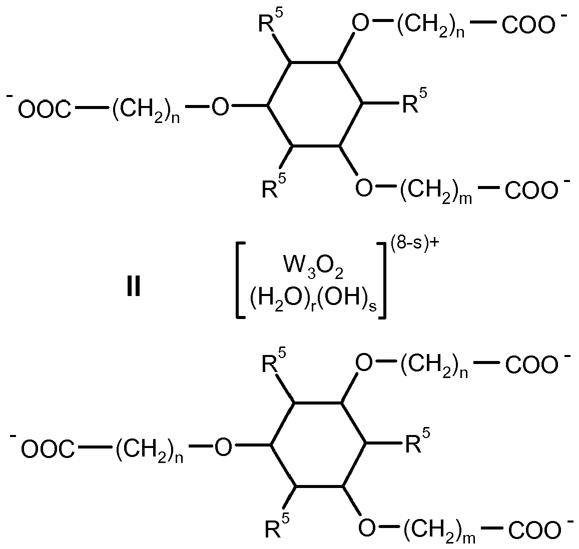 wherein
the substituents HOOC-(CH₂)ₙ-O exhibit all-cis configuration;
R⁵ is H or OH;
m is 1, 2;
n is 1, 2;
r is 0, 1, 2, 3;
and
s is 0, 1, 2, 3;
with the proviso that r + s is 3;
if necessary any protonated species and any deprotonated species of said compounds, including all isomeric forms of said compounds, including but not limited to enantiomers, diastereomers, regioisomers and mixtures thereof, and any pharmaceutically acceptable salt of such compounds.

4. Trinuclear tungsten cluster of the general formulae I or II:
Monoaqua-κ*O*-bis{µ₃-3,3',3"-[methylidynetris(methyleneoxy)]tripropanoato-1κ²*O*¹,*O*²:2κ²*O*^{1'},*O*³:3κ²*O*^{2'},*O*^{3'}}dihydroxido-κ²*O*-di-µ₃-oxido-1:2:3κ⁶*O*-triangulo-tritungsten(3 *W*-*W*)(IV)
Monoaqua-κ*O*-bis{µ₃-3,3',3"-[ethyl-1,1,1-idynetris(methyleneoxy)]tripropanoato-1κ²*O*¹,*O*²:2κ²*O*^{1'},*O*³:3κ²*O*^{2'},*O*³}dihydroxido-κ²*O*-di-µ₃-oxido-1:2:3κ⁶*O*-*triangulo-*tritungsten(3 *W*-*W*)(IV)
Monoaqua-κ*O*-bis{µ3-3,3',3"-[aminomethylidynetris(methyleneoxy)]tripropanoato-1κ²*O*¹,*O*²:2κ²*O*^{1'},*O*³:3κ²*O*²,*O*^{3'}}dihydroxido-κ²*O*-di-µ3-oxido-1:2:3κ⁶*O*-*triangulo-*tritungsten(3 *W*-*W*)(IV)
Monoaqua-κ*O*-bis{µ₃-3,3',3"-[2(2-carboxyethoxy)ethyl-1,1,1-idynetris(methylene-oxy)]tripropanoato-1κ²*O*¹,*O*²:2κ²*O*^{1'},*O*³:3κ²*O*^{2'},*O*³}-dihydroxido-κ²*O*-di-µ3-oxido-1:2:3κ⁶*O*-triangulo-tritungsten(3 *W*-*W*)(IV)
Monoaqua-κ*O*-bis{µ₃-3,3',3"-{[(2,3-dihydroxypropyl)amino]methylidynetris(methyleneoxy)}-tripropanoato-1κ²*O*¹,*O*²:2κ²*O*^{1'},*O*³:3κ²*O*^{2'},*O*^{3'}}dihydroxido-κ²*O*-di-µ₃-oxido-1:2:3κ⁶*O-triangulo*-tritungsten(3 *W*-*W*)(IV)
Monoaqua-κ*O*-bis(µ₃-3,3',3"-{[(1-deoxyxylit-1-yl)amino]methylodynetris(methyleneoxy)}-tripropanoato-1κ²*O*¹,*O*²:2κ²*O*^{1'},*O*³:3κ²*O*^{2'},*O*^{3'})-dihydroxido-κ²*O*-di-µ3-oxido-1:2:3κ⁶*O*-*triangulo*-tritungsten(3 *W*-*W*)(IV)
(µ₃-3,3',3"-{Aminomethylidynetris(methyleneoxy)}tripropanoato-1κ²*O*¹,*O*²:2κ²*O*^{1'},*O*³: 3κ²*O*^{2'},*O*^{3'})monoaqua-κO-dihydroxido-κ²O-di-µ₃-oxido-1:2:3κ⁶O-{µ3-3,3',3"-[propane-1,2,3-triyltris(oxy)]tripropanoato-1κ²*O*¹,*O*²:2κ²*O*^{1'},*O*³:3κ²*O*^{2'},*O*^{3'}}triangulo-tritungsten(3 *W*-*W*)(IV)
{µ₃-3,3',3"-[Aminomethylidynetris(methyleneoxy)]tripropanoato-1κ²*O*¹,*O*²:2κ²*O*^{1'},*O*³: 3κ²*O*²,*O*^{3'}}monoaqua-κ*O*-dihydroxido-κ²*O*-{µ3-3,3',3"-[methylidynetris(methylene-oxy)]tripropanoato-1κ²*O*¹,*O*²:2κ²*O*^{1'},*O*³:3κ²*O*^{2'},*O*^{3'}}di-µ₃-oxido-1:2:3κ⁶*O*-*triangulo-*tritungsten(3 *W*-*W*)(IV)
Monoaqua-κ*O*-dihydroxido-κ²*O*-(µ₃-3,3',3"-{[(2,3-dihydroxypropyl)amino]methylidynetris-(methyleneoxy)ltripropanoato-1κ²*O*¹,*O*²:2κ²*O*^{1'},*O*³:3κ²*O*^{2'},*O*^{3'})di-µ₃-oxido-1:2:3κ⁶*O*-{µ₃-3,3',3"-[propane-1,2,3-triyltris(oxy)]tripropanoato-1κ²*O*¹,*O*²:2κ²*O*¹*O*^{1'},*O*³: 3κ²*O*^{2'},*O*^{3'}}*triangulo-*tritungsten(3 *W*-*W*)(IV)
Monoaqua-κ*O*-bis{µ₃-3,3',3"-[(*1*α,*3*α,*5*α)-cyclohexane-1,3,5-triyltris(oxy)]tripropanoato-1κ²*O*¹,*O*²:2²κ*O*^{1'},*O*^{3'}:3κ²*O*^{2'},*O*^{3'}}dihydroxido-κ²*O*-di-µ₃₋oxido-1:2:3κ⁶*O*-*triangulo-*tritungsten(3 *W*-*W*)(IV)
Monoaqua-κ*O*-bis{µ₃-3,3',3"-[2-hydroxyethyl-1,1,1-idynetris(methyleneoxy)-tripropanoato-1κ²*O*¹,*O*²:2κ²*O*^{1'},*O*³:3κ²*O*^{2'},*O*^{3'}}dihydroxido-κ²*O*-di-µ₃-oxido-1:2:3κ⁶*O*-*triangulo-*tritungsten(3 *W*-*W*)(IV)
Monoaqua-κ*O*-bis(µ₃-3,3',3"-[2-methoxyethyl-1,1,1-idynetris(methyleneoxy)-tripropanoato-1κ²*O*¹,*O*²:2κ²*O*^{1'},*O*³,3κ²*O*^{2'},*O*³]-dihydroxido-κ²*O*-di-µ₃-oxido-1:2:3κ⁶*O-triangulo-*tritungsten(3 *W*-*W*)(IV)
Monoaqua-κ*O*-dihydroxido-κ²*O*-bis(µ₃-3,3',3"-[hydroxymethylidyne tris(methyleneoxy)tripropanoato-1κ²*O*¹,*O*²:2κ²*O*^{1'},*O*³3κ²*O*^{2'},*O*³]-di-µ₃-oxido-1:2:3κ⁶*O*-*triangulo*-tritungsten(3 *W-W*)(IV)
Monoaqua-κ*O*-dihydroxido-κ²*O*-(µ₃-3,3',3"-{[(2,3-dihydroxypropyl)amino]methylidynetris-(methyleneoxy)ltripropanoato-1κ²*O*¹,*O*²:2κ²O^{1'},*O*³:3κ²*O*^{2'},*O*^{3'})(µ₃-3,3',3"-[hydroxy-methylidynetris(methyleneoxy)tripropanoato-1κ²*O*¹,*O*²:2κ²*O*^{1'},*O*³:3κ²*O*^{2'},*O*^{3'}]-di-µ₃-oxido-1:2:3κ⁶*O*-*triangulo*-tritungsten(3 *W*-*W*)(IV)
Monoaqua-κ*O*-dihydroxido-κ²*O*-bis(µ₃-3,3',3"-{[(2-hydroxyethyl)amino]methylidyne- tris-(methyleneoxy)ltripropanoato-1κ²*O*¹,*O*²:2κ²*O*^{1'},*O*³:3κ²*O*^{2'},*O*^{3'})-di-µ₃-oxido-1:2:3κ⁶*O*-triangulo-tritungsten(3 *W*-*W*)(IV)
Monoaqua-κ*O*-dihydroxido-κ²*O*-(µ₃-3,3',3"-{[(2-hydroxyethy)amino]methylidynetris-(methyleneoxy)}tripropanoato-1κ²*O*¹,*O*²:2κ²*O*^{1'},*O*³:3κ²*O*^{2'},*O*^{3'})(µ₃-3,3',3"-[hydroxy-methylidynetris(methyleneoxy)tripropanoato-1κ²*O*¹,*O*²:2κ²*O*^{1'},*O*³:3κ²*O*^{3'}]di-µ₃-oxido-1:2:3κ⁶*O*-*triangulo*-tritungsten(3 *W*-*W*)(IV)
Monoaqua-κ*O*-bis{µ₃-3,3',3"-[(methoxyacetyl)aminomethylidynetris(methyleneoxy)] - tripropanoato-1κ²*O*¹,*O*²:2κ²*O*^{1'},*O*³:3K²*O*²,*O*^{3'}}dihydroxido-κ²*O*-di-µ₃-oxido-1:2:3κ⁶*O*-*triangulo-*tritungsten(3 *W*-*W*)(IV)
Monoaqua-κ*O*-dihydroxido-κ²*O*-(µ₃-3,3',3"-{[(2,3-dihydroxypropyl)amino]methylidynetris-(methyleneoxy)}tripropanoato-1κ²*O*¹,*O*²:2κ²*O*^{1'},*O*³:3κ²*O*^{2'},*O*^{3'}){µ₃-3,3',3"-[2-hydroxyethyl-1,1,1-idynetris(methyleneoxy)-tripropanoato-1κ²*O*¹,*O*²: 2κ²*O*^{1'},*O*³:3κ²*O*^{2'},*O*³}di-µ₃-oxido-1:2:3κ⁶*O*-*triangulo*-tritungsten(3 *W*-*W*)(IV)
{µ₃-3,3',3"-[aminomethylidynetris(methyleneoxy)}]tripropanoato-1κ κ²*O*¹,*O*²:2κ²*O*¹,*O*³ :3κ²*O*²,*O*^{3'}}monoaqua-κ*O*-dihydroxido-κ²*O*-{µ₃-3,3',3"-[(methoxyacetyl)-aminomethylidyne-tris(methyleneoxy)]tripropanoato-1κ²*O*¹,*O*²:2κ²*O*^{1'},*O*³:3κ²*O*^{2'},*O*^{3'})di-µ₃-oxido-1:2:3κ⁶*O-triangulo*-tritungsten(3 *W-W*)(IV)
Monoaqua-κ*O*-dihydroxido-κ²*O*-(µ₃-3,3',3"-{[(1-deoxyerythritol-1-yl)amino]methylidynetris-(methyleneoxy)}tripropanoato-1κ²*O*¹,*O*²:2κ²*O*^{1'},*O*³:3κ²*O*^{2'},*O*^{3'})di-µ₃-oxido-{µ₃-3,3',3"-[propane-1,2,3-triyltris(oxy)]tripropanoato-1κ²*O*¹,*O*²:2κ²*O*^{1'},*O*³: 3κ²*O*^{2'},*O*^{3'}}-1:2:3κ⁶*O-triangulo*-tritungsten(3 *W-W*)(IV)
Monoaqua-κ*O*-dihydroxido-κ²*O*-(µ₃-3,3',3"-{[(2,3-dihydroxypropan-1-yl)amino]-methylidynetris(methyleneoxy)ltripropanoato-1κ²*O*¹,*O*²:2κ²*O*^{1'},O³:3κ²*O*²,*O*³){µ₃-3,3',3"-[(methoxyacetyl)aminomethylidynetris(methyleneoxy)]tripropanoato-1κ²*O*¹,*O*²:2κ²*O*^{1'},*O*³:3κ²*O*^{2'},*O*^{3'}}-di-µ₃-oxido-1:2:3κ⁶*O*-*triangulo*-tritungsten(3 *W*-*W*)(IV)
Monoaqua-κ*O*-dihydroxido-κ²*O*-(µ₃-3,3',3"-{[(1-deoxyerythritol-1-yl)amino]-methylidynetris(methyleneoxy)}tripropanoato-1κ²*O*¹,*O*²:2κ²*O*^{1'},*O*³:3κ²*O*^{2'},*O*^{3'}){µ₃-3,3',3"-[(methoxyacetyl)aminomethylidynetris(methyleneoxy)]tripropanoato-1κ²*O*¹,*O*²:2κ²*O*^{1'},*O*³:3κ²*O*^{2'},*O*^{3'}-di-µ₃-oxido-1:2:3κ⁶*O*-*triangulo*-tritungsten(3 *W*-*W*)(IV) and if necessary protonated species and deprotonated species of said compounds and suitable salts thereof.

5. Trinuclear tungsten clusters comprising two tridentate carboxylic acid ligands of general formulae I or II of claims 2 and 3, obtainable by ligand exchange reaction of monoaqua-κ*O-*hexakis(µ-acetato-κ²*O*)-dihydroxido-κ*²O*-di-µ₃-oxido-1:2:3κ⁶*O*-*triangulo-*tritungsten(3 *W*-*W*)(IV) or a salt of this cluster or sodium hexakis(µ-acetato-κ²*O*)-tris(acetato-κ*O*)-di-µ₃-oxido-1:2:3κ⁶*O*-*triangulo*-tritungsten(3 *W*-*W*)(IV) in aqueous solution in the presence of an aliquot or slight excess of the desired tridentate ligand or a mixture of tridentate ligands, heating the components to temperatures in the range from 80° to 150°C in combination with the optional use of a pressure vessel, if necessary microwave irradiation, applying heating times range from 10 minutes up to 3 days, isolation and purification with chromatography or ion exchange chromatography.

6. Process for the preparation trinuclear tungsten clusters comprising two tridentate carboxylic acid ligands of general formula I or II of claims 2 and 3, by reaction of monoaqua-κ*O*-hexakis(µ-acetato-κ²*O*)-dihydroxido-κ²*O*-di-µ₃-oxido-1:2:3κ⁶*O*-triangulo-tritungsten(3 *W*-*W*)(IV) or a salt of this cluster or sodium hexakis(µ-acetato-κ²*O*)-tris(acetato-κ*O*)-di-µ₃-oxido-1:2:3κ⁶*O*-*triangulo*-tritungsten(3 *W*-*W*)(IV) in aqueous solution in the presence of an aliquot or slight excess of the desired tridentate ligand or a mixture of tridentate ligands, heating by temperatures in the range from 80° to 150°C in combination with the optional use of a pressure vessel, if necessary microwave irradiation, isolation and purification with chromatography or ion exchange chromatography.

7. Use of the compounds of general formulae I or II or mixtures thereof for the manufacture of diagnostic agents, especially X-ray diagnostic agents.

8. Tridentate tri- or tetracarboxylic acids as desired tridentate ligand resources as intermediates:
3-{3-(2-Carboxyethoxy)-2-[(2-carboxyethoxy)methyl]propoxy}propanoic acid
3-{3-(2-Carboxyethoxy)-2-[(2-carboxyethoxy)methyl]-2-methylpropoxy}propanoic acid 3-{2-Amino-3-(2-carboxyethoxy)-2-[(2-carboxyethoxy)methyl]propoxy}propanoic acid
3-{3-(2-Carboxyethoxy)-2,2-bis[(2-carboxyethoxy)methyl]propoxy}propanoic acid
3-{3-(2-Carboxyethoxy)-2-[(2-carboxyethoxy)methyl]-2-[(2,3-dihydroxypropyl)amino]-propoxy}propanoic acid
1-({1,3-Bis(2-carboxyethoxy)-2-[(2-carboxyethoxy)methyl]propan-2-yl}amino)-1-deoxyxylitol
3,3',3"-[Propane-1,2,3-triyltris(oxy)]tripropanoic acid
3-[3-(2-Carboxyethoxy)-2-[(2-carboxyethoxy)methyl]-2-(hydroxymethyl)propoxy]propanoic acid
3-[3-(2-Carboxyethoxy)-2-[(2-carboxyethoxy)methyl]-2-(methoxymethyl)propoxy]propanoic acid
3-{3-(2-Carboxyethoxy)-2-[(2-carboxyethoxy)methyl]-2-hydroxypropoxy}propanoic acid
3-{3-(2-Carboxyethoxy)-2-[(2-carboxyethoxy)methyl]-2-[(2-hydroxyethyl)amino]propoxy}-propanoic acid
3-{3-(2-Carboxyethoxy)-2-[(2-carboxyethoxy)methyl]-2-[(methoxyacetyl)amino]propoxy}-propanoic acid
1-({1,3-Bis(2-carboxyethoxy)-2-[(2-carboxyethoxy)methyl]propan-2-yl}amino)-1-deoxyerythrol
N-[1,3-Bis(2-carboxyethoxy)propan-2-yl]-beta-alanine
3,3',3"-[(all-cis)-Cyclohexane-1,3,5-triyltris(oxy)]tripropanoic acid
